(19) Europäisches Patentamt
European Patent Office
Office européen des brevets

(11) EP 2 349 312 B1

(12) EUROPÄISCHE PATENTSCHRIFT

(45) Veröffentlichungstag und Bekanntmachung des Hinweises auf die Patenterteilung:
**04.06.2014 Patentblatt 2014/23**

(21) Anmeldenummer: **09795303.8**

(22) Anmeldetag: **29.10.2009**

(51) Int Cl.:
***A61K 38/06*** $^{(2006.01)}$

(86) Internationale Anmeldenummer:
**PCT/DE2009/001514**

(87) Internationale Veröffentlichungsnummer:
**WO 2010/048941 (06.05.2010 Gazette 2010/18)**

(54) **N-TERMINAL MODIFIZIERTE TETRAPEPTIDDERIVATE MIT C-TERMINALEM ARGININMIMETIKUM**

N-TERMINALLY MODIFIED TETRAPEPTIDE DERIVATIVES HAVING A C-TERMINAL ARGININE MIMETIC

DÉRIVÉ TÉTRAPEPTIDIQUE MODIFIÉ EN POSITION N-TERMINALE AVEC UN MIMÉTIQUE D'ARGININE EN POSITION C-TERMINALE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **30.07.2009 DE 102009035593**
**29.10.2008 DE 102008056082**
**29.10.2008 DE 102008053693**

(43) Veröffentlichungstag der Anmeldung:
**03.08.2011 Patentblatt 2011/31**

(73) Patentinhaber: **Philipps-Universität Marburg**
**35032 Marburg (DE)**

(72) Erfinder:
- **STEINMETZER, Torsten**
  **07743 Jena (DE)**
- **BECKER, Gero**
  **35037 Marburg (DE)**
- **GARTEN, Wolfgang**
  **35392 Giessen (DE)**

(74) Vertreter: **Patentanwälte Olbricht Buchhold Keulertz**
**Partnerschaft**
**Bettinastrasse 53-55**
**60325 Frankfurt (DE)**

(56) Entgegenhaltungen:
**WO-A1-2007/046781 WO-A1-2008/049595**

- **LIN JIAN ET AL: "Design, synthesis, and biological evaluation of peptidomimetic inhibitors of factor XIa as novel anticoagulants." JOURNAL OF MEDICINAL CHEMISTRY 28 DEC 2006 LNKD- PUBMED:17181160, Bd. 49, Nr. 26, 28. Dezember 2006 (2006-12-28), Seiten 7781-7791, XP009132450 ISSN: 0022-2623**
- **MCNAUGHTON BRIAN R ET AL: "Resin-bound dynamic combinatorial chemistry." ORGANIC LETTERS 27 APR 2006 LNKD- PUBMED: 16623555, Bd. 8, Nr. 9, 27. April 2006 (2006-04-27), Seiten 1803-1806, XP009132438 ISSN: 1523-7060**
- **STEINMETZER TORSTEN ET AL: "Progress in the development of synthetic thrombin inhibitors as new orally active anticoagulants." CURRENT MEDICINAL CHEMISTRY SEP 2004 LNKD- PUBMED:15379714, Bd. 11, Nr. 17, September 2004 (2004-09), Seiten 2297-2321, XP009132412 ISSN: 0929-8673**
- **FUGERE M ET AL: "Cutting back on pro-protein convertases: the latest approaches to pharmacological inhibition" TRENDS IN PHARMACOLOGICAL SCIENCES, ELSEVIER, HAYWARTH, GB LNKD- DOI:10.1016/J.TIPS. 2005.04.006, Bd. 26, Nr. 6, 1. Juni 2005 (2005-06-01), Seiten 294-301, XP004912364 ISSN: 0165-6147**
- **ROCKWELL NATHAN C ET AL: "Precursor processing by kex2/furin proteases." CHEMICAL REVIEWS DEC 2002 LNKD- PUBMED:12475200, Bd. 102, Nr. 12, Dezember 2002 (2002-12), Seiten 4525-4548, XP002578946 ISSN: 0009-2665**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **BECKER GERO L ET AL: "Potent inhibitors of furin and furin-like proprotein convertases containing decarboxylated P1 arginine mimetics" JOURNAL OF MEDICINAL CHEMISTRY, AMERICAN CHEMICAL SOCIETY, WASHINGTON, US LNKD- DOI: 10.1021/JM9012455, Bd. 53, Nr. 3, 11. Februar 2010 (2010-02-11), Seiten 1067-1075, XP009131935 ISSN: 0022-2623**
- **BECKER GERO L ET AL: "Highly potent inhibitors of proprotein convertase furin as potential drugs for treatment of infectious diseases.", THE JOURNAL OF BIOLOGICAL CHEMISTRY 22 JUN 2012, vol. 287, no. 26, 22 June 2012 (2012-06-22), pages 21992-22003, XP007921976, ISSN: 1083-351X**
- **KUESTER MIRIAM ET AL: "Purification of the proprotein convertase furin by affinity chromatography based on PC-specific inhibitors.", BIOLOGICAL CHEMISTRY NOV 2011, vol. 392, no. 11, November 2011 (2011-11), pages 973-981, XP007921975, ISSN: 1437-4315**
- **GERO L BECKER ET AL: "New substrate analogue furin inhibitors derived from 4-amidinobenzylamide", BIOORGANIC & MEDICINAL CHEMISTRY LETTERS, PERGAMON, ELSEVIER SCIENCE, GB, vol. 21, no. 16, 19 June 2011 (2011-06-19) , pages 4695-4697, XP028247166, ISSN: 0960-894X, DOI: 10.1016/J.BMCL.2011.06.091 [retrieved on 2011-06-25]**


Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft N-terminal modifizierte Tetrapeptidderivate mit C-terminalem Argininmimetikum entsprechend Anspruch 1, Verfahren zur Herstellung der erfindungsgemäßen Tetrapeptidderivate, ein Arzneimittel enthaltend ein erfindungsgemäßes Tetrapeptidderivat und die Verwendung der erfindungsgemäßen Verbindung sowie des erfindungsgemäßen Arzneimittels.

Sie beschreibt neuartige Peptidmimetika, die als Inhibitoren der Serinprotease Furin und anderer Proproteinkonvertasen, die wie Furin C-terminal von multibasischen Spaltstellen ihre Substrate hydrolysieren, wirken. Zusätzlich werden Verfahren zu deren Herstellung bereitgestellt.

Peptidmimetika sind allgemein bekannt. Sie dienen in den Gebieten der Pharmazie, Medizin, organischen Chemie und Biochemie unter anderem als spezifische Hemmstoffe für bestimmte Enzyme. Dabei muss jedes Mimetikum speziell im Hinblick auf die jeweilige zu beeinflussende enzymatische Aktivität designt und optimiert werden. Mithin besteht eine starke Abhängigkeit der Wirksamkeit des Peptidmimetikums von dem jeweiligen Target-Enzym.

Die humanen Proproteinkonvertasen gehören zur Familie der Serinproteasen. Nach derzeitigem Wissensstand gibt es insgesamt 9 humane Proproteinkonvertasen, davon spalten 7 Vertreter (Furin, PC1/3, PC2, PC4, PC5/6, PACE4 und PC7) ihre Substrate hinter einer basischen Aminosäure, während zwei Mitglieder (SKI-1 und PCSK9) hinter nichtbasischen Aminosäureresten Peptidbindungen hydrolysieren (Seidah, N.G. und Prat, A., J. Mol. Med. 2007, 85, 685-696). Die Proproteinkonvertasen gehören zur Familie der subtilisinartigen Serinproteasen, die sich in ihrer Sequenz und Struktur sehr stark von anderen Serinproteasen unterscheiden, die ebenfalls hinter basischen P1-Resten spalten und zu denen beispielsweise die trypsinartigen Serinproteasen gehören (die in der Anmeldung verwendete Bezeichnung P1, P2, P3, P4 und P5 entspricht der von Schechter und Berger vorgeschlagenen Nomenklatur zur Bezeichnung der Aminosäurepositionen in Proteasesubstraten und substratanalogen Inhibitoren, Schechter & Berger, Biochem. Biophys. Res. Comm., 1967, 27, 157).

Im Gegensatz zu den trypsinartigen Serinproteasen ist für die Vertreter der hinter basischen P1-Resten spaltenden Proproteinkonvertasen charakteristisch, dass sie nur dann effizient Substrate umsetzen, wenn diese weitere basische Aminosäurereste N-terminal der Spaltstelle besitzen. Furin war der erste beschriebene Vertreter aus dieser Gruppe von 7 Proteasen und ist inzwischen auch am besten charakterisiert (Thomas, G., Nat. Rev. Mol. Cell. Biol., 2002, 3, 753-766). Für Furin wurde gefunden, dass es besonders dann effizient Substrate spaltet, wenn diese neben einer basischen P1-Aminosäure weitere basische Reste in P2- und P4-Position besitzen. Sehr ähnliche Substratspezifitäten wurden auch für PC1/3, PC2, PC5/6 und PC7 gefunden (siehe MEROPS Datenbank http://merops.sanger.ac.uk/ und Remacle A.G. et al. J. Biol. Chem., 2008, 283, 20897-20906), aber auch Substrate der PC4 und PACE4 benötigen basische Reste in P2- und P1-Position.

Es gibt zahlreiche Hinweise, dass die proteolytische Wirkung des Furins sehr wahrscheinlich die Entstehung oder Ausbreitung von Erkrankungen bewirkt. Möglicherweise können solche Wirkungen *in vivo* auch durch andere Proproteinkonvertasen übernommen werden, da sie eine ähnliche Substratspezifität wie das Furin besitzen. Deshalb gelten die im Folgenden gemachten Erläuterungen zur physiologischen Funktion des Furins prinzipiell auch für die anderen oben genannten Proproteinkonvertasen, die hinter basischen P1-Resten spalten.

So wurde Furin als Aktivator verschiedener bakterieller Toxine beschrieben, beispielsweise des Anthrax-Toxins, des *Clostridium septicum* $\alpha$-Toxins, des *Pseudomonas* Exotoxins, des Shigatoxins, der shigaartigen Toxine oder des Diphtherie-Toxins (zusammengefasst in Thomas, G., Nat. Rev. Mol. Cell. Biol., 2002, 3, 753-766). Diese Toxine gehören zu sogenannten A/B-Toxinen, die eine aktive Domäne (A) und eine Bindungsdomäne (B) enthalten, die beide über eine Furinspaltsequenz miteinander verbunden sind. Nach Rezeptorbindung der Toxine gelangen diese durch Endozytose in die Endosomen der befallenen Zelle und werden dort durch Furin gespalten, wodurch diese Toxine dann über verschiedene Mechanismen in das Zytosol der Zellen gelangen können (Thomas, G., Nat. Rev. Mol. Cell. Biol., 2002, 3, 753-766).

Eine Vielzahl weiterer Bakterien besitzt mindestens eine oder mehrere Toxine mit einer Furin-Spaltstelle, so z.B. *Bacillus cereus, B. anthracis, B. butulinum, Clostridium perfringens, C. difficile, C. spirioforme, Bordetella pertussis, Samonella enterica,* einige E. coli-Stämme, (beispielsweise E. *coli* 0157-H7), die als EHEC (Enterohaemorrhagische Escherichia coli-Stämme) hämolytische Kolitits, das hämolytische uraemische Syndrom, u.a. Nierenversagen verursachen. Shigella-und E. coli-Toxine werden wegen ihrer Ähnlichkeit auch als Verotoxine bezeichnet.

Furin besitzt auch eine essentielle Rolle bei der Aktivierung verschiedener pathogener Viren, beispielsweise bei hochpathogenen aviären Influenza A-Viren (HPAIV) (Thomas, G., Nat. Rev. Mol. Cell. Biol., 2002, 3, 753-766). Sie gehören alle zu den H5- bzw. H7-Subtypen, die sich serologisch von den anderen Subtypen unterscheiden lassen. Sie haben bislang als einzige eine multibasische Spaltstelle in ihrem Glykoprotein-Spike, dem Hämagglutinin, die von Furin und anderen Proproteinkonvertasen, z.B. PC5/6, gespalten wird. Es ist damit zu rechnen, dass in Zukunft noch weitere Viren anderer Subtypen identifiziert werden, die Furinspaltstellen in ihren Oberflächenproteinen besitzen. Zu diesen hochpathogenen aviären Influenza A-Viren gehören beispielsweise der in Hong Kong im Jahr 1997 aufgetretene Stamm H5N1, während H7N1 vor einigen Jahren in den Niederlanden zu beträchtlichen Verlusten bei Geflügel geführt hatte.

Die Bedeutung von Furin oder einer anderen Proproteinkonvertase mit ähnlicher Substratspezifität für Influenza wurde auch deutlich, als es mit normalerweise avirulenten aviären Influenzastämmen, die nur ein einzelnes Arginin an der Spaltstelle besitzen (also normalerweise nicht durch Furin gespalten werden) gelang, durch Mutation eine Furinspaltstelle zu generieren, wodurch eine sehr stark erhöhte Virulenz resultierte, wenn auch alle anderen Virulenzfaktoren vorhanden waren.

Weitere Virusbeispiele mit durch Furin und andere Proproteinkonvertasen aktivierbaren Glykoproteinen sind ca. 100 bedeutsame RNA- und DNA-Viren, die beim Menschen und vielen Tierarten zu bedeutsamen Erkrankungen führen. Dazu zählen bei den RNA-Negativstrangviren die Parainfluenzaviren, das Masern-Virus (measles virus), das Mumpsvirus, das Hundestaupevirus, das RSV (Respiratorische-Syncytial-Virus) beim Mensch und beim Rind, die Filoviren (Ebola- und Marburg-Virus, die schwere hämorrhagische Fieber verursachen) und das Newcastle-Disease-Virus (Virus der nicht-klassischen Geflügelpest). Bei den Plusstrang-RNA-Viren gehören zu den durch Furin aktivierbaren Spezies das HIV-1 (Humanes Immundefizienz-V.), HIV-2, HTLV (Humanes T-Lymphozytäres Leukämie-V.) und viele andere Retroviren, Coronaviren (z.B. das SARS-Coronavirus), die Flaviviren (Gelbfieber-Virus, West-Nil-Virus, Dengue-Virus), die sich zur Zeit als sogenannte "emerging viruses" aus ursprünglich eng begrenzten Gebieten über weite Teile der Welt verbreiten, vor allem aber in den tropischen Zonen. In unseren Breiten tritt das Frühsommer-Meningo-Encephalitis-Virus (engl. "tick born virus") auf. Weitere gefürchtete Viren sind das Venezuelan Equine Encephalitis Virus oder das Rift Valley Fever Virus.

All diese Viren besitzen auf ihrer Oberfläche ein Glykoprotein mit einer Furinspaltstelle. Nach Spaltung dieses Glykoproteins durch Furin wird eine Peptidsequenz demaskiert, wodurch die Virushülle mit Membranen der Virusinfizierten Zelle verschmelzen kann und dadurch das Virusgenom in die Zelle einbringt. Darüber hinaus bewirkt die Fusion in manchen Viren, dass biologisch aktive Peptide heraus gespalten werden. Durch Experimente mit HIV-1 und Zellen die kein Furin besitzen wurde gezeigt, dass möglicherweise bei der Aktivierung auch andere Proproteinkonvertasen mit ähnlicher Substratspezifität die Funktion des Furins übernehmen können.

Neben furinvermittelten Effekten bei bakteriell- und virusbedingten Erkrankungen kann Furin oder eine analoge Proproteinkonvertase auch bei anderen Erkrankungen beteiligt sein, diese verstärken oder sogar auslösen. So kann Furin verschiedene Proformen von Wachstumsfaktoren, Rezeptoren, Adhäsionsmolekülen oder Matrix-Metalloproteasen spalten, wodurch verschiedenen Erkrankungen ausgelöst werden. Zu diesen Erkrankungen gehören möglicherweise Diabetes, Arteriosklerose, Krebs/Metastasierung, aber auch Alzheimer oder die Ausbildung von Übergewicht (zusammengefasst in Bontemps, Y. et al., Med. Res. Rev., 2007, 27, 631-648). Bei neurodegenerativen Erkrankungen wie z.B. Alzheimer ist Furin möglicherweise an der Aktivierung der Zymogenformen von Sekretasen beteiligt, die für die Bildung des Aß-Peptides verantwortlich sind (Bennett, B.D. et al., J. Biol. Chem., 2000, 275, 37712-37717). Durch die bereits genannte furinvermittelte Spaltung von Matrix-Metalloproteasen, Adhäsionsmolekülen Wachstumsfaktoren, Wachstumsfaktorrezeptoren kann Furin oder eine analoge Proproteinkonvertase direkt bei der Entstehung, Entwicklung und dem Voranschreiten von Krebs sowie der Metastasierung und auch am Abbau extrazellulärer Matrixproteine beteiligt sein.

Aus der Literatur ist bekannt, dass Furin und die furinähnlichen Proproteinkonvertasen wichtige Funktionen bei der Regulation der Tumorigenese, der Angiongenese und der Metastasierung besitzen. Diese Proproteinkonvertasen spalten und aktivieren beispielsweise Vorläuferproteine von Wachstumsfaktoren, Wachstumsfaktorrezeptoren, Adhäsionsmolekülen aber auch Proformen der Matrixmetalloproteasen. Da all diese Proteine in der Tumorentwicklung involviert sind, sind die Proproteinkonvertasen neuartige Targets für die Entwicklung von Wirkstoffen zur Behandlung von Krebs und der Metastasierung (Bassi, D. E.; Mahloogi, H.; Al-Saleem, L.; Lopez De Cicco, R.; Ridge, J. A.; Klein-Szanto, A. J. Elevated furin expression in aggressive human head and neck tumors and tumor cell lines. Mol Carcinog 2001, 31, 224-32, Khatib, A. M.; Bassi, D.; Siegfried, G.; Klein-Szanto, A. J.; Ouafik, L. Endo/exo-proteolysis in neoplastic progression and metastasis. J Mol Med 2005, 83, 856-64.)

Die Arbeit von Lin et al. (2006) J. Med. Chem. 49, 7781-7791 beschäftigt sich mit Inhibitoren für den Faktor Xla, die die Koagulation unterbinden sollen. Die dort gezeigten Inhibitoren wurden jedoch lediglich auf ihre Hemmwirkung auf die trypsinartigen Serinproteasen Faktor Xla untersucht. Hochwirksame Inhibitoren von subtilisinartigen Serinproteasen, insbesondere Proteinkonvertasen, wurden nicht gezeigt.

Die Arbeit von McNaughton and Miller (2006) Org. Lett. 27, 1803-1806 beschäftigt sich mit Auswahlmethoden für spezifische Liganden. Hochwirksame Inhibitoren von subtilisinartigen Serinproteasen, insbesondere Proteinkonvertasen, wurden jedoch nicht gezeigt.

Die WO 2008/049595 A1 betrifft ebenfalls Hemmstoffe trypsinartiger Serinproteasen, die neben Plasmin auch Plasmakallikrein hemmen, sowie deren Herstellung und Verwendung als Arzneimittel, vorzugsweise zur Behandlung von Blutverlust, insbesondere bei hyperfibrinolytisehen Zuständen, bei Organtransplantationen oder herzchirurgischen Eingriffen vor allem mit cardiopulmonalem Bypass oder als Bestandteil eines Fibrinklebers. Hochwirksame Inhibitoren von subtilisinartigen Serinproteasen, insbesondere Proteinkonvertasen, wurden wiederum nicht gezeigt.

Eine Übersicht von potentiellen Substratsequenzen des Furins und der anderen Proproteinkonvertasen, die eine basische Aminosäure als P1-Rest erfordern ist, ist im Anhang einer vor kurzem erschienenen Publikation zur

Substratspezifität dieser Enzyme enthalten (Supplement zu Remacle A.G. et al. J. Biol. Chem., 2008, 283, 20897-20906). Allerdings stehen bisher noch keine geeigneten Hemmstoffe für diese Proteasen zur Verfügung, obwohl in den vergangenen Jahren von verschiedenen Gruppen versucht wurde, Furininhibitoren zu entwickeln. Dazu gehören auch Verbindungen, die von Polyarginin-Peptiden abgeleitet wurden. So hemmt beispielsweise Nona-D-Arginin-Amid Furin mit einer Hemmkonstante von 1,3 nM, während das kürzere Hexa-D-Arg-Amid einen $K_i$-Wert von 7,6 nM besitzt (Kacprzak, M.M. et al., J. Biol. Chem., 2004, 279, 36788-36794). Die WO 2007/046 781 A1 beschäftigt sich mit Inhibitoren der Endoprotease Furin. Dabei werden unter anderem Oligoarginine vorgeschlagen. Weiterhin werden darin Tetrapeptidderivate mit einem Argininmimetikum als C-terminale Struktur gezeigt, wobei das Argininmimetikum einen modifizierten Phosphonat-Rest aufweist.

Einen Überblick über bekannte Hemmstoffe des Furins gibt eine kürzlich erschienene Übersichtsarbeit (Basak, A., J. Mol. Med., 2005, 83,844-855). Dazu gehören auch reine Peptidderivate, die sich von der Prodomäne des Furins ableiten, aber auch Peptidylchlormethylketone (Hallenberger, S. et al., Nature, 1992, 360, 358-361), Peptidylaldehyde mit C-terminalem Arginal (Jean, F. et al., Biochem. J., 1993, 292, 891-899), Peptidyl-Semicarbazone und auch Peptide mit stabilisierten Pseudopeptidbindungen (Angliker, H., J. Med. Chem., 1995, 38, 4014-4018) mit denen man versucht hatte, Bindungstaschen C-terminal der Spaltstelle für die Hemmung zu nutzen. Beschrieben wurden auch Diterpen-Derivate, die Furin im mikromolaren Bereich inhibieren (Basak, A. et al., Biochem. J., 1999, 338, 107-113). Allerdings sind diese Derivate ebenso wie die anderen genannten Peptidderivate mit aktivierten P1-Carbonylgruppen nicht für eine weitere klinische Entwicklung geeignet. Besonders nachteilhaft bei diesen bekannten Hemmstoffen ist, dass sie alle eine sehr kurze Halbwertszeit im Blut sowie eine hohe intrinsische Reaktivität besitzen.

Der Erfindung liegt daher die Aufgabe zu Grunde, für therapeutische Anwendungen geeignete Wirkstoffe bereitzustellen, die Furin oder analoge Proproteinkonvertasen mit hoher Aktivität und Spezifität inhibieren und für die Behandlung von Erkrankungen verwendbar sind, bei denen diese Proteasen beteiligt sind. Insbesondere sollen die Wirkstoffe eine geringe intrinsische Reaktivität aufweisen.

Die genannte Aufgabe wird erfindungsgemäß durch modifizierte Tetrapeptidderivate gemäß Anspruch 1 gelöst. Vorteilhafte Ausgestaltungen der erfindungsgemäßen Tetrapeptidderivate ergeben sich aus den Unteransprüchen 2 bis 6.

Die erfindungsgemäßen Inhibitoren bestehen aus den 5 Segmenten P5, P4, P3, P2 und P1. Dabei ist P1 ein C-terminal befindliches Argininmimetikum, P2, P3 und P4 sind Amino- oder Iminosäurereste und P5 ist eine N-terminale Modifizierung des Amino- oder Iminosäurerestes P4. P5 ist dabei vorteilhaft eine variable Struktur, beispielsweise ein Acyl-, Sulfonyl- oder Alkylrest, der an die N-terminale Funktion des Restes P4 gekoppelt ist. Außerdem wird die Verwendung der erfindungsgemäßen Inhibitoren zur Herstellung von Arzneimitteln für die Therapie und/oder Prophylaxe von bakteriellen und viralen Erkrankungen offenbart. Die erfindungsgemäßen Verbindungen eignen sich auch für die Herstellung von Arzneimitteln, zur Therapie und/oder Prophylaxe von Krebs/Metastasierung, Alzheimer, Arteriosklerose, Diabetes oder Übergewicht.

Besonders vorteilhaft ist es dabei, wenn die erfindungsgemäße Verbindung ein Substrat oder ganz besonders bevorzugt ein Inhibitor für die Proproteinkonvertasen Furin, PC1/3, PC2, PC4, PC5/6, PACE4 und/oder PC7 ist.

Überraschend wurde gefunden, dass N-terminal modifizierte Tetrapeptidmimetika mit einem C-terminalen Argininmimetikum gemäß der allgemeinen Formel

P5-P4-P3-P2-P1 (I)

Furin sehr wirksam hemmen, wenn die Reste P1, P2, P3, P4 und P5 in Anlehnung an die Definition nach Schechter und Berger (Schechter und Berger, Biochem. Biophys. Res. Comm., 1967, 27, 157-162) folgende Definitionen besitzen:

- P1 ein decarboxyliertes Argininmimetikum ist, ausgewählt aus folgenden Strukturen:

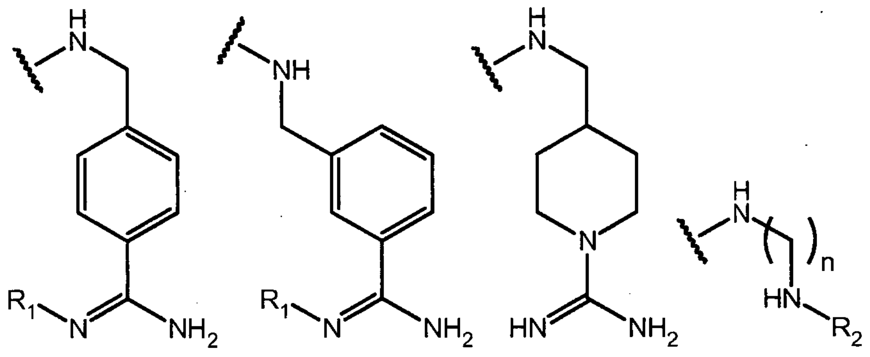

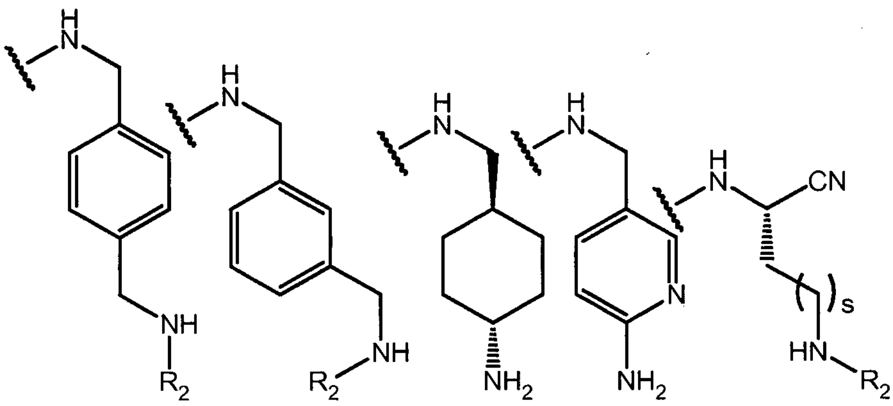

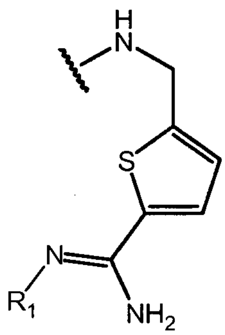

wobei

- $R_1$ ein H, OH, O-$CH_3$, $NH_2$, O-CO-$CH_3$ oder -CO-O-$(CH_2)_m$-$CH_3$ mit m eine ganze Zahl von 1 bis 5 ist, und
- $R_2$ ein H oder eine Amidinogruppe

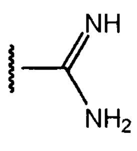

ist, und

- n gleich 3, 4 oder 5 ist, und
- s gleich 2 oder 3 ist, und

- P2 eine Amino- oder Iminosäure ausgewählt aus folgenden Strukturen

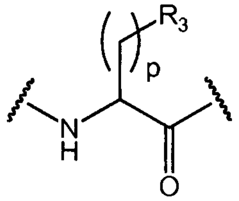 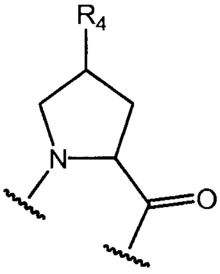

ist, und wobei

- p eine ganze Zahl von 0 bis 5 ist, und
- $R_3$ eine Amino- oder eine Guanidinogruppe sein kann, oder eine -O-Guanidinogruppe wie im Canavaninrest, oder ein einfach oder mehrfach substituierter oder unsubstituierter Aryl- oder Heteroarylrest mit 5 bis 10 Ringatomen, wobei der Heteroarylring bis zu 3 Heteroatome enthalten kann ausgewählt aus N, S oder O, wobei der Substituent am Aryl- oder Heteroarylring ausgewählt ist aus $-NH_2$, $-CH_2-NH_2$, -Amidino, -Hydroxyamidino, -Guanidino,$-CH_2$-Guanidino, -Halogen, -Cl, -Br, -I, -CN, $-CF_3$, Alkyl mit 1-3 C-Atomen oder Alkoxy mit 1-3 C-Atomen, $-COOR_5$ mit $R_5$ ein Wasserstoff oder eine Alkyl mit 1 bis 3 C-Atomen, und
- $R_4$ ein H, OH oder $-O-(CH_2)_q-R_6$ mit q eine ganze Zahl von 2 bis 4 und $R_6$ eine Amino- oder Guanidinogruppe ist, und

- P3 ein beliebiger natürlicher oder unnatürlicher $\alpha$-Amino- oder $\alpha$-Iminosäure mit L- oder R-Konfiguration ist, und
- P4 eine $\alpha$-Amino- oder $\alpha$-Iminosäure ausgewählt aus folgenden Strukturen

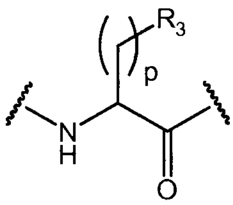 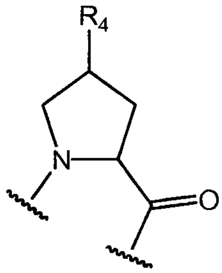

ist, wobei p, $R_3$ und $R_4$ definiert sind wie zuvor, und

- P5 ausgewählt ist aus

- einem -H oder $-CO-X-R_7$ mit X entweder nicht vorhanden oder X gleich O oder NH, wobei $R_7$ ein unsubstituierter oder gegebenenfalls einfach oder mehrfach substituierter verzweigter oder unverzweigter, gesättigter oder ungesättigter Alkylrest mit 1 bis 24 C-Atomen und 0 bis 3 Doppelbindungen ist, oder wobei $R_7$ ein unsubstituierter oder gegebenenfalls einfach oder mehrfach substituierter Aryl-, Heteroaryl, Aralkyl-, Heteroaralkyl oder Cycloalkylrest mit 5 bis 20 Kohlenstoffatomen ist, wobei der Heteroarylring bis zu 3 Heteroatome enthalten kann ausgewählt aus N, S oder O, wobei der oder die gegebenenfalls vorhandenen Substituenten an $R_7$ unabhängig voneinander ausgewählt werden aus $-NH_2$, $-CH_2-NH_2$, -Amidino, -Hydroxyamidino, -Guanidino, $-CH_2$-Guanidino, -Halogen, -Cl, -Br, -I, -CN, $-CF_3$, Alkyl mit 1-3 C-Atomen oder Alkoxy mit 1-3 C-Atomen, $-COOR_5$ mit $R_5$ ein Wasserstoff oder eine Alkylgruppe mit 1 bis 3 C-Atomen, oder wobei $R_7$ einen Cholesterolrest darstellt, oder
- einem Pyroglutamylrest oder einem anderen Amino- oder Iminosäurerest, oder
- einem $-SO_2-R_7$ wobei $R_7$ definiert ist wie zuvor, oder
- einem unverzweigten oder verzweigten Alkyl mit 1 bis 10-C-Atomen, wobei der Alkylrest gegebenenfalls mit einem Halogen, einer $NH_2$-, Guanidino-, COOH- oder $COOR_5$-Gruppe modifiziert und $R_5$ wie oben definiert ist, oder
- ein mit einem bifunktionellen Rest acyliertes Sphingosin bzw. mit einem bifunktionellen Rest acyliertes Sphingosyl-phosphorylcholin folgender Strukturen:

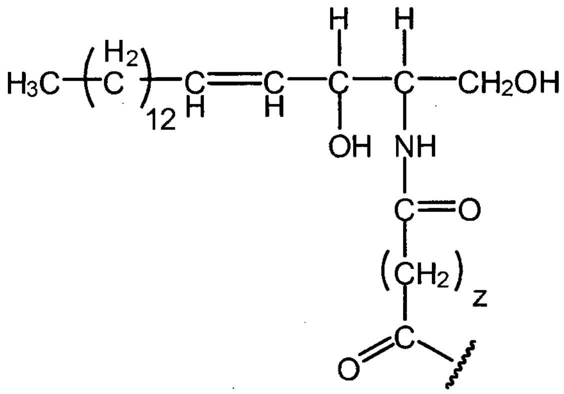

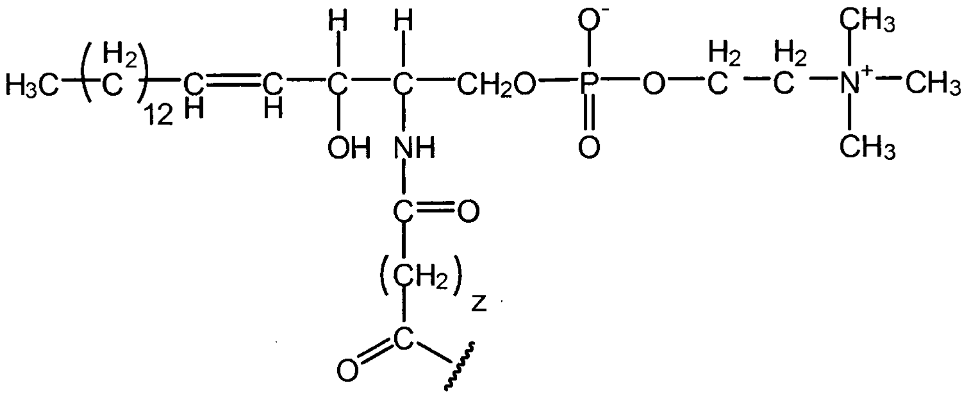

ist und wobei z eine ganze Zahl zwischen 2 und 20 ist.

Gemäß einer bevorzugten Ausführungsform enthalten die erfindungsgemäßen Verbindungen als P1-Reste die folgenden Strukturen:

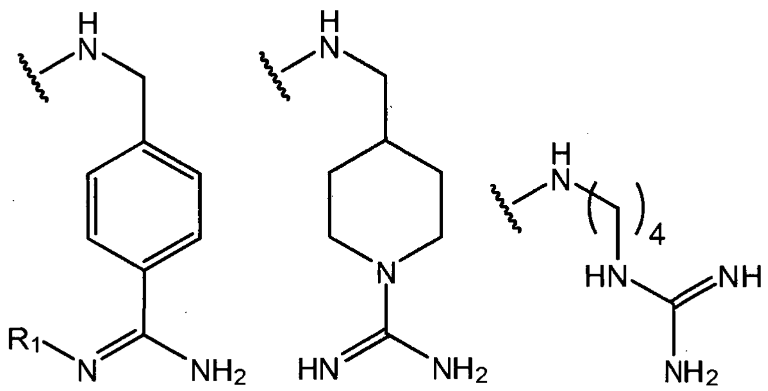

wobei $R_1$ wie oben definiert ist und wobei das 4-Amidinobenzylamid-Derivat mit einem Wasserstoffatom als Rest $R_1$ ganz besonders bevorzugt ist. Um eine verbesserte Membranpermeabilität der Inhibitoren zu erreichen, kann deren Hydrophobizität erhöht werden, indem Prodrugs der 4-Amidinobenzylamid-Derivate eingebaut werden (Ettmayer et al., J. Med. Chem. 2004, 47, 2393-2404), wobei der Rest $R_1$ in diesen Fällen -OH, $-O-CH_3$, $-NH_2$, $-O-CO-CH_3$ oder $-CO-O-(CH_2)_m-CH_3$ mit m eine ganze Zahl von 1 bis 5 sein kann. Diese Prodrugs sind auch Gegenstand der vorliegenden Erfindung, Sie werden erst im Organismus in die inhibitorisch wirksamen Verbindungen mit freier Amidinogruppe umgewandelt.

Bei P1 handelt es sich erfindungsgemäß um ein decarboxyliertes Argininmimetikum. Insbesondere sind dabei Benzamidin-Strukturen günstig. Verbindungen mit einem solchen P1-Rest weisen eine besonders geringe intrinsische Reaktivität auf.

Die anderen genannten P1 Bausteine sind bekannte Argininmimetika, die für die Entwicklung von Inhibitoren der trypsinartigen Serinprotase Thrombin verwendet wurden (siehe Übersichtsarbeit Steinmetzer und Stürzebecher in Current Med. Chem. 2004, 11, 2297-2321). Die Synthese dieser Bausteine ist dem Fachmann bekannt.

In einer weiteren bevorzugten Ausführungsform ist der Rest P2 eine basische Aminosäure in der L-Konfiguration, besonders bevorzugt sind die Aminosäurereste Arginin, Lysin sowie deren um eine Methylengruppe in der Seitenkette längeren oder kürzeren Homo- und Nor-Derivate. Ebenso sind Canavanin, 3- und 4-Amidinophenylalanin, 3- und 4-Aminomethylphenylalanin oder aber auch 3- und 4-Guanidinophenylalanin bevorzugte P2-Reste, wobei auch alle genannten Phenylalaninderivate als Homo- und Norderivate besonders geeignet sind. Als P2 Reste sind weiterhin auch Pyridylalanin und Homopyridylalanin bevorzugt, wobei der Stickstoff in jeder möglichen Position des Pyridylringes stehen kann.

In einer weiteren bevorzugten Ausführungsform ist der P3-Rest ein beliebiger natürlicher oder unnatürlicher $\alpha$-Amino- oder $\alpha$-Iminosäurerest in der L-Konfiguration, wobei als ganz besonders bevorzugte Aminosäurereste die proteinogenen Amino- und Iminosäurereste geeignet sind.

In einer weiteren bevorzugten Ausführungsform ist der Rest P4 eine basische Aminosäure in der L-Konfiguration, besonders geeignet sind die Aminosäurereste Arginin und Lysin sowie deren um eine Methylengruppe in der Seitenkette längeren oder kürzeren Homo- und Nor-Derivate. Ebenso sind Canavanin, 3- und 4-Amidinophenylalanin, 3- und 4-Aminomethylphenylalanin oder aber auch 3- und 4-Guanidinophenylalanin bevorzugte P4-Reste, wobei auch alle genannten Phenylalaninderivate als Homo- und Norderivate besonders geeignet sind. Als P4 Reste sind weiterhin auch Pyridylalanin und Homopyridylalanin bevorzugt, wobei der Stickstoff in jeder möglichen Position des Pyridylringes stehen kann.

Der P5 Rest ist sehr variabel und kann entweder im einfachsten Fall nur ein Wasserstoffatom sein, bevorzugt ist er aber ein von natürlich vorkommenden Fettsäuren abgeleiteter hydrophober gesättigter oder gegebenenfalls auch ein oder mehrfach ungesättigter Acylrest, bevorzugt ist aber auch Aralkyl-CO-Rest, oder ein Heteroaralkyl-CO-Rest oder ein Sulfonylrest, wobei all diese Rest gegebenenfalls unsubstituiert oder mit bis zu drei Resten substituiert vorliegen können. Als Substituenten sind besonders geeignet: Halogene, im Besonderen Chloratome, Aminogruppen, Carboxylgruppen die auch als Ester vorliegen können, besonders als Ethylester, Amidino-, Hydroxyamidino-, Guanidino-, -$CH_2$-Guanidino-, $CF_3$- und Alkylgruppen mit 1-3 C-Atomen oder auch Alkoxygruppen mit 1-3 C-Atomen.

Besonders geeignete P5 Gruppen auch die folgenden Strukturen:

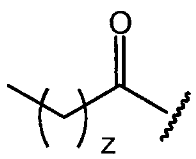

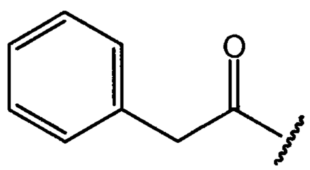

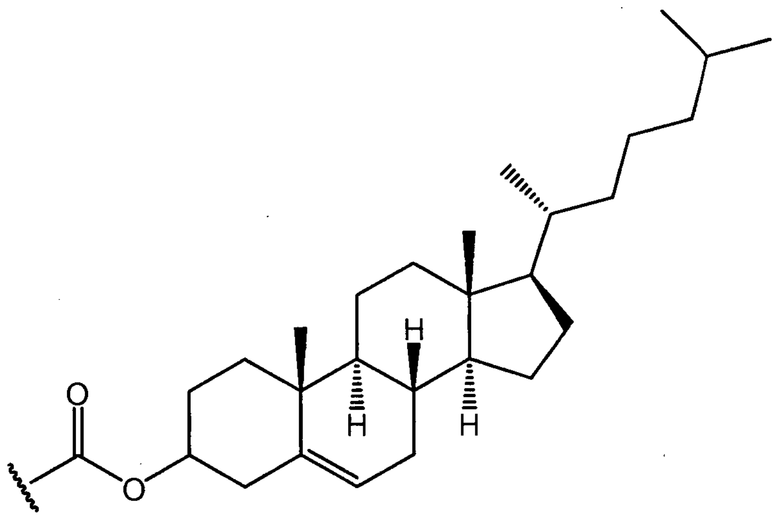

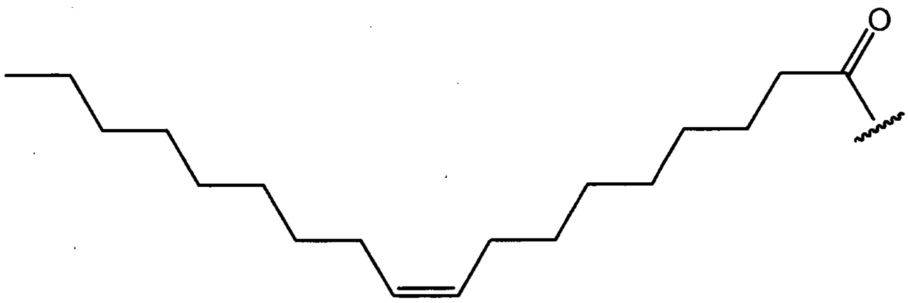

wobei z eine ganze Zahl von 1 bis 18 ist, ganz besonders bevorzugt ist z gleich 8, 14 oder 16 und auch mit einem bifunktionellen Rest acyliertes Shpingosin bzw. mit einem bifunktionellen Rest acyliertes Shpingosyl-phosphorylcholin folgender Strukturen:

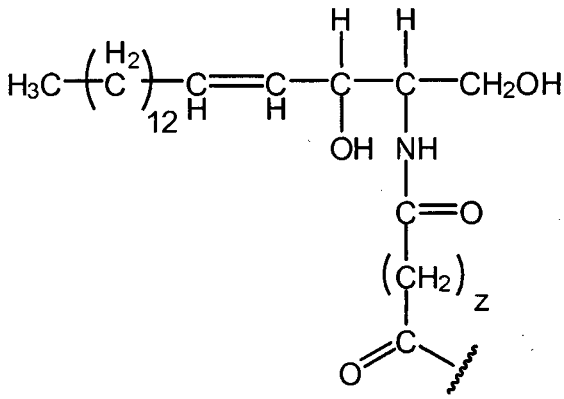

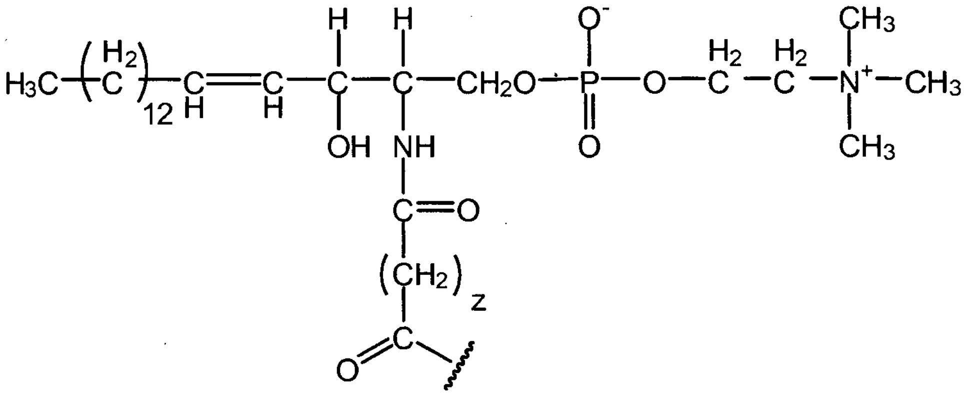

und wobei z eine ganze Zahl zwischen 2 und 20 ist.

Gegenstand der Erfindung sind auch Verfahren zur Synthese der beschriebenen Verbindungen, wobei die Synthese durch Kombination von Festphasen- und Lösungssynthesen oder auch durch reine Lösungssynthese möglich ist.

Bei der Synthese einer Verbindung gemäß der allgemeinen Formel (I) mittels Kombination von Festphasen- und Lösungssynthese werden folgende Schritte durchgeführt:

- Synthese des Segments P5-P4-P3-P2 durch Festphasenpeptidsynthese, beispielsweise an einem 2-Chlorotritylchlorid-Harz nach Standardverfahren der Fmoc-Chemie,
- anschließende Abspaltung des Peptidsegments vom Harz unter geeigneten sauren Bedingungen, wobei Seitenkettenschutzgruppen entweder erhalten oder abgespalten werden,
- Kopplung eines geeigneten P1-Bausteins mittels racemisierungsfreier Standardverfahren, der gegebenenfalls geschützt oder ungeschützt vorliegen kann, und gegebenenfalls
- Abspaltung aller eventuell noch vorhandenen Schutzgruppen, dazu gehört beispielsweise die Behandlung mit entsprechenden Säuren, beispielsweise mit Trifluoressigsäure, HBr in Eisessig, HCl in Eisessig, HF, Trifluormethansulfonsäure, wobei die Säuren gegebenenfalls nach Scavanger enthalten können, beispielsweise Wasser, Triisopropylsilan, Anisol, Dimethylsulfid oder andere gängige Scavanger mit freien Mercaptogruppen, gegebenenfalls werden noch vorhandene Schutzgruppen durch katalytische Hydrierung mit Wasserstoff und Palladium auf Aktivkohle als Katalysator entfernt, und

- finale Reinigung durch Kristallisation, Chromatographie, bevorzugt Umkehrphasen- oder Ionenaustauschchromatographie oder durch Gegenstromverteilung, und
- gegebenenfalls Überführung in eine andere bzw. geeignetere Salzform.

Alternativ wird beispielsweise zur Synthese von Verbindungen mit einem C-terminalen Agmatinrest im ersten Schritt ein Tritylchlorid-Harz mit 1,4-Diaminobutan nach Standardverfahren beladen und nacheinander die restlichen Bausteine P2, P3, P4 mittels Fmoc-Chemie und P5 mittels Standardverfahren gekoppelt, es erfolgt die Abspaltung des Peptides vom Harz mit geeigneten Säuren oder säureenthaltenden Abspaltcocktails, wobei entweder alle Schutzgruppen entfernt werden oder aber bestimmte Schutzgruppen wie beispielsweise in der Seitenkette des Lysins erhalten bleiben, gefolgt vom finalen Aufbau der Guanidinogruppe durch Umsetzung von Aminogruppen mit PyrazolCarboxamidin x HCl und DIPEA entweder in DMF oder gegebenenfalls auch in organisch wässrigen Lösungsmitteln Natriumcarbonat enthaltend oder in reiner wässriger Natriumcarbonatlösung (Bernatowicz, M.S. et al., J. Org. Chem., 1992, 57, 2497-2502). Analog können auch beliebige andere Diamine für die Beladung des Tritylchlorid-Harzes verwendet werden. Wird am Ende auf die Umsetzung mit Pyrazolcarboxamidin verzichtet, erhält man die Verbindungen mit einer C-terminalen Aminogruppe.

Für Synthesen im größeren Maßstab ("Upscaling") sind besonders Verfahren geeignet, bei denen nur reine Lösungssyntheseschritte angewendet werden. Üblicherweise beginnt die Lösungspeptidsynthese mit einem geeigneten C-terminalen P1-Baustein, an den der folgende Nα-urethan- und falls notwendig auch seitenkettengeschütze P2-Baustein gekoppelt wird, gefolgt von Abspaltung der Nα-Urethanschutzgruppe und Ankopplung des folgenden Nα-urethangeschützen Restes. Falls notwendig, können alle bei der Synthese auftretenden Intermediate gereinigt werden. Als Nα-Urethanschutzgruppe eignet sich besonders die Boc-Schutzgruppe. Die Synthese wird auf analoge Weise komplettiert, in den letzten Schritten wird der P5-Baustein gekoppelt und danach alle Schutzgruppen entfernt. Mittels dieser reinen Lösungssynthesestrategie können Racemisierungen bei den Kopplungsschritten minimiert werden, die oftmals ein Problem bei Segment-Kopplungen darstellen, wie sie oben bei der kombinierten Festphasen- und Lösungssynthese auftreten können. Eine Lösungssynthese umfasst beispielsweise folgende Schritte:

a) Synthese eines an der terminalen Amidino- oder Guanidinogruppegeeignet geschützten P1-Bausteins mit freier Aminogruppe, die entweder als Salz oder freie Base vorliegen kann, z.B. von folgender Verbindung:

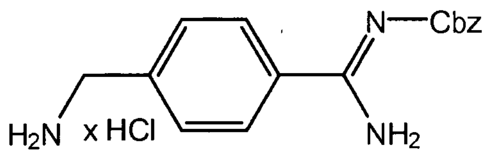

(beschrieben in Lila et al., Synth. Commun., 1998, 28, 4419-4429)

b) Kopplung einer Nα-Boc oder anderweitig geeignet Nα-urethangeschützten Amino- oder Iminosäure mit geeigneter Seitenkettenschutzgruppe, falls notwendig, an die freie Aminogruppe des P1-Baustein, wobei prinzipiell alle geeigneten Kopplungsverfahren der Peptidchemie mit bekannten Hilfsbasen verwendet werden können, dazu gehören das Mischanhydridverfahren, Carbodiimidverfahren, Aktivesterverfahren, Azidverfahren, Propanphosphonsäureanhydrid-Ver-fahren oder Kopplung mit Triazimoch, TBTU, HBTU, BOP, PyBOP, HATU oder anderer Kopplungsreagentien basierend auf Uronium- oder Phosphoniumsalzen, gefolgt von einer Aufarbeitung des Reaktionsansatzes durch gängige Wasch- und Kristallisationsverfahren sowie gegebenenfalls gefolgt von einem Chromatographieschritt zur Reinigung, und gefolgt von standardmäßiger Abspaltung der Nα-Schutzgruppe unter Erhalt des P2-P1-Segments mit freier Aminogruppe am P2-Baustein, so wird beispielsweise für die Abspaltung der Nα-Boc-Schutzgruppe in der Regel Trifluoressigsäure oder HCl in einem geeigneten organischen Lösungsmittel wie Essigester, Essigsäure, Diethylether oder Dioxan verwendet,

c) Kopplung einer Nα-Boc oder anderweitig geeignet Nα-urethangeschützten P3-Amino- oder Iminosäure an die freie Aminogruppe des P2-P1-Segments aus Schritt b) analog der im Schritt b) beschriebenen Methoden, gefolgt von der im Schritt b) beschriebenen Aufarbeitung des Reaktionsansatzes und der im Schritt b) beschriebenen Abspaltung der Nα-Schutzgruppe unter Erhalt des P3-P2-P1-Segments mit freier N-terminaler Aminogruppe am P3-Rest,

d) Kopplung einer Nα-Boc oder anderweitig geeignet Nα-urethangeschützten P4-Amino- oder Iminosäure an die

freie Aminogruppe des P3-P2-P1-Segments aus Schritt c) analog der im Schritt b) beschriebenen Methoden, gefolgt von der im Schritt b) beschriebenen Aufarbeitung des Reaktionsansatzes und der im Schritt b) beschriebenen Abspaltung der Nα-Schutzgruppe unter Erhalt des P4-P3-P2-P1-Segments mit freier N-terminaler Aminogruppe am P4-Rest,

e) Kopplung des P5-Restes an die freie Aminogruppe des P4-P3-P2-P1-Segments aus Schritt d) gegebenenfalls analog der im Schritt b) beschriebenen Methoden, oder Kopplung eines bereits als Säurechlorid oder Aktivester, speziell als Succinimidester, vorliegenden P5-Bausteins in Gegenwart von Hilfsbasen ohne weiteres Kopplungsreagenz.

f) Abspaltung aller noch vorhandenen Schutzgruppen durch gängige Verfahren, dazu gehört beispielsweise die katalytische Hydrierung mit Wasserstoff und Palladium auf Aktivkohle als Katalysator, oder die Behandlung mit entsprechenden Säuren, beispielsweise mit Trifluoressigsäure, HBr in Eisessig, HCl in Eisessig, HF, Trifluormethansulfonsäure, wobei die Säuren gegebenenfalls nach Scavanger enthalten können, beispielsweise Wasser, Triisopropylsilan, Anisol, Dimethylsulfid oder andere gängige Scavanger mit freien Mercaptogruppen, und

g) finale Reinigung durch Kristallisation, Chromatographie, bevorzugt Umkehrphasen- oder Ionenaustauschchromatographie oder durch Gegenstromverteilung, und

h) gegebenenfalls Überführung in eine andere bzw. geeignetere Salzform.

Gegenstand der Erfindung sind auch Verfahren bei denen Segmentkopplungen in Lösung durchgeführt werden, wobei für solche Segmentkopplungen besonders racemisierungsfreie Verfahren verwendet werden, beispielsweise die Azidkopplung, bei der ein geeigneter N-terminal und seitenkettengeschützter Peptidmethylester durch Umsetzung in das Hydrazid überführt wird, aus dem dann mit Standardverfahren das Azid hergestellt wird, dass für die Kopplung an einen Baustein oder ein Segment mit freier Aminogruppe eingesetzt wird. Andererseits können auch andere Kopplungsverfahren genutzt werden, wobei besonders schwache Hilfsbasen, beispielsweise Collidin, verwendet werden, oder geeignete Additive, beispielsweise 6-Cl-HOBt, HOAt eingesetzt werden.
Für die oben genannten Verfahren können alle bekannten peptidchemischen Synthesemethoden, einschließlich sämtlicher Aminosäurederivate mit geeignetenschutzgruppen, geeignete Kopplungsreagentien, geeignete Abspaltreagentien in entsprechenden Lösungsmitteln eingesetzt werden, die ausführlich in Standardwerken der Peptidchemie und organischen Chemie detailliert beschrieben sind (Houben-Weyl Band E22, Synthesis of Peptides).

Gegenstand der Erfindung sind auch pharmazeutisch geeignete bzw. verträgliche Salze. Diese Salze müssen ein pharmazeutisch verträgliches Anion oder Kation aufweisen. Geeignete pharmazeutisch verträgliche Säureadditionssalze der erfindungsgemäßen Verbindungen sind z. B. Salze anorganischer Säuren, wie Salzsäure, Bromwasserstoff-, Phosphor-, Methaphosphor-, Salpeter-, Sulfon- und Schwefelsäure oder organischer Säuren, wie z. B. Essigsäure, Benzolsulfon-, Benzoe-, Zitronen-, Ethansulfon-, Fumar-, Glucon-, Glykol-, Isethion-, Milch-, Lactobion-, Malein-, Methansulfon-, Bernstein-, p-Tuluonsulfon-, Wein- und Trifluoressigessigsäure. Für medizinische Zwecke wird in besonders bevorzugter Weise das Chlor- oder Acetatsalz verwendet. Geeignete phamazeutisch verträgliche basische Salze sind z. B. Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel *in-vitro* Anwendungen.

Der im folgenden verwendete Begriff "physiologisch funktionelles Derivat" bezeichnet jedes physiologisch verträgliche Derivat einer erfindungsgemäßen Verbindung der Formel I, z. B. einen Ester, der bei Verabreichung an einen Säuger, wie z. B. den Menschen, in der Lage ist, (direkt oder indirekt) eine Verbindung der Formel I oder einen aktiven Metaboliten hiervon zu bilden. Zu den physiologisch funktionellen Derivaten zählen auch Prodrugs der erfindungsgemäßen Verbindung. Solche Prodrugs können *in vivo* zu einer erfindungsgemäßen Verbindung metabolisiert werden. Diese Prodrugs können selber wirksam sein oder nicht. Geeignete Prodrugs für Benzamidin-Gruppen sind beispielsweise Verbindungen, bei denen das Amidin als Hydroxyamidin, Methoxyamidin, Acetylhydroxyamidin, Alkyloxycarbonylamidin, speziell als Ethyloxycarbonylamidin oder Hexyloxycarbonylamidin vorliegen (Ettmayer . et al., J. Med. Chem. 2004, 47, 2393-2404).

Die erfindungsgemäßen Verbindungen können auch in verschiedenen stereoisomeren Formen aber auch in polymorphen Formen vorliegen, z. B. als amorphe und kristalline polymorphe Formen.

Nachfolgend beziehen sich alle Verweise auf Verbindungen gemäß Formel (I) wie vorstehend beschrieben, sowie auf deren Salze, Solvate und physiologisch funktionellen Derivate.

Die vorliegende Erfindung betrifft auch die Verwendung der Verbindungen der Formel (I) als Arzneimittel zur Behandlung und Prophylaxe bakterieller und viraler Erkrankungen, sowie von Diabetes, Arteriosklerose, Krebs/Metastasierung, aber auch von Alzheimer oder der Ausbildung von Übergewicht, bei denen Furin oder analoge Proproteinkonvertasen, die hinter basischen P1-Resten spalten, beteiligt sind. Auch eine pharmazeutische Zusammensetzung enthaltend eine Verbindung der Formel (I) ist Gegenstand dieser Erfindung. Die Menge der Verbindung gemäß Formel

(I), die erforderlich ist, um den gewünschten biologischen Effekt zu erreichen, ist abhängig von einer Reihe von Faktoren, z. B. der gewählten spezifischen Verbindung, der beabsichtigten Verwendung, der Art der Verabreichung und dem klinischen Zustand des Patienten. Zur Prophylaxe oder Therapie der oben genannten Zustände können die Verbindungen gemäß Formel (I) selbst als Verbindung verwendet werden, vorzugsweise liegen sie jedoch mit einem Träger oder Hilfsstoff in Form einer pharmazeutischen Zusammensetzung vor. Der Träger bzw. Hilfsstoff muss natürlich verträglich sein, in dem Sinn, dass er mit den anderen Bestandteilen der Zusammensetzung kompatibel und nicht gesundheitsschädlich für den Patienten ist.

Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im Wesentlichen darin bestehen, dass die Bestandteile mit pharmakologisch verträglichen Träger-und/oder Hilfsstoffen gemischt werden.

Erfindungsgemäße pharmazeutische Zusammensetzungen sind insbesondere solche, die für orale, rektale, topische, perorale (z. B. sublinguale) und parentale (z. B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch Aerosole sind geeignete pharmazeutische Zusammensetzungen für die erfindungsgemäßen Verbindungen, die auch in Form von Aerosol-Sprays besonders für die Behandlung und Prophylaxe von Erkrankungen des Respirationstraktes geeignet sind. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete Pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im Allgemeinen werden die Zusammensetzungen durch gleichmäßiges und homogenes Vermischen des Wirkstoffs mit einem flüssigen und/oder fein verteilten festen Träger hergestellt, wonach das Produkt, falls erforderlich, geformt wird.

So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose oder Gummi arabicum oder Tragant, und Pastillen, die die Verabreichung einer inerten Basis wie Gelatine und Glycerin oder Saccharose und Gummi arabicum umfassen.

Geeignete pharmazeutische Zusammensetzungen für die parentale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich eine Verabreichung auch subkutan, intramuskulär oder intradermal als Injektion erfolgen kann.

Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Bezüglich der weiteren Formulierung wird auf gängige Handbücher verwiesen.

Die Erfindung betrifft auch Verfahren zur Herstellung von pharmazeutischen Zusammensetzungen, bei denen man eine oder mehrere Verbindungen der allgemeinen Formel (I) mit geeigneten Träger- und Hilfsstoffen (siehe oben) vermischt.

Besonders vorteilhaft kann die erfindungsgemäße Verbindung oder ein erfindungsgemäßes Arzneimittel zur Therapie oder Prophylaxe einer viral oder bakteriell bedingten Erkrankung sowie zur Therapie und Phrophylaxe von Diabetes, Arteriosklerose, Krebs, Alzheimer oder Übergewicht eingesetzt werden, insbesondere in oraler, subkutaner, intravenöser oder transdermaler Form oder in Form eines Sprays zur Behandlung von Erkrankungen im Respirationstrakt.

Vorteilhaft kann die erfindungsgemäße Verbindung oder ein erfindungsgemäßes Arzneimittel dabei zur Therapie oder Prophylaxe von Erkrankungen hervorgerufen durch bakterielle Erreger, insbesondere hervorgerufen durch Erreger aus der Gruppe *Pseudomonas spec., Shigella spec., Corynebacterium diphtheriae, Bacillus cereus, Bacilllus anthracis, Bacillus butulinum, Clostridium perfringens, Clostridium difficile, Clostridium septicum, Clostridium sprioforme, Bordetella pertussis, Samonella enterica* oder durch ausgewählten *Escherichia coli*-Stämmen, insbesondere *E.coli*-Stämme, die hämolytische Kolitits, das Hämolytische uraemisches Syndrom und/oder Nierenversagen verursachen, ganz insbesondere *E.coli* 0157-H7 eingesetzt werden.

Bei der Therapie oder Prophylaxe von Erkrankungen hervorgerufen durch Viren, kann die Verbindung oder das Arzneimittel insbesondere bei Erkrankungen hervorgerufen durch Viren der Gruppe

- ■ hochpathogene aviäre Influenza A-Viren, vor allem der H5- bzw. H7-Subtypen,
- ■ RNA-Negativstrangviren wie den Parainfluenzaviren, das Masern-Virus, das Mumpsvirus, das Hundestaupevirus, das Respiratorische-Syncytial-Virus, Filoviren wie das Ebola- und Marburg-Virus und das Newcastle-Disease-Virus, sowie .
- ■ Plusstrang-RNA-Viren, wie HIV-1, HIV-2, HTLV und andere Retroviren, insbesondere Coronaviren wie das SARS-Coronavirus, die Flaviviren, Gelbfieber-Viren, West-Nile-Virus., Dengue-Virus.oder das Frühsommer-Meningo-Encephalitis-Virus, das Venezuelan equine encephalitis Virus oder das Rift Valley Fever Virus verwendet werden.

Man erkennt, dass die erfindungsgemäße Verbindung oder das erfindungsgemäße Arzneimittel ganz besonders vorteilhaft zur Hemmung der Proproteinkonvertasen Furin, PC1/3, PC2, PC4, PC5/6, PACE4 und/oder PC7 verwendet werden kann. Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:

Fig. 1 die Hemmung der HA-Spaltung des Vogelgrippevirus durch unterschiedliche Konzentrationen des Inhibitors 1 und des Referenzinhibitors Dec-Arg-Val-Lys-Arg-CMK

Fig. 2 die multizyklische Vermehrung des FPV in Zellkultur in Gegenwart von Inhibitor 1 und ohne Inhibitor.

Eine detaillierte Beschreibung der Figuren findet sich bei der Beschreibung der Beispiel 7 und 8.

## Ausführungsbeispiele

### Methoden zur Analyse der Verbindungen

### Analytische HPLC

Zur analytischen reversed-phase-HPLC wurde eine HPLC-Anlage LC-10A der Firma Shimadzu, bestehend aus den Teilsystemen CTO-10A Säulenofen, LC-10ATvp Pumpen (2 x), DGU-14A Degaser, SIL-10Axl Autoinjektor, SCL-10Avp Systemcontroller, SPD-M10Avp Photodiodenarraydetektor und einer Säule 250/4,6 Nucleodur 100-5 C18 ec der Firma Macherey-Nagel (Düren, Deutschland), unter Benutzung der zugehörigen Software Shimadzu CLASS-VP, Version 7.2.1, verwendet. Die Detektion erfolgte bei 220 nm. Als Elutionsmittel dienten Wasser mit 0,1 % TFA (A) und Acetonitril mit 0,1 % TFA (B) bei einer Flussrate von 1 mL/min und einem linearen Gradienten (Anstieg von 1 % B/min). Die jeweiligen Startbedingungen (% B) sind bei den Synthesen angegeben.

### Präparative HPLC

Zur präparativen RP-HPLC wurde eine HPLC-Anlage der Firma Varian, bestehend aus den Teilsystemen Varian PrepStar Model 218 präparative Pumpen (2 x), Varian ProStar Model 320 UV-Vis Detektor, Varian Fraktionssammler Model 701, und einer Säule VP 250/32 Nucleodur 100-5 C8 ec der Firma Macherey-Nagel (Düren, Deutschland), unter Benutzung der zugehörigen Star-Software V. 6.0 verwendet. Die Detektion erfolgte bei 220 nm. Als Elutionsmittel dienten ebenfalls Wasser mit 0,1 % TFA (A) und Acetonitril mit 0,1 % TFA (B) bei einer Flussrate von 20 mL/min und einem geeigneten Gradienten.

### Massenspektroskopie

Die Spektren wurden mit einem Gerät der Firma Applied Biosystems (QTrap 2000) oder einem Spektrometer vom Typ Autospec der Firma Micromass aufgenommen.

## Dünnschichtchromatographie

Für die Dünnschichtchromatographie wurden Kieselgel-Fertigplatten Adamant $UV_{254}$ der Firma Macherey-Nagel verwendet. Als Laufmittel diente ein Gemisch aus n-Butanol, Eisessig und Wasser (4:1:1). Die Detektion der Verbindungen erfolgte durch UV-Absorption bei 254 nm. Zusätzlich wurden zur Detektion als Sprühreagenzien eine Ninhydrinlösung (300 mg Ninhydrin gelöst in 100 mL n-Butanol und 3 mL Eisessig) und nach Inkubation der DC-Platte in Chloratmosphäre eine *o*-Tolidinlösung (150 mg *o*-Tolidin und 2,1 g KI gelöst in 2 mL Eisessig und 148 mL Wasser) verwendet.

## NMR

Die $^1$H- und $^{13}$C-NMR-Spektren wurden mit einem NMR-Spektrometer vom Typ ECX-400 der Firma Jeol Inc. USA aufgenommen ($^1$H-NMR: 400 MHz, $^{13}$C-NMR: 100 MHz). Die Proben wurden in kommerziell erhältlichen deuterierten Lösemitteln der handelsüblichen Qualitäten gelöst. Die chemischen Verschiebungen wurden als $\delta$-Werte in parts per million (ppm) angegeben, als Referenz wurden die Lösungsmittelsignale verwendet.

## Verwendete Abkürzungen

| | |
|---|---|
| Ac | Acetyl |
| Amb | Amidobenzyl |
| Boc | tert.-Butyloxycarbonyl |
| Bzl | Benzyl |
| Cbz | Benzyloxycarbonyl |
| DIPEA | Diisopropylethylamin |
| DCM | Dichlormethan |
| DMF | N,N-Dimethylformamid |
| Fmoc | Fluorenylmethyloxycarbonyl |
| hArg | Homoarginin |
| HBTU | O-Benzotriazol-N,N,N',N'-tetramethyl-uronium-hexafluoro-phosphat |
| HPLC | Hochleistungs-Flüssigkeitschromatographie |
| i.V. | im Vakuum |
| LM | Lösungsmittel |
| MS | Massenspektroskopie |
| NMM | N-Methylmorpholin |
| NMP | N-Methylpyrrolidon |
| NMR | Nukleare Magnetresonanzspektroskopie |
| Phac | Phenylacetyl |
| PyBOP | Benzotriazol-1-yl-N-oxy-tris(pyrrolidino)phosphoniumhexafluoro-phosphat |
| PyBrOP | Bromo-tris-pyrrolidino-phosphonium-Hexafluorophosphat |
| RT | Raumtemperatur |
| tBu | tert.-Butyl |
| TFA | Trifluoressigsäure |
| Tfa | Trifluoracetyl |
| THF | Tetrahydrofuran |

Alle eingesetzten Fmoc-Aminosäuren, Harze, Kupplungsreagentien und anderen Reagentien für die Synthesen wurden von den Firmen Orpegen, Iris Biotech, Novabiochem, Bachem, Aldrich, Fluka oder Acros bezogen.

## Beispiel 1

Phac-Arg-Val-Arg-4-Amidinobenzylamid x 3 TFA = **Inhibitor 1**

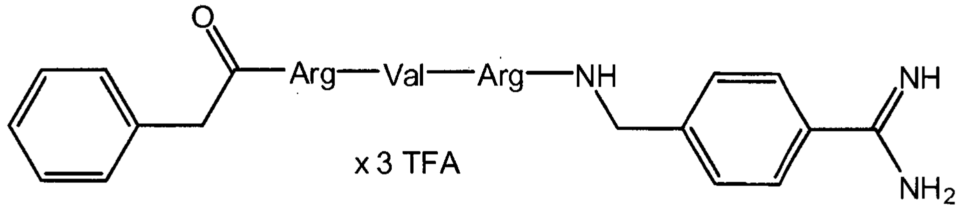

bzw. als ausführliche Struktur

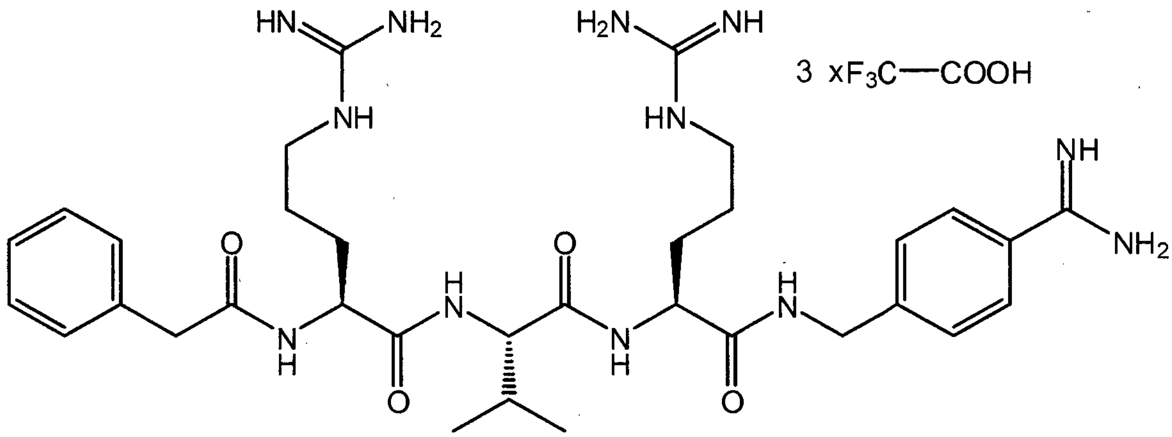

(im Folgenden werden die Standardaminosäuren immer im Drei-Buchstabencode angegeben).

Schritt a) 4-Amidinobenzylamin x 2 HCl

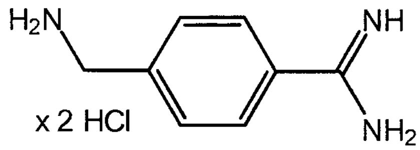

10 g (32,5 mmol) Boc-4-Acetylhydroxyamidinobenzylamid (hergestellt analog Schweinitz, A. et al., Med. Chem., 2006, 2, 349-361) wurden in 300 mL 90 % Essigsäure gelöst, mit 650 mg Katalysator (10% Pd/C) versetzt und mit Wasserstoff 6 h bei ca. 35° C und 36 h bei RT hydriert. Der Katalysator wurde abfiltriert und das LM i.V. entfernt. Der verbliebene Rückstand wurde in 150 mL Wasser gelöst, mit 40 mL konzentrierter HCl versetzt und 1,5 h gerührt. Die Lösung wurde i.V. eingeengt, der Rückstand wieder in Wasser gelöst und das LM i.V. eingeengt. Erneutes Lösen in Wasser und Lyophilisieren liefert das Produkt (weißlicher Feststoff, 6.8 g (30,6 mmol)).

$^1$H-NMR (400 MHz, $D_2O$): $\delta$ [ppm] = 4,46 (s, 2 H, H-1), 7,80-8,01 (2d, 4 H, arom.). $^{13}$C-NMR (100 Mhz, $D_2O$): $\delta$ [ppm] = 42,64 (2 H, H-1), 128,65 (2 H, H-3), 129,26 (1 H, H-5), 129,61 (2 H, H-4), 138,69 (1 H, H-2), 166,47 (1 H, H-6).

Schritt b) Phac-Arg(Pbf)-Val-Arg(Pbf)-OH

Die Beladung des 2-Chlor-Tritylchlorid-Harzes (1,5 g) mit Fmoc-Arg(Pbf)-OH erfolgte in einem Glasgefäß mit Frittenboden analog einer Standardprozedur des Herstellers mit äquimolaren Mengen an Fmoc-Arg(Pbf)-OH (1,51 g, 2,325 mmol) und 4 eq. DIPEA (9,3 mmol, 1,55 mL) bezogen auf die angegebene Harzbeladung (1,55 mmol/g) in trockenem DCM über einen Zeitraum von 2 h. Danach wurde die Reaktionslösung abgesaugt, das Harz 3 x mit DCM/Methanol/DIPEA (85/10/5 (v/v)), 3 x mit DCM, 2 x mit DMF und 2 x mit DCM gewaschen und getrocknet. Anschließend wurden die restlichen Bausteine mittels eines Standard-Fmoc-Syntheseprotokolls manuell gekoppelt, wobei im letzten Schritt Phenylessigsäure an Stelle einer Fmoc-Aminosäure verwendet wurde. Nach Beendigung der Synthese wurde das Harz mehrfach mit DMF und DCM gewaschen. Die Abspaltung vom Harz erfolgte mit 1 % TFA in DCM (v/v) in 30 min unter Schütteln. Die Abspaltlösung wurde herausgedrückt und i.V. eingeengt, anschließend wurde die Abspaltung vom Harz unter identischen Bedingungen 2 x wiederholt. Das Produkt wurde mit 80 % tert.Butanol lyophilisiert und direkt

für den nächsten Syntheseschritt verwendet (weißes Pulver, HPLC: Start bei 30 % B, Elution bei 35,57 min, MS ber. 1051,5, MS gef. 1074,3 $(M+Na)^+$).

Schritt c) Phac-Arg-Val-Arg-4-Amidinobenzylamid x 3 TFA = **Inhibitor 1**

105 mg (0,1 mmol) Phac-Arg(Pbf)-Val-Arg(Pbf)-OH als Rohprodukt, 50,9 mg (0,3 mmol) 6-Cl-HOBt, 23 mg (0,1 mmol) 4-Amidinobenzylamid x 2 HCl und 57,3 mg (0,11 mmol) PyBOP wurden in 500 µL DMF gelöst und mit 51,4 µL (0,3 mmol) DIPEA versetzt. Der Ansatz wurde 2 h bei RT gerührt. Das LM wurde i.V. entfernt und der verbleibende ölige Rückstand mit 2 mL einer Mischung aus 95 % TFA, 2,5 % Triisopropylsilan und 2,5 % Wasser (v/v) versetzt. Nach 3 h schütteln bei RT wurde das Produkt durch Zugabe von Diethylether gefällt, abzentrifugiert und das Präzipitat 2 x mit Diethylether gewaschen. Die Reinigung erfolgte durch präparative HPLC, die produktenthaltenden Fraktionen wurden vereint und lyophilisiert (weißes Pulver 60 mg, (HPLC: Start bei 1 % B, Elution bei 24,17 min, MS ber. 678,4, MS gef. 679,4 $(M+H)^+$).

**Beispiel 2**

Phac-Arg-Val-Arg-Agmatin x 3 TFA = **Inhibitor 2**

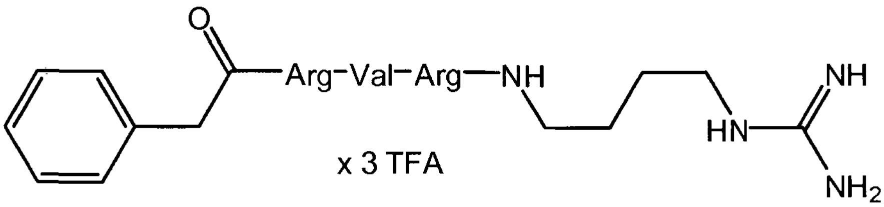

Schritt a) Phac-Arg-Val-Arg-NH-$(CH_2)_4$-$NH_2$ x 3 TFA = **Inhibitor 2a**

100 mg Tritylchlorid-Harz wurden mit 4-fachem Überschuss an 1,4-Diaminobutan (0,6 mmol) bezogen auf die angegebene Harzbeladung (1,5 mmol/g) des Herstellers in den Reaktionsgefäßen des Peptidsyntheseautomaten (Syro 2000, Multisyntech GmbH, Witten, Deutschland) mit trockenem THF als LM über einen Zeitraum von 2 h beladen. Danach wurde das LM abgesaugt und das Harz 3 x mit DCM/Methanol/DIPEA (85/10/5 (v/v)), 3 x mit DCM, 2 x mit DMF und 2 x mit DCM gewaschen.
Die folgenden Syntheseschritte erfolgten mittels eines multiplen Peptidsyntheseautomaten nach einem Standard-Fmoc-Protokoll in DMF/NMP mit Doppelkopplung und jeweils 4-fachen Überschüssen an Fmoc-Aminosäure, HOBt und HBTU in Gegenwart von 8 Äquivalenten DIPEA. Für die Fmoc-Abspaltung wurde jeweils eine Mischung aus Piperidin/DMF/NMP (1:1:1) verwendet. Im letzten Synthesezyklus wurde Phenylessigsäure analog einer normalen Fmoc-Aminosäure gekoppelt. Nach Beendigung der Synthese wurde das Harz mehrfach mit DMF und DCM gewaschen.
Die Reaktionsgefäße wurden aus dem Automaten entfernt und das Harz mit 2 mL einer Mischung aus 95 % TFA, 2,5 % Triisopropylsilan und 2,5 % Wasser (v/v) versetzt. Nach 2 h schütteln bei RT wurde die Abspaltlösung herausgedrückt und das Produkt durch Zugabe von Diethylether gefällt und abzentrifugiert. Das erhaltene Präzipitat wurde 2 x mit Diethylether gewaschen und anschließend durch präparative HPLC gereinigt, die produktenthaltenden Fraktionen vereint und lyophilisiert (weißes Pulver, HPLC: Start bei 1 % B, Elution bei 22,3 min, MS ber. 617,79, MS gef. 309,68 $(M+2H)^{2+}/2$).

Schritt b) Phac-Arg-Val-Arg-Agmatin x 3 TFA = **Inhibitor 2**

48 mg (ca. 0,05 mmol) an Phac-Arg-Val-Arg-NH-$(CH_2)_4$-$NH_2$ x 3 TFA wurden in einer Mischung aus 1 mL DMF und 1 mL 1 M $Na_2CO_3$-Lösung mit 0,15 mmol Pyrazolcarboxamidin x HCl und 0,2 mmol DIPEA versetzt. Der Ansatz wurde bei RT 36 h gerührt, das LM i.V. entfernt und das Produkt durch präparative HPLC gereinigt und lyophilisiert (weißes Pulver, HPLC: Start bei 1 % B, Elution bei 23,3 min, MS ber. 659,8, MS gef. 660,4 $(M+H)^+$).

**Beispiel 3**

Phac-Arg-Val-Arg-4-Amidomethyl-N-Amidinopiperidid x 3 TFA = **Inhibitor 3**

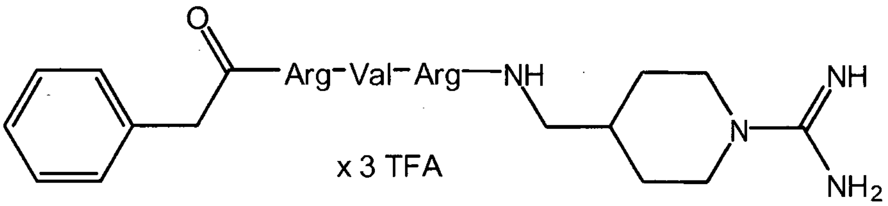

Schritt a) N-Boc-(4-Aminomethyl)-Piperidid

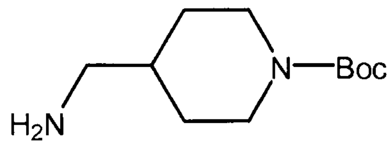

210,4 mg (1 mmol) 4-(Tfa-Amidomethyl)-Piperidin (Steinmetzer et al., J. Enz. Inhib., 1999, 14, 203-216) wurden mit 174 μL (1 mmol) DIPEA in 2,5 mL DMF gelöst. Unter Eiskühlung wurden 240 mg (1,1 mmol) $Boc_2O$ zugegeben, der Ansatz wurde 1 h unter Eiskühlung und über Nacht bei RT gerührt, anschließend das LM i.V. entfernt, der Rückstand in EE aufgenommen, 3 x mit 5 % $KHSO_4$-Lsg. und 3 x mit ges. NaCl-Lösung gewaschen und mit $Na_2SO_4$ getrocknet. Das LM wurde erneut i.V. entfernt (öliger Rückstand, der bei 4 °C langsam kristallisiert). Das Zwischenprodukt wurde mit 3 mL Dioxan und 3 mL 1 M NaOH gelöst und bei 40 °C für 3 h gerührt. Im Anschluß wurden 10 mL Wasser zugesetzt und das Produkt 3 x mit DCM extrahiert. Die vereinten DCM-Phasen wurden mit $Na_2SO_4$ getrocknet und anschließend das LM i.V. entfernt (weißer Feststoff, HPLC: Start bei 1 % B, Elution bei 26,6 min).

Schritt b) Phac-Arg-Val-Arg-4-(Amidomethyl)-Piperidin x 3 TFA = **Inhibitor 3a**

Die Kopplung von N-Boc-(4-Aminomethyl)-Piperidid an Phac-Arg(Pbf)-Val-Arg(Pbf)-OH (erhalten aus Schritt b des Synthesebeispiels 1) aus erfolgte analog des Schritts c aus Synthesebeispiel 1. Nach Abspaltung der am Peptid verbliebenen Pbf- und Boc-Schutzgruppen mit einer Mischung aus 95 % TFA, 2,5 % Triisopropylsilan und 2,5 % Wasser (v/v) über einen Zeitraum von 2 h bei RT wurde das in der Abspaltlösung enthaltene Zwischenprodukt durch Zugabe von Diethylether gefällt und abzentrifugiert, das Präzipitat 2 x mit Diethylether gewaschen und i.V. getrocknet. Das Produkt wurde durch präparative HPLC gereinigt, die produktenthaltenden Fraktionen wurden vereint und lyophilisiert (weißes Pulver, HPLC: Start bei 1 % B, Elution bei 22,7 min, MS ber. 643,43, MS gef. 644,5 $(M+H)^+$.

Schritt c) Phac-Arg-Val-Arg-4-(Amidomethyl)-N-Amidinopiperid x 3 TFA = **Inhibitor 3**

Der Aufbau der Guanidinogruppe erfolgte durch Umsetzung des Phac-Arg-Val-Arg-4-(Amidomethyl)-Piperidin x 3 TFA mit Pyrazolcarboxamidin x HCl und DIPEA analog der im Schritt b des Beispiels 2 beschriebenen Prozedur. Das Produkt wurde durch präparative HPLC gereinigt und lyophilisiert (weißes Pulver, HPLC: Start bei 1 % B, Elution bei 24,0 min, MS ber. 685,5, MS gef. 686,6 $(M+H)^+$.

**Beispiel 4**

Analog der Synthesestrategie für die Inhibitoren 2 und 2a wurden weitere Verbindungen hergestellt. Für den ersten Syntheseschritt, die Beladung des Tritylchlorid-Harzes (je ca. 100 mg mit einer Ausgangsbeladung von 1,5 mmol/g) wurde jeweils das entsprechende Diamin-Derivat eingesetzt, beispielsweise 1,3-Diaminopropan für die Inhibitoren 4 und 4a, oder p-Diaminoxylol für die Verbindungen 6 und 6a. Die Strukturen der Inhibitoren sind in Tabelle 1 zusammengefasst, alle Verbindungen wurden nach präparativer HPLC als TFA-Salze erhalten.

**Tabelle: 1** Hemmstoffe der allgemeinen Struktur Phac-Arg-Val-Arg-P1.

| Nr. des Inhibitors | P1 | HPLC (% B) MS ber. MS qef. (M+H)$^+$ |
|---|---|---|
| **4** | 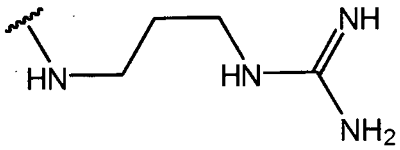 | 23,7<br>645,4<br>646,5 |
| **4a** | 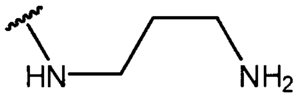 | 23,0<br>603,4<br>604,6 |
| **5** | 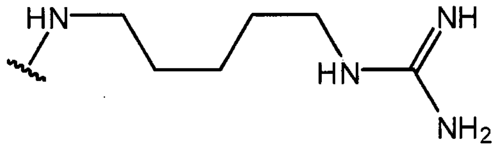 | 25,4<br>673,4<br>674,7 |
| **5a** | 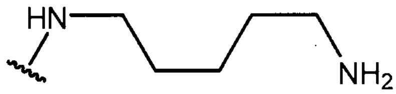 | 23,8<br>631,4<br>632,5 |
| **6** | 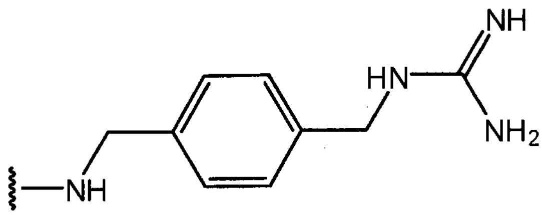 | 26,3<br>707,4<br>354,7* |
| **6a** | 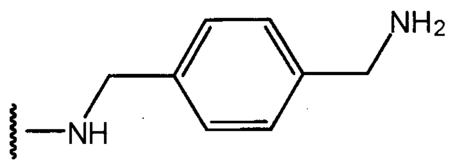 | 25,2<br>656,4<br>333,7* |
| **7** | 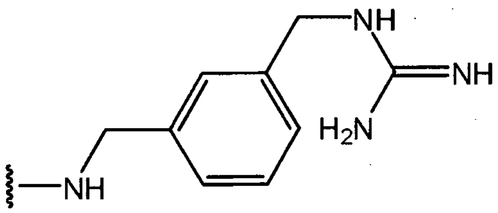 | 26,9<br>707,4<br>708,6 |
| **7a** | 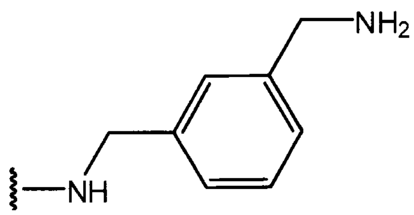 | 25,7<br>656,4<br>333,8* |
| | | *oder (M+2H)$^{2+}$/2 |

**Beispiel 5**

Die Inhibitoren 8-11 wurden entsprechend der folgenden Synthesestrategie nach oben beschriebenen Standardverfahren hergestellt, als Lysin-Drivat wurde Fmoc-Lys(Cbz)-OH für die Festphasenpeptidsynthese eingesetzt.

Schritt a) Synthese von Phac-Arg(Pbf)-Val-Lys(Cbz)-NH-$(CH_2)_4$-NH-Trityl-Harz

Die Synthese erfolgte analog des Schritts a) im Synthesebeispiel 2 unter Verwendung von Tritylchlorid-Harz und Fmoc-Lys(Cbz)-OH als Baustein, allerdings ohne Abspaltung der Schutzgruppen und des Peptids vom Harz.

Schritt b) Phac-Arg-Val-Lys(Cbz)-NH-$(CH_2)_4$-$NH_2$ als 2 x TFA-Salz = **Inhibitor 8**

Die Abspaltung der Pbf-Schutzgruppe und des Peptids vom Phac-Arg(Pbf)-Val-Lys(Cbz)-NH-$(CH_2)_4$-NH-Trityl-Harz erfolgte mit einer Mischung aus 95 % TFA, 2,5 % Triisopropylsilan und 2,5 % Wasser (v/v) über einen Zeitraum von 1,5 h bei RT unter Erhalt von Phac-Arg-Val-Lys(Cbz)-NH-$(CH_2)_4$-$NH_2$ als 2 x TFA-Salz. Das Produkt wurde mit Diethylether gefällt, 2 x mit Diethylether gewaschen und durch präparative HPLC gereinigt (Inhibitor 8, weißes lyophilisiertes Pulver nach präparativer HPLC, HPLC: Start bei 1 % B, Elution bei 36,2 min, MS ber. 723,4, MS gef. 724,6 $(M+H)^+$).

Schritt c) Phac-Arg-Val-Lys(Cbz)-Agmatin x 2 TFA = **Inhibitor 9**

Phac-Arg-Val-Lys(Cbz)-NH-$(CH_2)_4$-$NH_2$ x 2 TFA (Inhibitor 8) wurde mit 3-fachem Überschuss an Pyrazolcarboxamidin x HCl und 4-fachem Überschuss an DIPEA in DMF umgesetzt. Das Produkt wurde durch präparative HPLC gereinigt (Inhibitor 9, weißes lyophilisiertes Pulver nach präparativer HPLC, HPLC: Start bei 1 % B, Elution bei 36,8 min, MS ber. 765,5, MS gef. 766,5 $(M+H)^+$.

Schritt d) Phac-Arg-Val-Lys-Agmatin x 3 TFA = **Inhibitor 10**

Phac-Arg-Val-Lys(Cbz)-Agmatin x 2 TFA (Inhibitor 9) wurde mit $H_2$ und 10 Massenprozent Pd/C als Katalysator in 90 % Essigsäure 12 h bei RT hydriert und das Produkt durch präparative HPLC gereinigt (Inhibitor 10, weißes lyophilisiertes Pulver nach präparativer HPLC, HPLC: Start bei 1 % B, Elution bei 23,1 min, MS ber. 631,4, MS gef. 316,7 $(M+2H)^{2+}/2$).

Schritt e) Phac-Arg-Val-hArg-Agmatin x 3 TFA = **Inhibitor 11**

Phac-Arg-Val-Lys-Agmatin x 3 TFA (Inhibitor 10) wurde mit einer Mischung aus gleichen Volumina DMF und 1 M $Na_2CO_3$-Lösung mit ca. 3 eq. Pyrazolcarboxamidin x HCl und 4 eq. DIPEA analog Schritt b des Beispiels 2 umgesetzt (Inhibitor 11, weißes lyophilisiertes Pulver nach präparativer HPLC, HPLC: Start bei 1 % B, Elution bei 24,3 min, MS ber. 673,4, MS gef. 674,4 $(M+H)^+$.

**Beispiel 6**

Enzymkinetische Untersuchungen zur Bestimmung der Hemmwirkung auf Furin

Die Bestimmung der Hemmwirkung wurde mit einem Fluoreszenz-Plattenreader Safire$^2$ der Firma Tecan ($\lambda_{Ex}$ = 380 nm, $\lambda_{Em}$ = 460 nm) und Pyr-Arg-Thr-Lys-Arg-AMC (Bachem, Nr.: I-1650) als Substrat durchgeführt. Rekombinant hergestelltes humanes Furin (Kacprzak, M.M. et al., J. Biol. Chem., 2004, 279, 36788-36794) wurde im Messansatz in einer Konzentration von ca. 0,95 nM verwendet.
Für die Bestimmung der Hemmkonstanten wurde bei einer gleichbleibenden Substratkonzentration (12,5 μM im Ansatz) die Inhibitorkonzentration mindestens über den Bereich einer Größenordnung variiert, und jeweils die steady-state Geschwindigkeit bestimmt. Parallel zu jeder Inhibitormessung wurden mittels einer v/S-Charakteristik $K_m$ und $V_{max}$ ermittelt. Die Berechnung der $K_i$-Werte erfolgte durch Anpassung der bestimmten Geschwindigkeiten als Funktion der Inhibitorkonzentration an die Geschwindigkeitsgleichung für kompetitiv reversibelbindende Inhibitoren:

$$v = \frac{V_{max} \cdot [S]}{K_m \cdot (1 + \frac{[I]}{K_i}) + [S]}$$

**Tabelle 2:** $K_i$-Werte (μM) ausgewählter Inhibitoren für die Hemmung von Furin

| Inhibitor | $K_i$-Wert (μM) |
|---|---|
| 1 | 0,0014 |
| 2 | 0,077 |
| 2a | 25 |
| 3 | 0,068 |
| 3a | 10 |
| 4 | 0,097 |
| 4a | 1,3 |
| 5 | 1,6 |
| 10 | 0,049 |
| 11 | 0,047 |

**Beispiel 7**

**Hemmung der proteolytischen Spaltung des Hämagglutinins (HA) des Vogelgrippevirus (FPV)**

Das HA-Oberflächenprotein des Vogelgrippevirus (gehört zu den hochpathogenen Influenzaviren der Subtypen H5 und H7) wird als Vorläuferprotein HA0 hergestellt. HA0 wird an einer multibasischen Spaltstelle durch die Protease Furin oder durch ein furinähnliches Enzym hydrolysiert, diese Proteasen kommen praktisch in allen Zellen vor. Dabei entsteht die Untereinheit HA1 und die membrangebundene Untereinheit HA2. Diese Spaltung ist Voraussetzung für die Bildung infektiöser Viren, damit sie mit der Endosomenmembran der Zielzelle fusionieren können. Inhibiert man die proteolytische Spaltung von HA in HA1 und HA2, werden die Fusion und damit auch die weitere Vermehrung der Viren gehemmt. Mit Vogelgrippeviren infizierte Zellen wurden mit unterschiedlichen Konzentrationen des Inhibitors 1 und eines bereits früher beschriebenen Kontrollinhibitors (Decanoyl-Arg-Val-Lys-Arg-Chlormethylketon, Hallenberger, S. et al., Nature, 1992, 360, 358-361) versetzt und die Spaltung des HA untersucht.

In Figur 1 ist die Hemmung der HA-Spaltung des Vogelgrippevirus durch unterschiedliche Konzentrationen des Inhibitors 1 und des Referenzinhibitors Dec-Arg-Val-Lys-Arg-CMK dargestellt.

Das Gelbild A zeigt dabei die Ergebnisse folgenden Experimentes: Konfluente MDCK Zellkulturen wurden mit Influenzavirus A/FPV/Rostock/34 (H7N1) bei einer MOI von 10 pro Zelle infiziert. 16 Stunden nach Infektion wurden die Zellen lysiert und die Hemmung der HA-Spaltung (PSKKRKKR↓GLFG) in Abhängigkeit der Inhibitorkonzentration mittels SDS-PAGE und Western-Blotting untersucht. Die viralen Proteine wurden immunchemisch mit einem Kaninchen-Anti-FPV-Serum unter Verwendung des ECL-Tests der Firma Pierce nachgewiesen: HA0 (82 kDa), Nucleoprotein NP (56 kDa) und HA1 (~50 kDa), wobei HA2 (32 kDa) mit dem verwendeten Antiserum nicht nachweisbar ist.

Die statistische Auswertung ist in Bild B der Figur 1 dargestellt. Man erkennt die Quantifizierung der Hemmung der HA-Spaltung. Es wurden jeweils drei unabhängige Experimente für beide Inhibitoren durchgeführt, die Quantifizierung erfolgte mittels eines zweiten monoklonalen Antikörpers, der mit einem IR-Farbstoff markiert und mit dem LI-COR Odyssey Image-System nachweisbar ist. Die maximale Menge an HA0 bei 50 μM Dec-Arg-Val-Lys-Arg-CMK wurde als 100 %ige Hemmung der Spaltung gesetzt. Die Intensitäten der HA0-Banden bei den unterschiedlichen Inhibitorkonzentrationen wurden mit der Intensität der Banden für das Nucleoprotein (NP bei 56 kDa) normiert.

**Beispiel 8**

**Hemmung multizyklischer Replikation des FPV in Gegenwart des Inhibitors 1**

In einem weiteren Experiment wurden die Langzeiteffekte des Inhibitors 1 auf die multizyklische Vermehrung des Vogelgrippevirus (FPV) untersucht (Methode siehe Garten et al., Virology 1989, 172, 25-31). MDCK-Zellen wurden mit FPV bei niedriger Multiplizität der Infektion (MOI) von $10^{-5}$ infiziert und in Gegenwart einer Konzentration von 25 μM an Inhibitor 1 über einen Zeitraum von 72 h inkubiert. Zur Kontrolle wurden die infizierten Zellen ohne Inhibitor inkubiert. Die Vermehrung des FPV wurde durch Virustitration in den Zellkulturüberständen mit einem Standard Hämagglutinations-Test untersucht (Garten et al., Virology 1989, 172, 25-31, Figur 3). Die Virusfreisetzung in der mit dem Inhibitor behandelten Gruppe ist im Vergleich zur unbehandelten Kontrollgruppe um ca. 24 h verzögert. Die verzögerte Virusvermehrung in Gegenwart des Inhibitors macht auch deutlich, dass die Vitalität der Zellen durch den Inhibitor nicht beeinflusst wird.

Figur 2 zeigt die multizyklische Vermehrung des FPV in Zellkultur in Gegenwart (ungefüllte Balken) von Inhibitor 1 (25 μM) und ohne Inhibitor (gefüllte Balken). MDCK-Zellkulturen wurden mit dem Influenza-Stamm A/FPV/Rostock/34

(H7N1) bei einem MOI von $10^{-5}$ pro Zelle bei 37 °C inkubiert. Bei den angegebenen Zeitpunkten (10, 18, 32, 48, 60, and 72 h nach Infektion) wurde die Virusmenge im Medium mit dem Hämagglutinationstest bestimmt. Es sind die Mittelwerte von vier unabhängigen Experimenten gezeigt.

**Beispiel 9**

Entsprechend der oben beschriebenen Methoden wurden auch für die in Tabelle 3 dargestellten synthetisierten Inhibitoren die Ki-Werte zur Hemmung des Furins bestimmt.

**Tabelle 3:** Strukturen weiterer synthetisierter Inhibitoren und $K_i$-Werte zur Hemmung des Furins für ausgewählte Inhibitoren (n.b. = nicht bestimmt).

| Nr. | Struktur | HPLC min | MS ber. | MS gef. | $K_i$ ($\mu$M) |
|---|---|---|---|---|---|
| 12 | 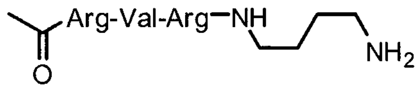 | 13,64 | 541,69 | 542,21 $(M+H)^+$ | 41,21 |
| 13 | 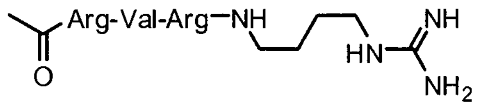 | 15,86 | 583,73 | 195,45 $(M+3H)^{3+}$ | 0,315 |
| 14 | 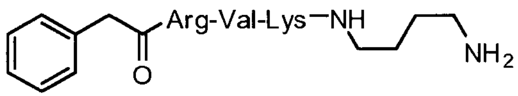 | 21,76 | 589,41 | 590,41 $(M+H)^+$ | 5,41 |
| 15 | 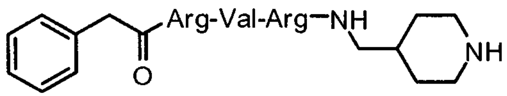 | 23,12 | 643,43 | 644,4 $(M+H)^+$ | 9,708 |
| 16 | 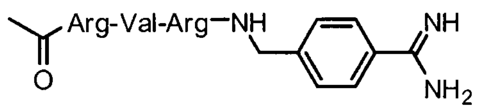 | 16,83 | 602,38 | 201,84 $(M+3H)^{3+}$ | 0,002 |
| 17 | 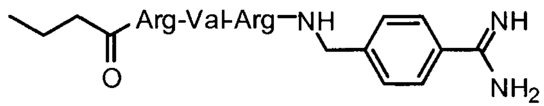 | 20,40 | 630,41 | 316,26 $(M+2H)^{2+}$ | 0,00077 |
| 18 | 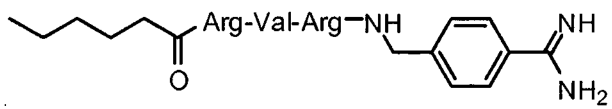 | 26,29 | 658,44 | 330,3 $(M+2H)^{2+}$ | 0,00082 |
| 19 | 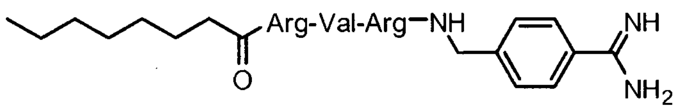 | 32,73 | 686,47 | 344,29 $(M+2H)^{2+}$ | 0,00082 |
| 20 | 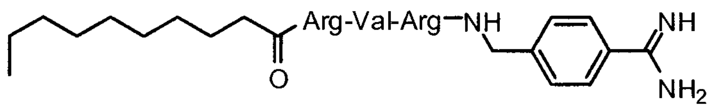 | 38,78 | 714,5 | 239,26 $(M+3H)^{3+}$ | 0,0024 |
| 21 | 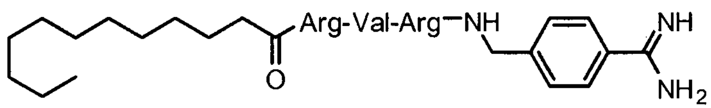 | 15,16 | 742,53 | 372,32 $(M+H)^+$ | 0,0067 |

(fortgesetzt)

| Nr. | Struktur | HPLC min | MS ber. | MS gef. | $K_i$ (μM) |
|---|---|---|---|---|---|
| 22 | 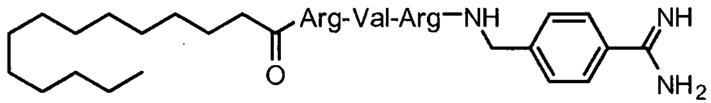 | 20,40* | 770,56 | 386,37 $(M+2H)^{2+}$ | 0,0569 |
| 23 | 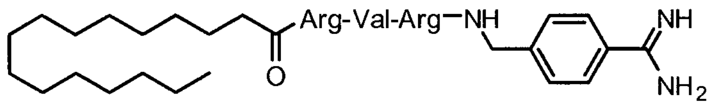 | 25,38* | 798,6 | 267,28 $(M+3H)^{3+}$ | 0,024 |
| 24 | 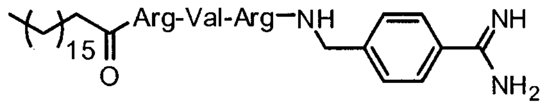 | 30,66* | 826,63 | 276,63 $(M+3H)^{3+}$ | 2,46 |
| 25 | 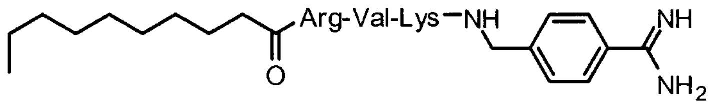 | 38,12 | 686,5 | 344,29 $(M+2H)^{2+}$ | 0,0033 |
| 26 | 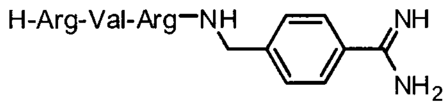 | 15,12 | 560,37 | 561,3 $(M+H)^{+}$ | 0,0076 |
| 27 | 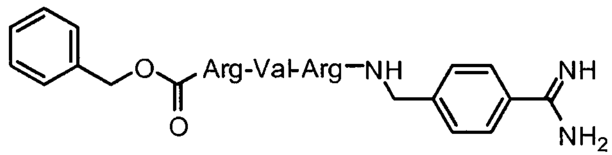 | 27,78 | 694,4 | 232,57 $(M+3H)^{3+}$ | 0,0024 |
| 28 | 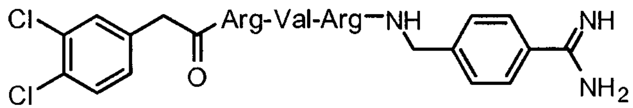 | 31,72 | 746,33 | 374,24 $(M+2H)^{2+}$ | 0,0012 |
| 29 | 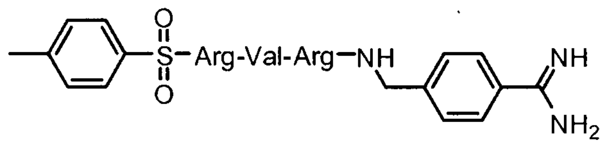 | 27,02 | 714,37 | 358,25 $(M+2H)^{2+}$ | 0,0048 |

(fortgesetzt)

| Nr. | Struktur | HPLC min | MS ber. | MS gef. | $K_i$ (μM) |
|---|---|---|---|---|---|
| 30 | 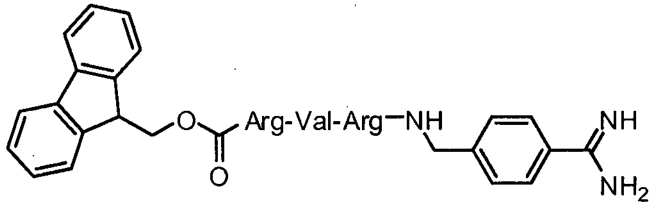 | 36,80 | 782,43 | 261,93 $(M+3H)^{3+}$ | 0,011 |
| 31 | 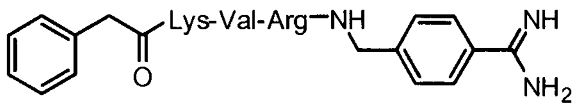 | 27,00 | 650,4 | 326,3 $(M+2H)^{2+}$ | 0,285 |
| 32 | 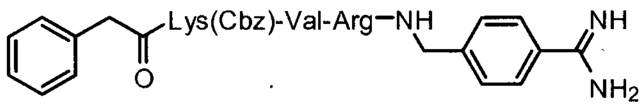 | 39,49 | 784,44 | 393,26 $(M+2H)^{2+}$ | 0,7 |
| 33 | 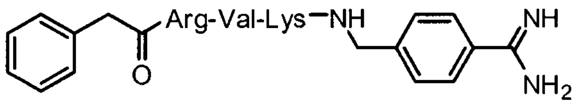 | 23,98 | 650,4 | 326,22 $(M+2H)^{2+}$ | 0,0015 |
| 34 | 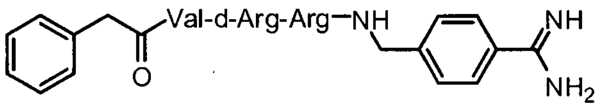 | 25,95 | 678,41 | 227,24 $(M+3H)^{3+}$ | 7,34 |
| 35 | 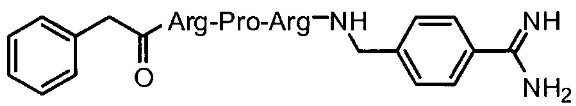 | 22,77 | 676,39 | 226,5 $(M+3H)^{3+}$ | 0,031 |
| 36 | 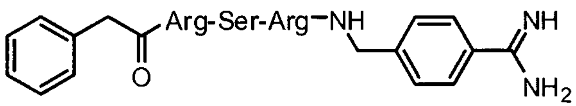 | 20,70 | 666,37 | 334,26 $(M+H)^{+}$ | 0,015 |
| 37 | 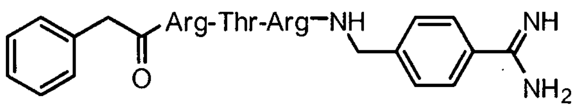 | 21,40 | 680,39 | 341,26 $(M+H)^{+}$ | 0,005 |
| 38 | 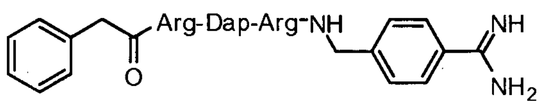 | 19,87 | 665,39 | 666,22 $(M+H)^{+}$ | 0,0028 |

(fortgesetzt)

| Nr. | Struktur | HPLC min | MS ber. | MS gef. | $K_i$ (μM) |
|---|---|---|---|---|---|
| 39 | 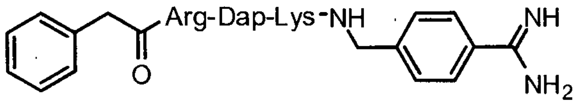 | 19,06 | 637,38 | 638,25 $(M+H)^+$ | 0,0037 |
| 40 | 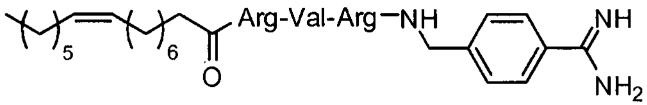 | 54,50 | 796,6 | 399,8 $(M+2H)^{2+}$ | n.b. |
| 41 | 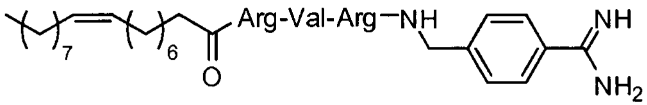 | 58,20 | 824,6 | 413,63 $(M+2H)^{2+}$ | n.b. |
| 42 | 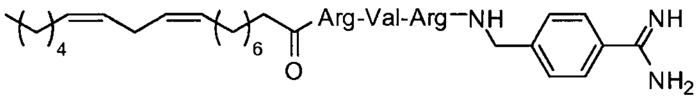 | 55,10 | 822,6 | 412,56 $(M+2H)^{2+}$ | n.b. |
| 43 | 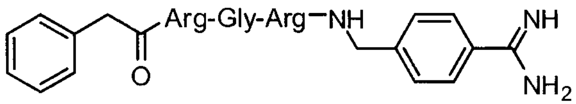 | 21,40 | 636,4 | 319,32 $(M+2H)^{2+}$ | 0,042 |
| 44 | 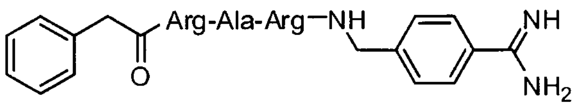 | 21,80 | 650,4 | 326,35 $(M+2H)^{2+}$ | 0,019 |
| 45 | 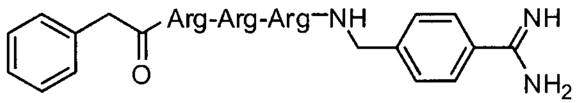 | 21,50 | 735,4 | 368,83 $(M+2H)^{2+}$ | 0,0026 |
| 46 | 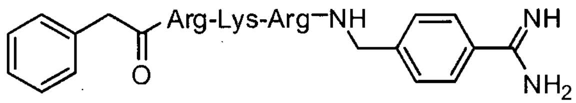 | 19,90 | 707,4 | 354,83 $(M+2H)^{2+}$ | n.b. |
| 47 | 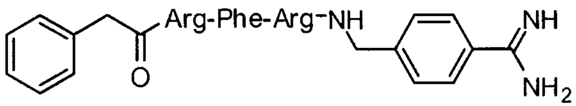 | 29,90 | 726,4 | 364,32 $(M+2H)^{2+}$ | n.b. |
| 48 | 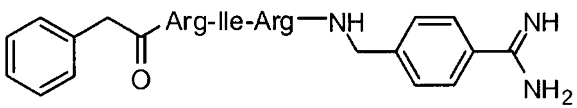 | 27,10 | 692,4 | 347,34 $(M+2H)^{2+}$ | n.b. |

(fortgesetzt)

| Nr. | Struktur | HPLC min | MS ber. | MS gef. | $K_i$ (µM) |
|---|---|---|---|---|---|
| 49 | 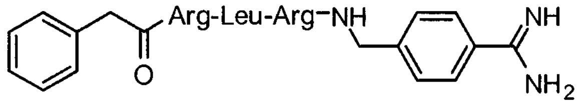 | 27,50 | 692,4 | 347,32 $(M+2H)^{2+}$ | n.b. |
| 50 | 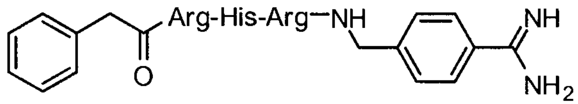 | 20,20 | 716,4 | 359,29 $(M+2H)^{2+}$ | n.b. |
| 51 | 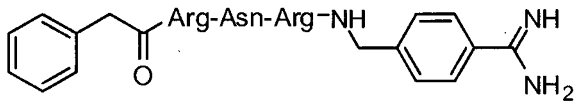 | 20,60 | 693,4 | 347,9 $(M+2H)^{2+}$ | n.b. |
| 52 | 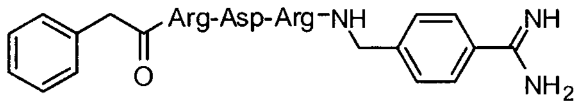 | 21,00 | 694,4 | 348,3 $(M+2H)^{2+}$ | n.b. |
| 53 | 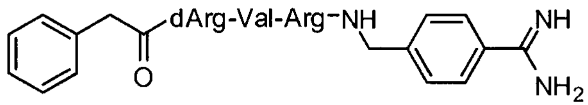 | 22,90 | 678,4 | 340,33 $(M+2H)^{2+}$ | n.b. |
| 54 | 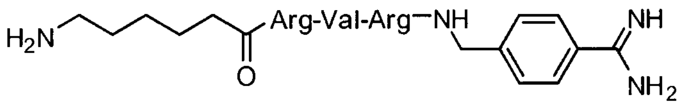 | 16,50 | 673,5 | 337,88 $(M+2H)^{2+}$ | n.b. |
| 55 | 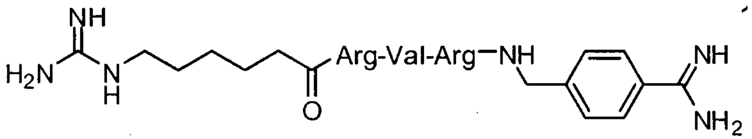 | 19,30 | 715,5 | 358,88 $(M+2H)^{2+}$ | n.b. |
| 56 | 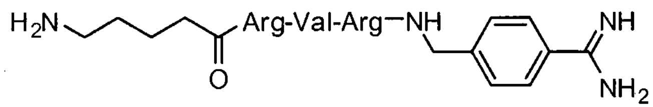 | 15,70 | 659,4 | 330,91 $(M+2H)^{2+}$ | n.b. |
| 57 | 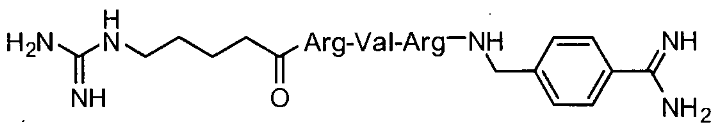 | 17,30 | 701,5 | 351,88 $(M+2H)^{2+}$ | n.b. |

(fortgesetzt)

| Nr. | Struktur | HPLC min | MS ber. | MS gef. | $K_i$ (µM) |
|---|---|---|---|---|---|
| 58 | 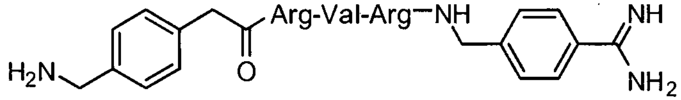 | 18,10 | 707,4 | 354,89 $(M+2H)^{2+}$ | n.b. |
| 59 | 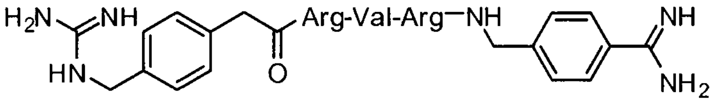 | 19,70 | 749,5 | 375,91 $(M+2H)^{2+}$ | n.b. |
| 60§ | 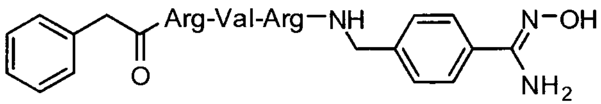 | 32,10 | 694,4 | 348,22 $(M+2H)^{2+}$ | n.b. |
| 61# | 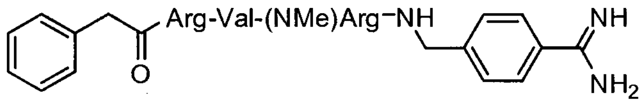 | 30,40 | 692,4 | 347,37 $(M+2H)^{2+}$ | n.b. |
| 62# | 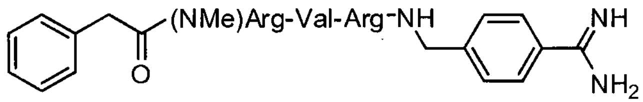 | 30,40 | 692,4 | 347,38 $(M+2H)^{2+}$ | n.b. |

*Start der HPLC bei 30 % B, bei allen anderen Verbindungen Start der HPLC bei 1 % B.

$ Bei dieser Verbindung handelt es sich um ein Hydroxyamidino-Prodrug des Inhibitors 1.

# Diese Verbindungen wurden unter Verwendung von Fmoc-N(Methyl)Arg(Mtr)-OH (Bachem, Schweiz, Bestellnummer B-2840) unter Verwendung von PyBOP und PyBrOP als Kopplungsreagenz nach bereits früher beschriebenen Methoden hergestellt (Steinmetzer et al., Biol. Chem. 381, 603-610, 2000).

## Patentansprüche

1. Verbindung gemäß Formel (I)

P5-P4-P3-P2-P1 (I)

wobei P5-P4-P3-P2-P1 ein Tetrapeptidderivat ist und wobei P1 ein Argininmimetikum, P2, P3, P4 jeweils ein Amino- und/oder Iminosäurerest und P5 eine N-terminale Modifizierung des Amino- oder Iminosäurerestes P4 ist, **dadurch gekennzeichnet, dass**
P1 ein decarboxyliertes Argininmimetikum, ausgewählt aus folgenden Strukturen, ist:

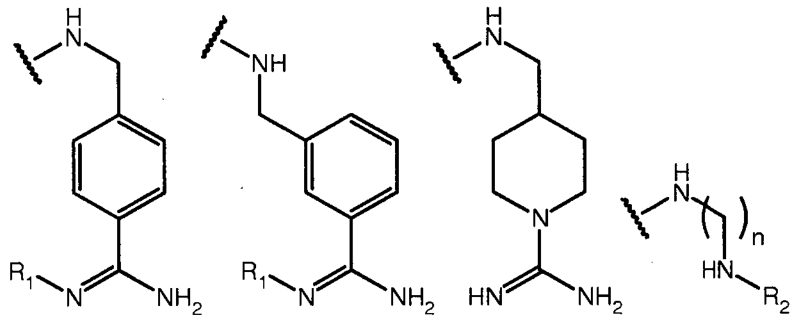

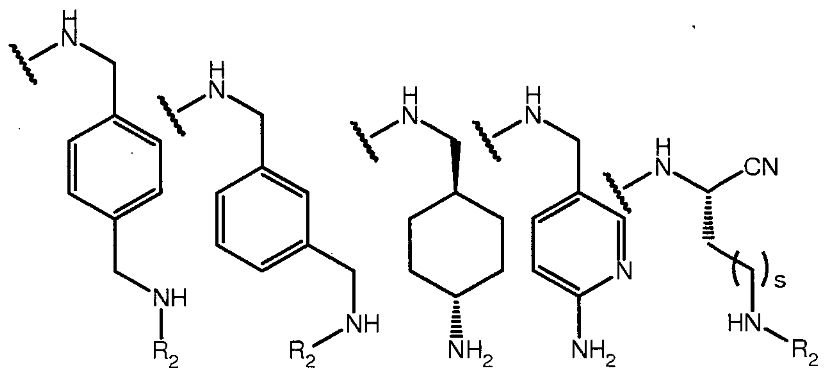

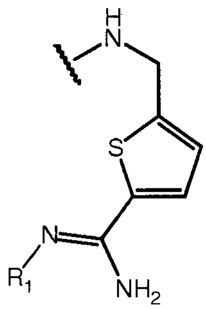

wobei

- $R_1$ ausgewählt ist aus der Gruppe H, OH, $O\text{-}CH_3$, $NH_2$, $O\text{-}CO\text{-}CH_3$ oder $\text{-}CO\text{-}O\text{-}(CH_2)_m\text{-}CH_3$ wobei m eine ganze Zahl von 1 bis 5 ist, und
- $R_2$ ein H oder eine Amidinogruppe ist, und
- n gleich 3, 4 oder 5 ist, und s gleich 2 oder 3 ist,

P2 und P4 unabhängig voneinander jeweils ein Amino- oder Iminosäurerest ist, ausgewählt aus folgenden Strukturen

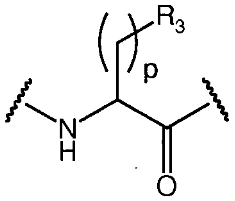 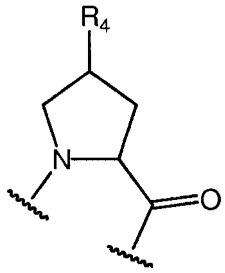

und wobei

- ■ p eine ganze Zahl von 0 bis 5 ist, und
- ■ $R_3$ eine Amino- oder eine Guanidinogruppe sein kann, oder eine -O-Guanidinogruppe, oder ein einfach oder mehrfach substituierter oder unsubstituierter Aryl- oder Heteroarylrest mit 5 bis 10 Ringatomen, wobei der Heteroarylring bis zu 3 Heteroatome enthalten kann ausgewählt aus N, S oder O, wobei der Substituent am Aryl- oder Heteroarylring ausgewählt ist aus $-NH_2$, $-CH_2-NH_2$, -Amidino, -Hydroxyamidino, -Guanidino, $-CH_2$-Guanidino, -Halogen, -Cl, -Br, -I, -CN, $-CF_3$, -Alkyl mit 1-3 C-Atomen oder -Alkoxy mit 1-3 C-Atomen, $-COOR_5$ mit $R_5$ ein Wasserstoff oder eine Alkyl mit 1 bis 3 C-Atomen, und
- ■ $R_4$ ein -H, -OH oder $-O-(CH_2)_q-R_6$ ist mit q eine ganze Zahl von 2 bis 4 und $R_6$ eine Amino- oder Guanidinogruppe ist;

P3 ein beliebiger natürlicher oder unnatürlicher $\alpha$-Amino- oder $\alpha$-Iminosäurerest mit L- oder D-Konfiguration ist und
P5 ausgewählt ist aus einer der Gruppen

- ■ -H, $-CO-R_7$ oder $-CO-X-R_7$, oder
- ■ Pyroglutamylrest oder ein anderen Amino- oder Iminosäurerest, oder
- ■ $-SO_2-R_7$, oder
- ■ unverzweigter oder verzweigter Alkylrest mit 1 bis 10-C-Atomen, wobei der Alkylrest gegebenenfalls mit einem Halogen, einer $NH_2$-, Guanidino-, COOH- oder $COO-R_5$-Gruppe, oder
- ■ ein mit einem bifunktionellen Rest acyliertes Shpingosin oder mit einem bifunktionellen Rest acyliertes Shpingosyl-phosphorylcholin folgender Strukturen

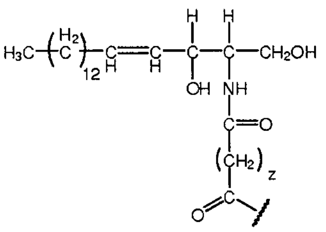 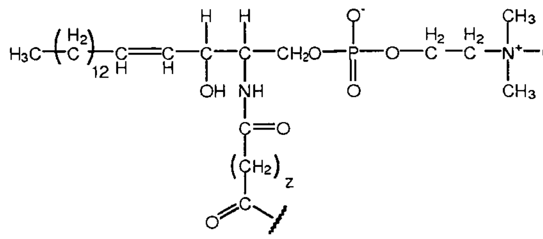

wobei

- ■ z eine ganze Zahl zwischen 2 und 20 ist,
- ■ X gleich O oder NH ist,
- ■ $R_5$ ein Wasserstoff oder eine Alkylgruppe mit 1 bis 3 C-Atomen, und
- ■ $R_7$ ein

  i) unsubstituierter oder gegebenenfalls einfach oder mehrfach substituierter verzweigter oder unverzweigter, gesättigter oder ungesättigter Alkylrest mit 1 bis 24 C-Atomen und 0 bis 3 Doppelbindungen ist, oder
  ii) ein unsubstituierter oder gegebenenfalls einfach oder mehrfach substituierter Aryl-, Heteroaryl-, Aralkyl-, Heteroaralkyl- oder **Cycloalkylrest** mit 5 bis 20 Kohlenstoffatomen ist, wobei der Heteroarylring bis zu 3 Heteroatome enthalten kann ausgewählt aus N, S oder O, wobei der oder die gegebenenfalls vorhandenen Substituenten an $R_7$ unabhängig voneinander ausgewählt werden aus $-NH_2$, $-CH_2-NH_2$, -Amidino, -Hydroxyamidino, -Guanidino, $-CH_2$-Guanidino, -Halogen, -Cl, -Br, -I, -CN, $-CF_3$,

-Alkyl mit 1-3 C-Atomen oder Alkoxy mit 1 bis 3 C-Atomen, -COO-$R_5$ oder ein Cholesterolrest ist, wobei P5 bevorzugt ausgewählt ist aus der Gruppe -CO-$R_7$, - CO-X-$R_7$ oder $SO_2$-$R_7$, wobei P5 besonders bevorzugt ausgewählt ist aus den folgenden Strukturen

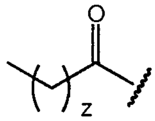

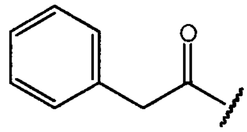

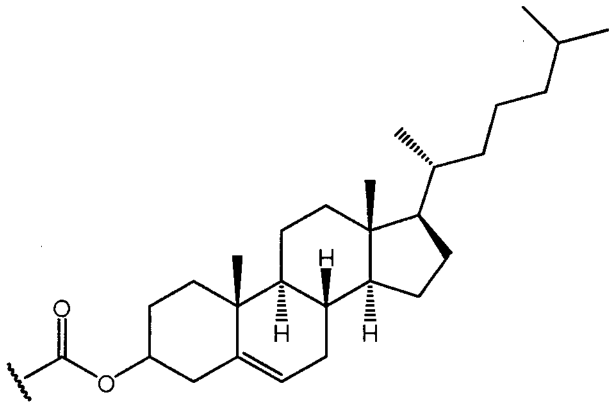

,

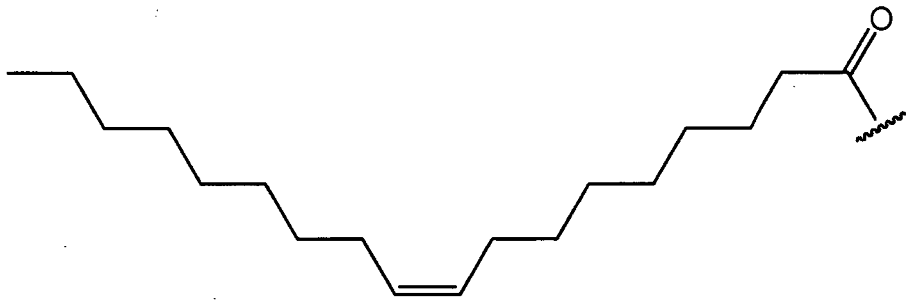

wobei z eine ganze Zahl von 1 bis 18 ist, besonders bevorzugt 8, 14 oder 16.

2. Verbindung nach Anspruch 1, **dadurch gekennzeichnet, dass** P1 bevorzugt ausgewählt ist aus folgenden Strukturen

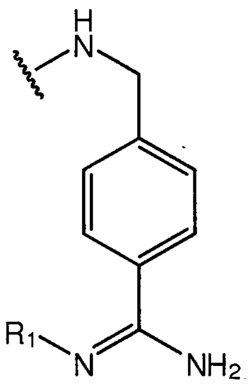

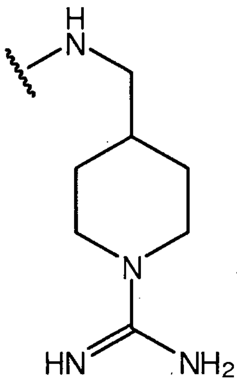

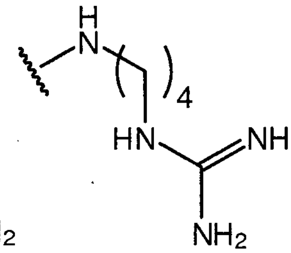

,

besonders bevorzugt

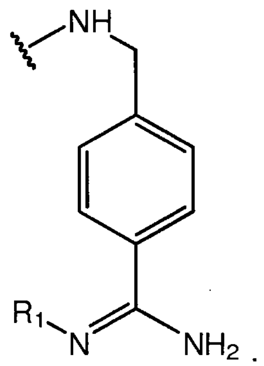

3. Verbindung nach Anspruch 2, **dadurch gekennzeichnet, dass** P2 in L - Konfiguration vorliegt und bevorzugt ausgewählt ist aus den Strukturen

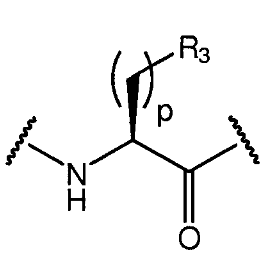

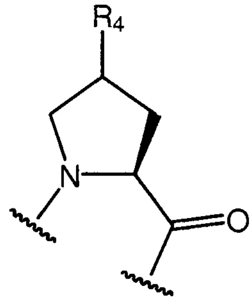

,

besonders bevorzugt

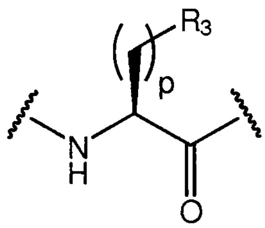

,

wobei R3 bevorzugt

- eine Amino-, Guanidino- oder -O-Guanidinogruppe ist oder ein substituierter Arylrest mit 6 Ringatomen oder ein unsubstituierter Pyridylrest ist, wobei der Stickstoff im Pyridylring an beliebiger Stelle stehen kann und gegebenenfalls auch als N-Oxid vorliegt, und wobei der Substituent am Arylring ausgewählt ist aus $-NH_2$, $-CH_2-NH_2$, -Amidino, -Hydroxyamidino, -Guanidino, oder $-CH_2$-Guanidino.

4. Verbindung nach Anspruch 3, **dadurch gekennzeichnet, dass** P4 ein Aminosäurerest in L-Konfiguration ist.

5. Verbindung nach Anspruch 4, **dadurch gekennzeichnet, dass** P3 ein nichtbasischer α-Amino- oder α-Iminosäurerest in L-Konfiguration, bevorzugt der Rest einer proteinogenen α-Amino- oder α-Iminosäure, ist.

6. Verbindung nach einem der Ansprüche 1 bis 5, **dadurch gekennzeichnet, dass**

- P1 ein 4-Amidinobenzylamid-Rest,
- P2 ein Arginyl-, Lysyl- oder Canavaninrest in L-Konfiguration,

- ■ P3 ein nichtbasischer α-Aminosäure- oder α-Iminosäurerest in L-Konfiguration,
- ■ P4 ein Arginyl-, Lysyl- oder Canavaninrest in L-Konfiguration, und
- ■ P5 ein Phenylacetyl- oder ein anderer Acylrest ist,

wobei die Verbindungen, einschließlich deren Salze,

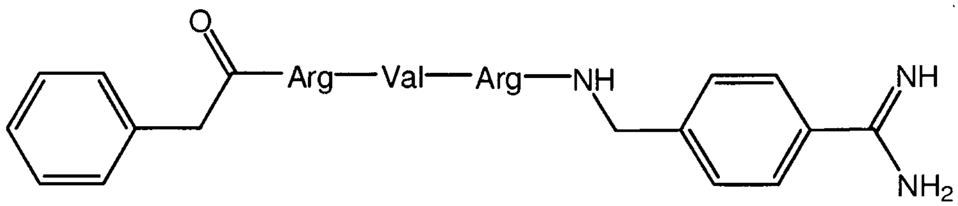

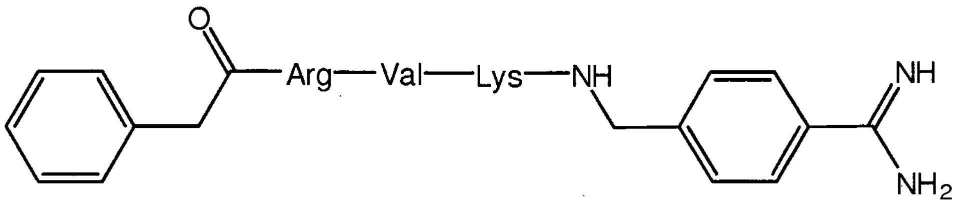

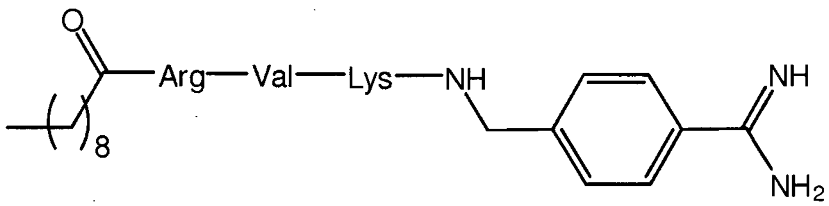

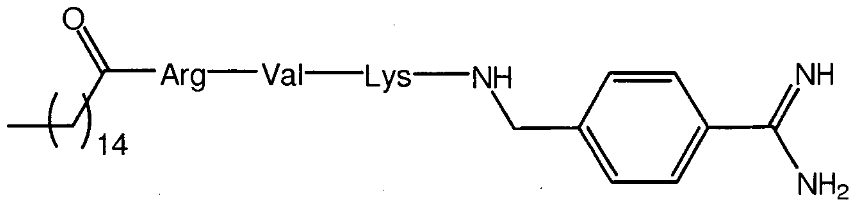

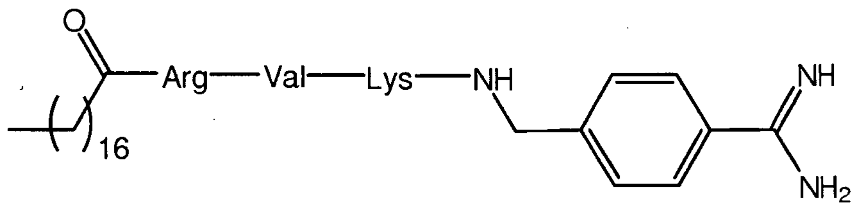

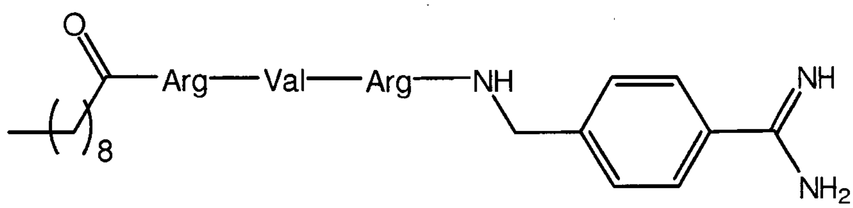

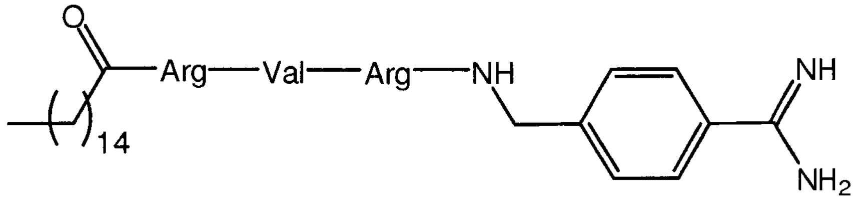

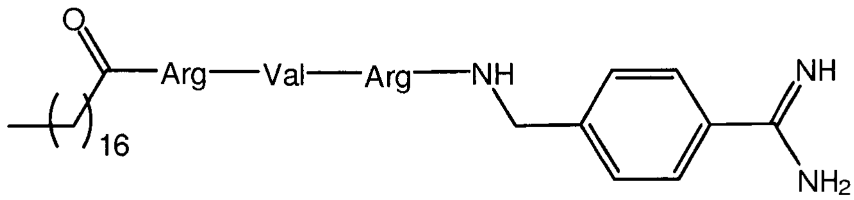

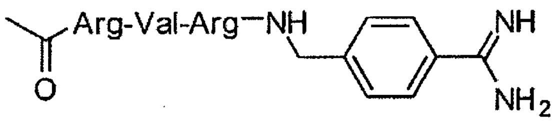

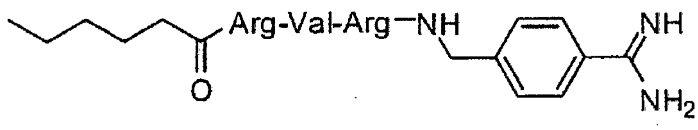

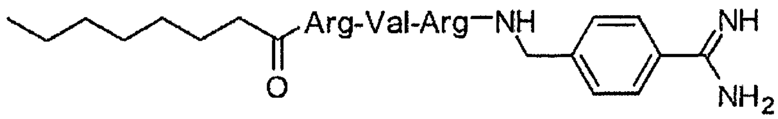

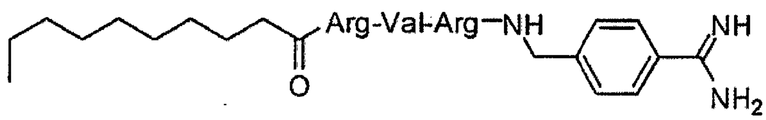

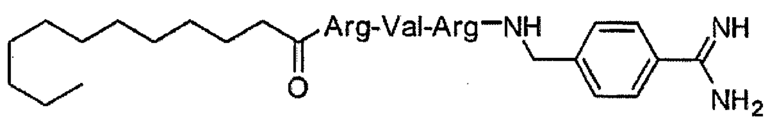

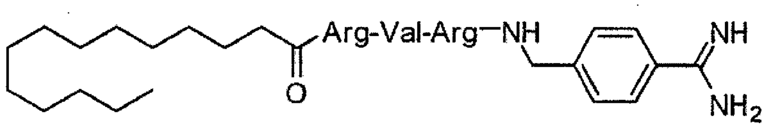

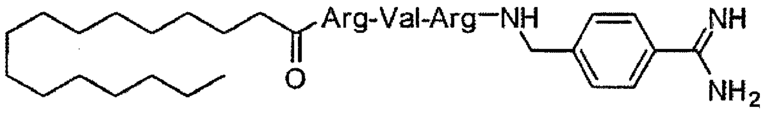

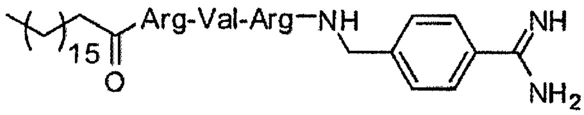

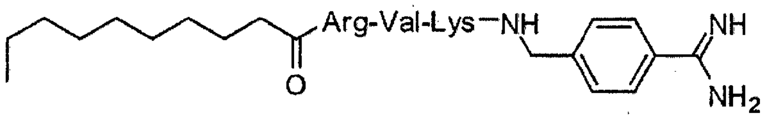

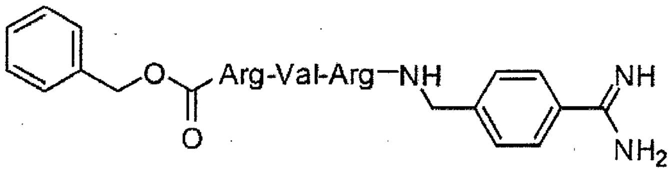

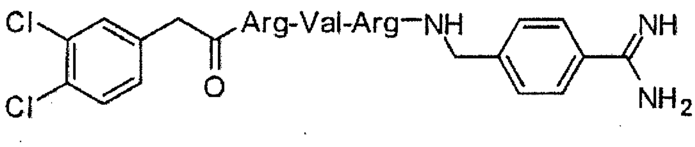

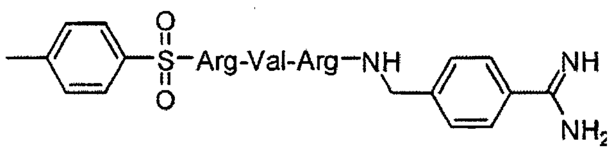

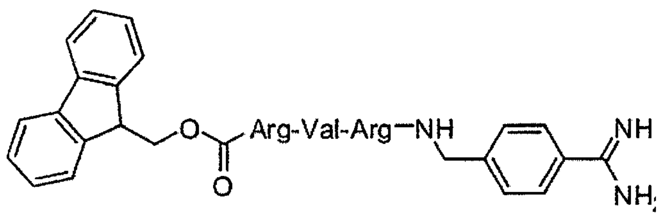

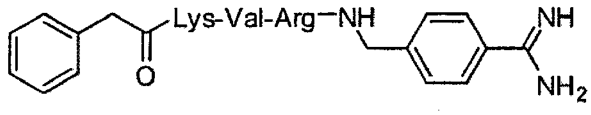

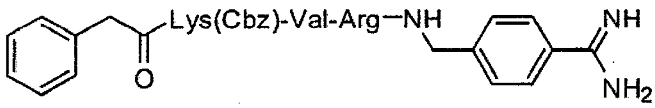

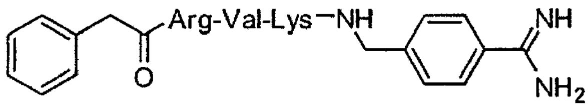

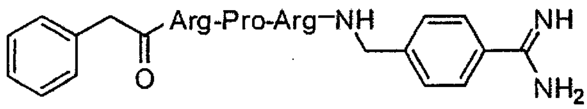

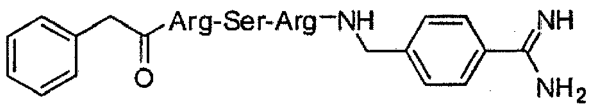

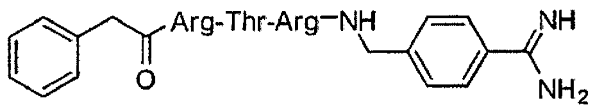

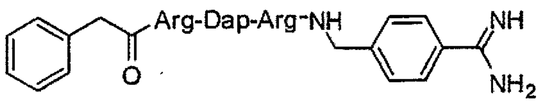

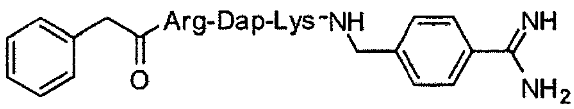

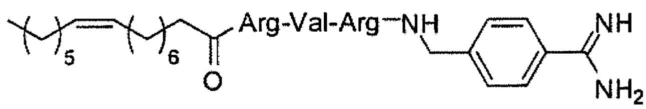

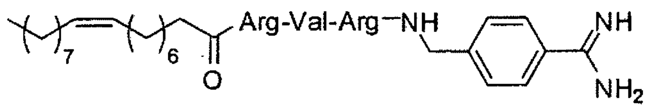

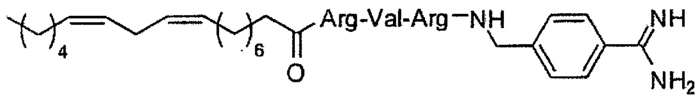

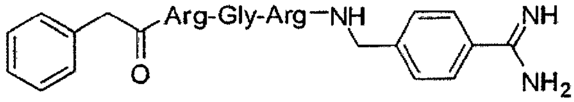

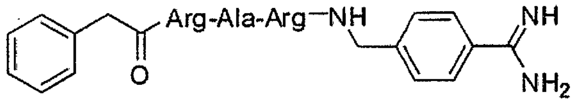

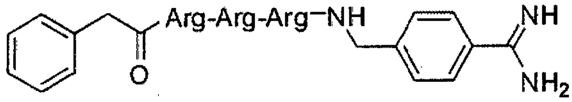

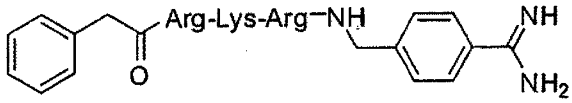

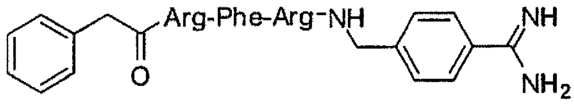

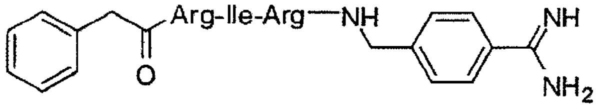

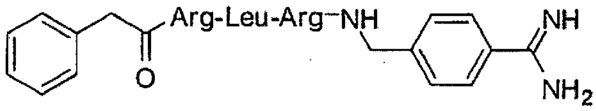

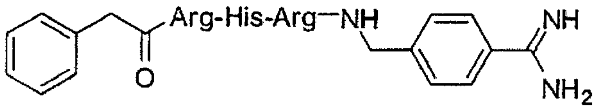

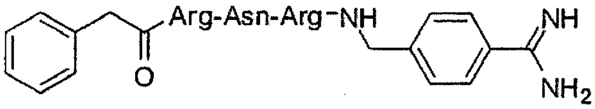

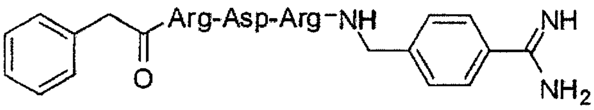

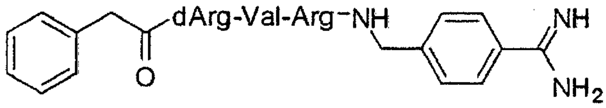

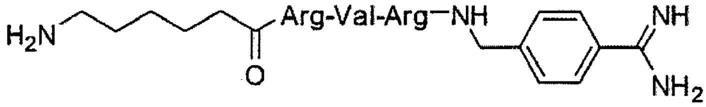

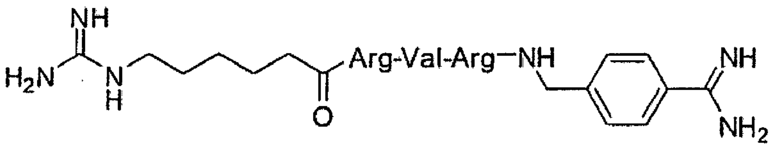

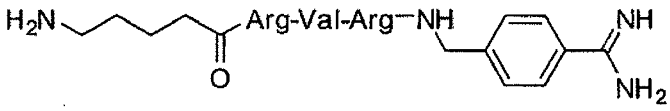

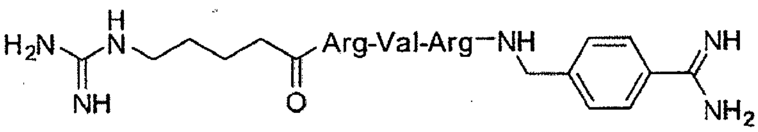

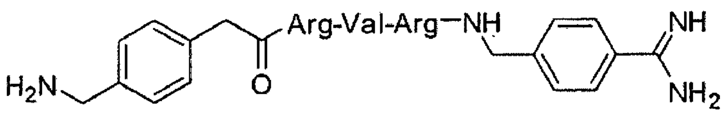

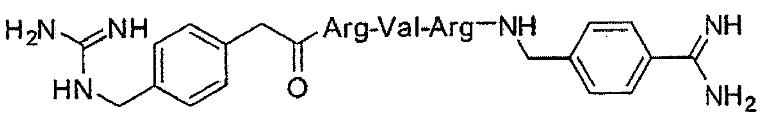

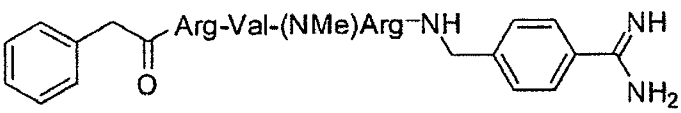

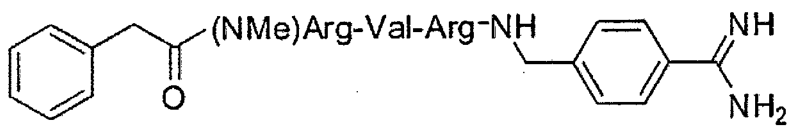

ganz besonders bevorzugt sind.

7. Verfahren zur Herstellung einer Verbindung gemäß einem der Ansprüche 1 bis 6 durch eine Kombination von Festphasen- und Lösungssynthese mittels folgender Schritte:

- ■ Synthese des Segments P5-P4-P3-P2 durch Festphasenpeptidsynthese,
- ■ Abspaltung des Peptidsegments vom Harz unter geeigneten sauren Bedingungen, wobei Seitenkettenschutzgruppen entweder erhalten oder abgespalten werden,
- ■ Kopplung eines geeigneten P1-Bausteins mittels racemisierungsfreier Standardverfahren, wobei der P1-Baustein entweder geschützt oder ungeschützt vorliegt,
- ■ Reinigung durch Kristallisation, Chromatographie, bevorzugt Umkehrphasen- oder Ionenaustauschchromatographie oder durch Gegenstromverteilung,

8. Verfahren zur Herstellung einer Verbindung gemäß einem der Ansprüche 1 bis 6 durch Lösungssynthese umfassend folgende Schritte:

a) Synthese eines an der terminalen Amidino- oder Guanidinogruppe geeignet geschützten P1 mit freier Aminogruppe, wobei die Aminogruppe entweder als Salz oder freie Base vorlieget, bevorzugt Synthese von folgender Verbindung:

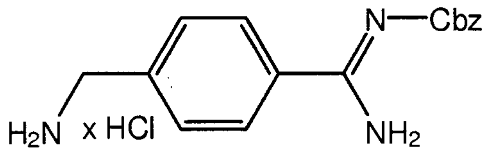

b) Kopplung einer Nα-Boc oder anderweitig geeignet Nα-urethangeschützten Amino- oder Iminosäure mit oder ohne geeigneter Seitenkettenschutzgruppe an die freie Aminogruppe des P1-Bausteins,
c) Aufarbeitung des Reaktionsansatzes durch gängige Wasch- und Kristallisationsverfahren
d) Abspaltung der Nα-Schutzgruppe unter Erhalt des P2-P1-Segments mit freier Aminogruppe am P2-Baustein,
e) Kopplung der Nα-Boc- oder anderweitig geeignet Nα-urethangeschützten P3-Amino- oder Iminosäure an die freie Aminogruppe des P2-P1-Segments,
f) Aufarbeitung des Reaktionsansatzes durch gängige Wasch- und Kristallisationsverfahren
g) Abspaltung der Nα-Schutzgruppe unter Erhalt des P3-P2-P1-Segments mit freier N-terminaler Aminogruppe am P3-Rest,
h) Kopplung der Nα-Boc oder anderweitig geeignet Nα-urethangeschützten P4-Amino- oder Iminosäure an die freie Aminogruppe des P3-P2-P1-Segments aus Schritt h)
i) Aufarbeitung des Reaktionsansatzes durch gängige Wasch- und Kristallisationsverfahren
j) Abspaltung der Nα-Schutzgruppe unter Erhalt des P4-P3-P2-P1-Segments mit freier N-terminaler Aminogruppe am P4-Rest,
k) Kopplung des P5-Restes an die freie Aminogruppe des P4-P3-P2-P1-Segments aus Schritt j)
l) Abspaltung aller noch vorhandenen Schutzgruppen,
m) Reinigung durch Kristallisation und/oder Chromatographie,

9. Arzneimittel, enthaltend mindestens eine Verbindung gemäß einem der Ansprüche 1 bis 6.

10. Verbindung gemäß einem der Ansprüche 1 bis 6 oder ein Arzneimittel gemäß Anspruch 9 zur Therapie oder Prophylaxe einer viral oder bakteriell bedingten Erkrankungen, sowie zur Therapie und Prophylaxe von Diabetes, Arteriosklerose, Krebs, Alzheimer oder Übergewicht, insbesondere in oraler, subkutaner, intravenöser oder transdermaler Form oder in Form eines Sprays zur Behandlung von Erkrankungen im Respirationstrakt, wobei die bakteriell bedingten Erkrankungen , insbesondere hervorgerufen sind durch Erreger aus der Gruppe *Pseudomonas spec., Shigella spec., Corynebacterium diphtheriae, Bacillus cereus, Bacilllus anthracis, Bacillus butulinum, Clostridium perfringens, Clostridium difficile, Clostridium septicum, Clostridium sprioforme, Bordetella pertussis, Samonella enterica* oder durch ausgewählten *Escherichia coli*-Stämmen, insbesondere *E.coli*-Stämme, die hämolytische Kolitits, das Hämolytische uraemisches Syndrom und/oder Nierenversagen verursachen, ganz insbesondere *E.coli* 0157-H7.

**11.** Verbindung gemäß einem der Ansprüche 1 bis 6 oder ein Arzneimittel gemäß Anspruch 9 zur Therapie oder Prophylaxe von Erkrankungen hervorgerufen durch Viren, insbesondere hervorgerufen durch Viren der Gruppe

- ■ hochpathogene aviäre Influenza A-Viren, vor allem der H5- bzw. H7-Subtypen,
- ■ RNA-Negativstrangviren wie den Parainfluenzaviren, das Masern-Virus, das Mumpsvirus, das Hundestaupevirus, das Respiratorische-Syncytial-Virus, Filoviren wie das Ebola- und Marburg-Virus und das Newcastle-Disease-Virus, sowie
- ■ Plusstrang-RNA-Viren, wie HIV-1, HIV-2, HTLV und andere Retroviren, insbesondere Coronaviren wie das SARS-Coronavirus, die Flaviviren, Gelbfieber-Viren, West-Nile-Virus, Dengue-Virus oder das Frühsommer-Meningo-Encephalitis-Virus, das Venezuelan equine encephalitis Virus oder das Rift Valley Fever Virus.

**12.** Verbindung gemäß einem der Ansprüche 1 bis 6 oder ein Arzneimittel gemäß Anspruch 9 zur Hemmung der Proproteinkonvertasen Furin, PC1/3, PC2, PC4, PC5/6, PACE4 und/oder PC7.

**13.** Verbindung gemäß einem der Ansprüche 1 bis 6 oder ein Arzneimittel gemäß Anspruch 9 zur Therapie von Krebs und zur Hemmung der Metastasierung.

## Claims

**1.** Compound according to formula (I)

$$P5\text{-}P4\text{-}P3\text{-}P2\text{-}P1 \qquad (I)$$

wherein P5-P4-P3-P2-P1 is a tetrapeptide derivative and wherein P1 is an arginine mimetic, P2, P3, P4 each denote an amino and/or imino acid group and P5 is an N-terminal modification of the amino or imino acid group P4, **characterised in that**
P1 is a decarboxylated arginine mimetic selected from among the following structures:

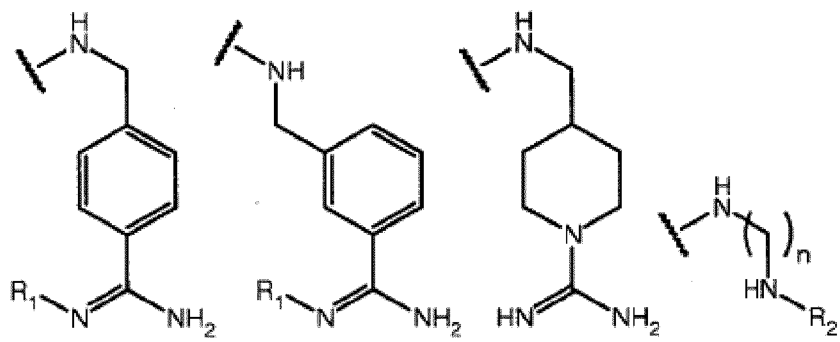

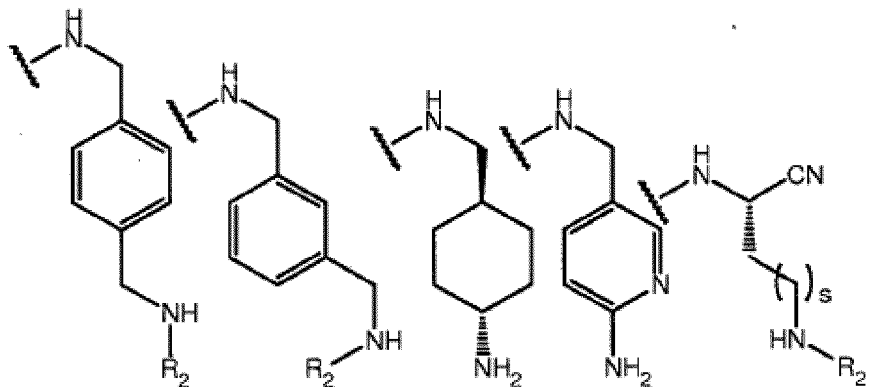

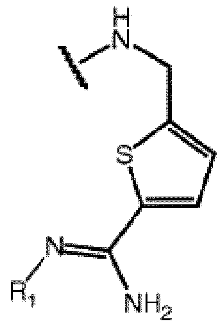

wherein

- $R_1$ is selected from among H, OH, O-$CH_3$, $NH_2$, O-CO-$CH_3$ or -CO-O-$(CH_2)_m$-$CH_3$ where m is an integer from 1 to 5, and
- $R_2$ is an H or an amidino group, and
- n is equal to 3, 4 or 5, and s is equal to 2 or 3,

P2 and P4 independently of one another each denote an amino or imino acid group, selected from among the following structures

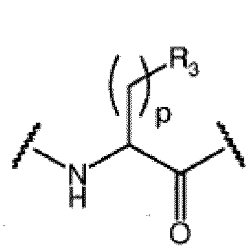

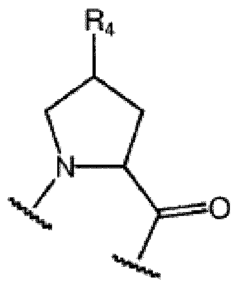

and wherein

- p is an integer from 0 to 5, and
- $R_3$ may be an amino or a guanidino group, or an -O-guanidino group, or a mono-or polysubstituted or unsubstituted aryl or heteroaryl group having 5 to 10 ring atoms, wherein the heteroaryl ring may contain up to 3 heteroatoms selected from among N, S or O, the substituent at the aryl or heteroaryl ring being selected from among -$NH_2$, -$CH_2$-$NH_2$, -amidino, -hydroxyamidino, -guanidino, -$CH_2$-guanidino, -halogen, -Cl, -Br, -I, -CN, -$CF_3$, -alkyl with 1-3 C atoms or -alkoxy with 1-3 C atoms, -$COOR_5$ where $R_5$ is a hydrogen or an alkyl with 1 to 3 C atoms, and
- $R_4$ is an -H, -OH or -O-$(CH_2)_q$-$R_6$ where q is an integer from 2 to 4 and $R_6$ is an amino or guanidino group;

P3 is any desired natural or unnatural α-amino or α-imino acid group of L or D configuration and P5 is selected from one of the groups

- -H, -CO-$R_7$ or -CO-X-$R_7$, or
- pyroglutamyl group or another amino or imino acid group, or
- -$SO_2$-$R_7$, or
- unbranched or branched alkyl group with 1 to 10 C atoms, wherein the alkyl group optionally with a halogen, an $NH_2$-, guanidino, COOH- or COO-$R_5$ group, or
- a sphingosine acylated with a bifunctional group or sphingosyl-phosphorylcholine acylated with a bifunctional group, having the following structures:

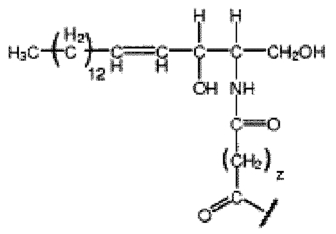

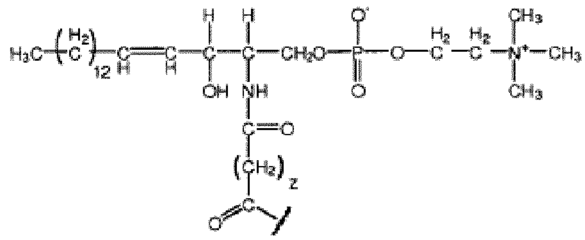

wherein

- z is an integer between 2 and 20,
- X is equal to O or NH,
- $R_5$ is a hydrogen or an alkyl group with 1 to 3 C atoms, and
- $R_7$ is

i) an unsubstituted or optionally mono- or polysubstituted branched or unbranched, saturated or unsaturated alkyl group with 1 to 24 carbon atoms and 0 to 3 double bonds, or
ii) an unsubstituted or optionally mono- or polysubstituted aryl, heteroaryl, aralkyl, heteroaralkyl or cycloalkyl group having 5 to 20 carbon atoms, wherein the heteroaryl ring may contain up to 3 heteroatoms selected from among N, S or O, where the substituent or substituents which may be present at $R_7$ are selected independently of one another from among -$NH_2$, -$CH_2$-$NH_2$, -amidino, -hydroxyamidino, -guanidino, -$CH_2$-guanidino, -halogen, -Cl, -Br, -I, -CN, -$CF_3$, -alkyl with 1-3 C atoms or alkoxy with 1-3 C atoms, -COO-$R_5$ or a cholesterol group, P5 preferably being selected from among -CO-$R_7$, -CO-X-$R_7$ or $SO_2$-$R_7$, P5 particularly preferably being selected from among the following structures:

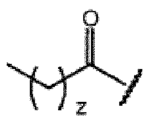

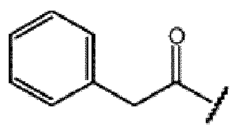

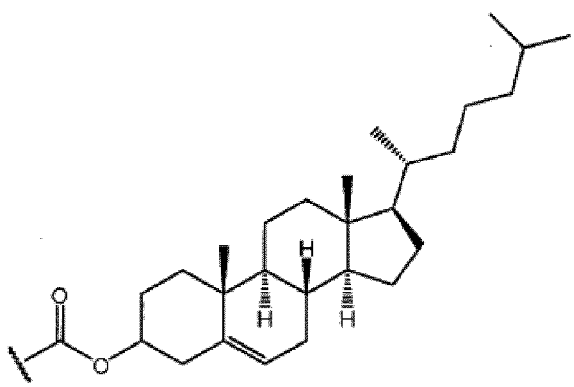

,

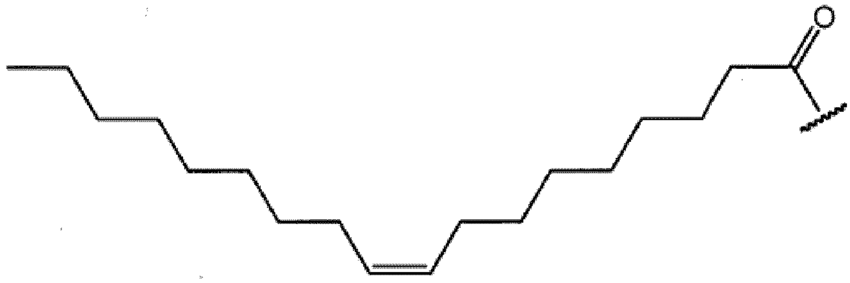

wherein z is an integer from 1 to 18, particularly preferably 8, 14 or 16.

2. Compound according to claim 1, **characterised in that** P1 is preferably selected from among the following structures:

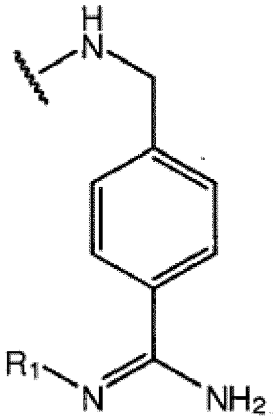

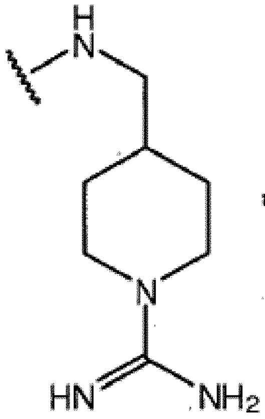

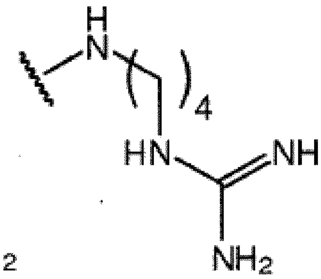

,

particularly preferably

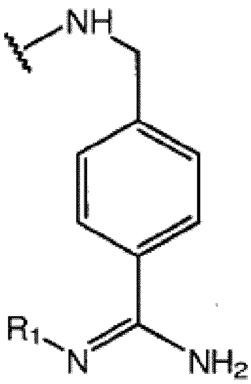

.

3. Compound according to claim 2, **characterised in that** P2 is in the L configuration and is preferably selected from among the structures

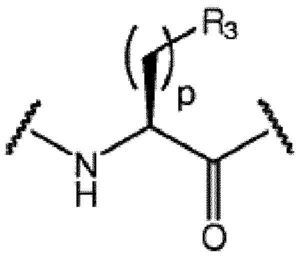

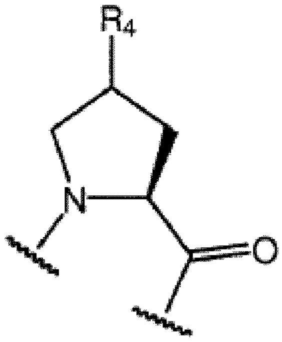

,

particularly preferably

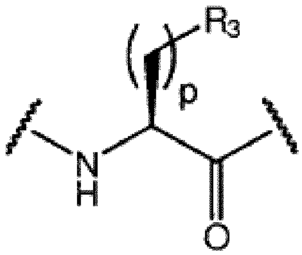

,

where $R_3$ is preferably

- an amino, guanidino or -O-guanidino group or a substituted aryl group with 6 ring atoms or an unsubstituted pyridyl group, wherein the nitrogen may be at any desired position in the pyridyl ring and is optionally also present as an N-oxide, and wherein the substituent on the aryl ring is selected from among $-NH_2$, $-CH_2-NH_2$, amidino, hydroxyamidino, guanidino or $-CH_2$-guanidino.

**4.** Compound according to claim 3, **characterised in that** P4 is an amino acid group in the L configuration.

**5.** Compound according to claim 4, **characterised in that** P3 is a non-basic α-amino or α-imino acid group in the L configuration, preferably the group of a proteinogenic α-amino or α-imino group.

**6.** Compound according to one of claims 1 to 5, **characterised in that**

- P1 is a 4-amidinobenzylamide group,
- P2 is an arginyl, lysyl or canavanine group in the L configuration,
- P3 is a non-basic α-amino or α-imino acid group in the L configuration,
- P4 is an arginyl, lysyl or canavanine group in the L configuration, and
- P5 is a phenylacetyl or another acyl group,

the following compounds, including their salts,

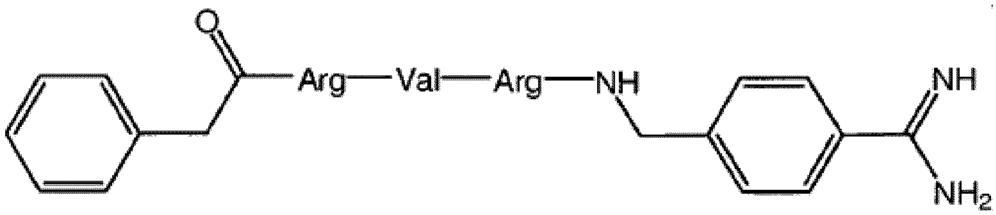

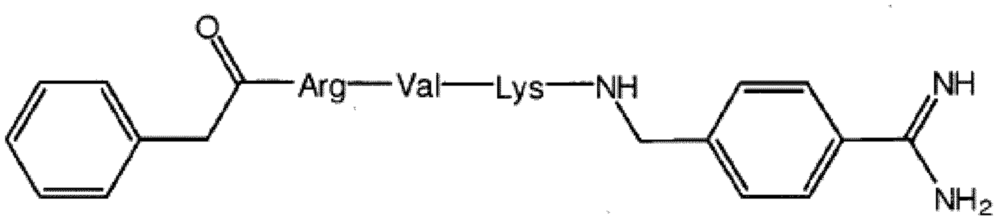

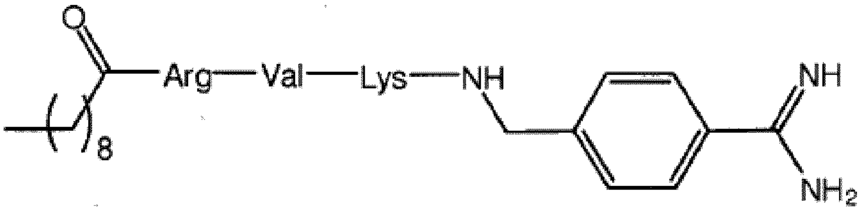

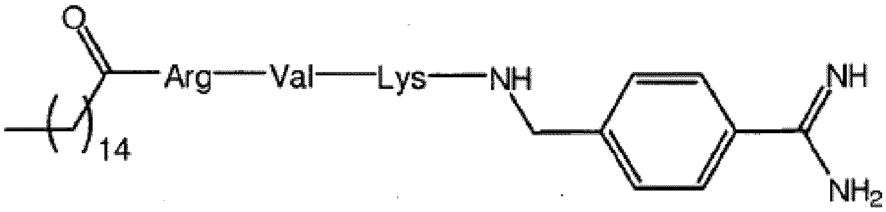

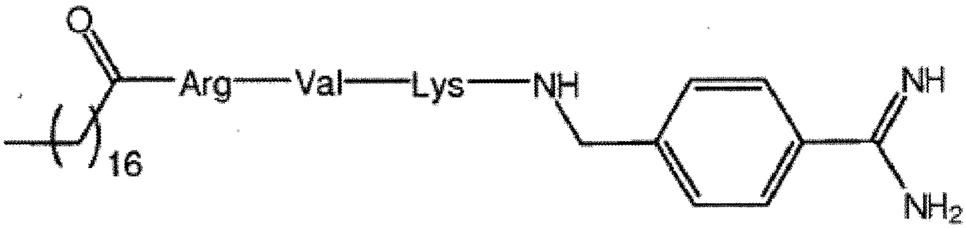

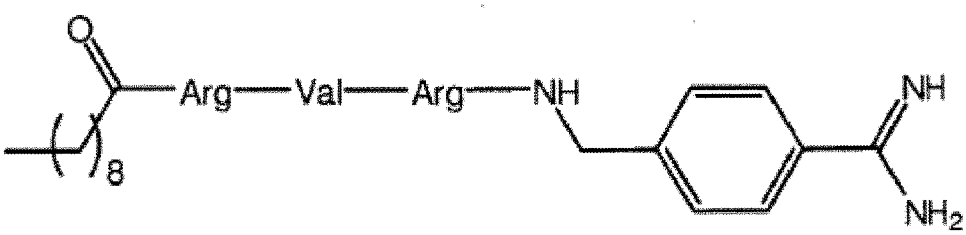

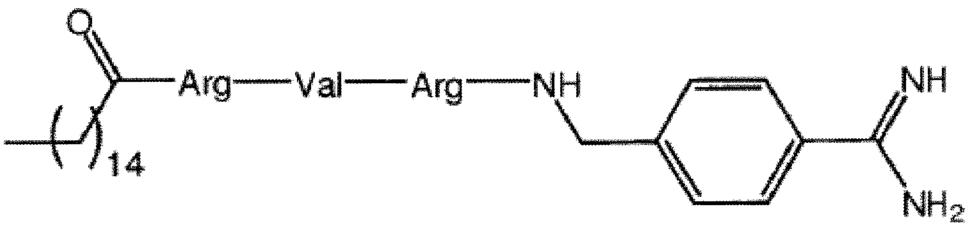

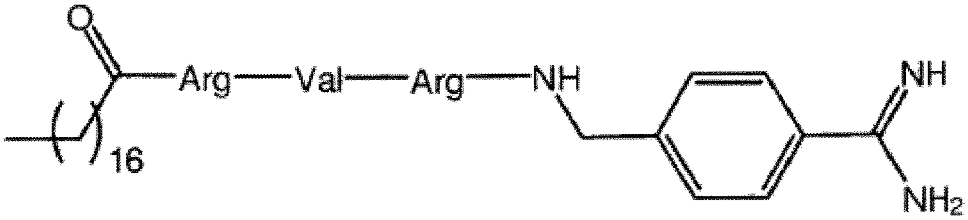

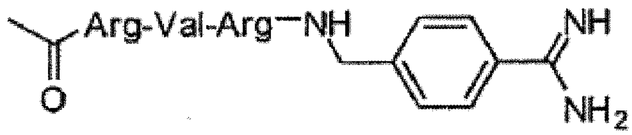

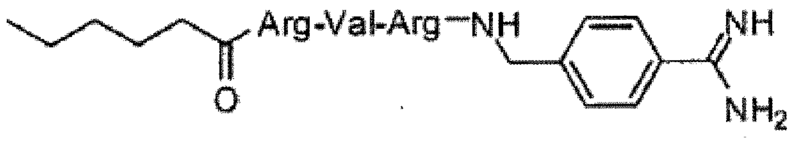

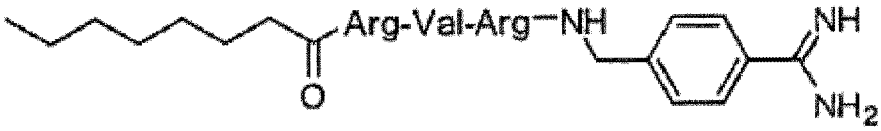

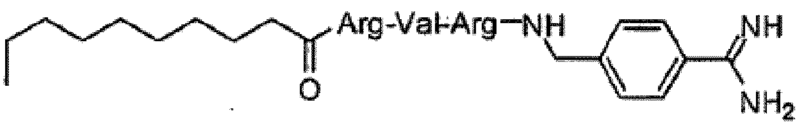

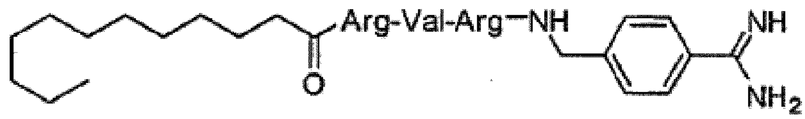

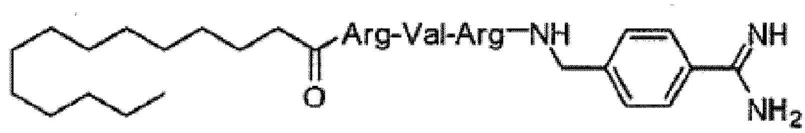

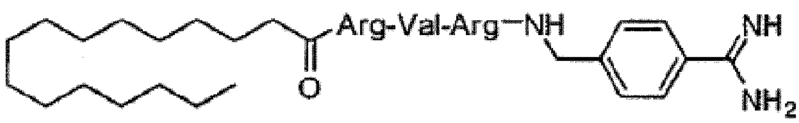

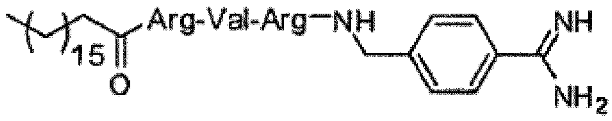

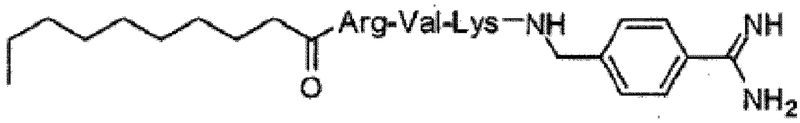

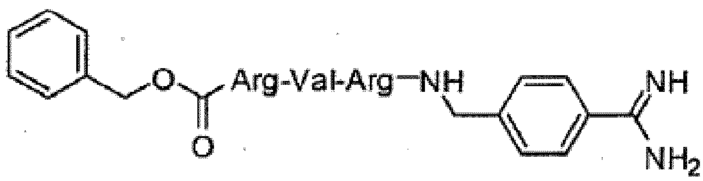

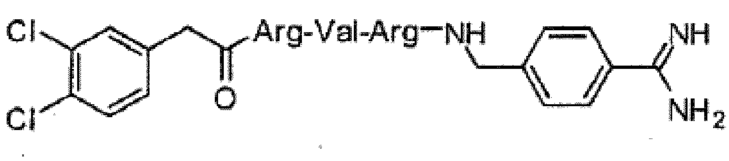

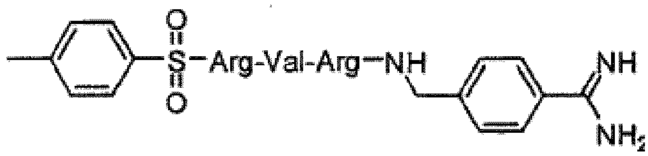

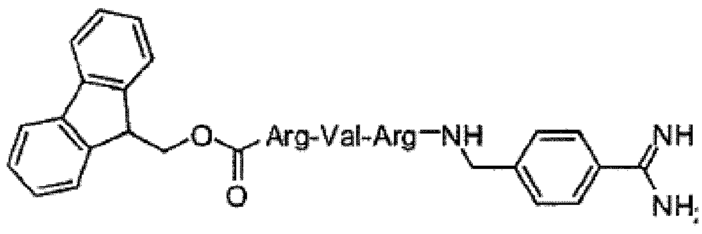

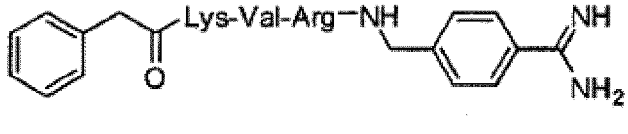

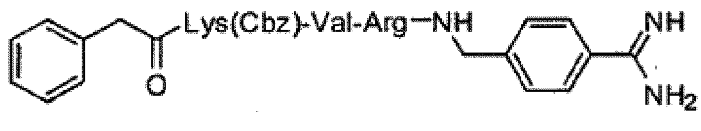

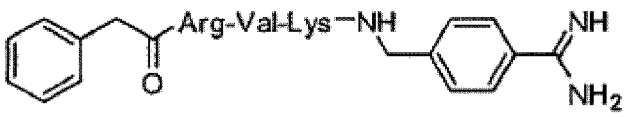

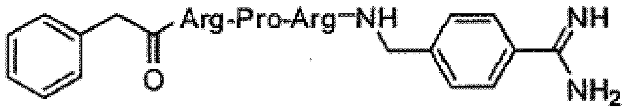

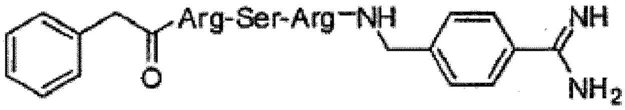

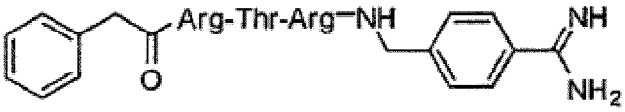

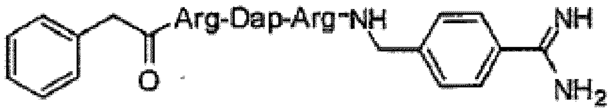

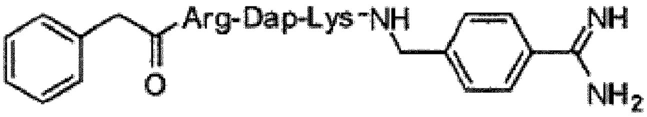

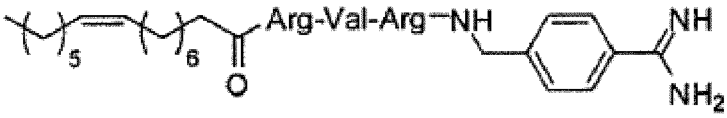

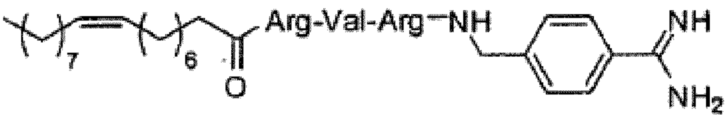

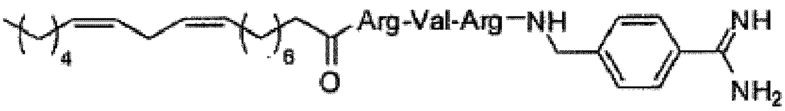

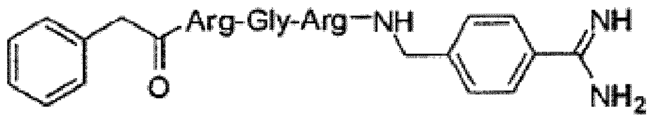

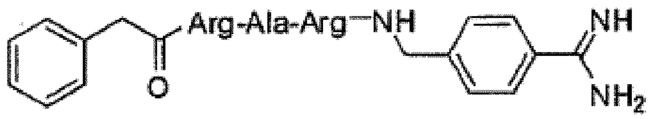

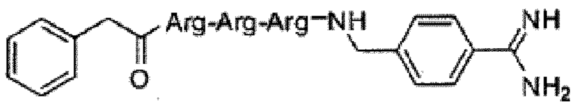

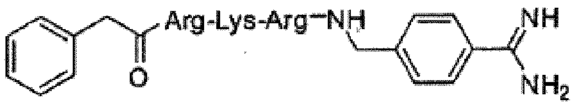

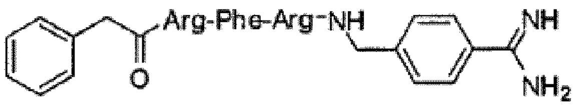

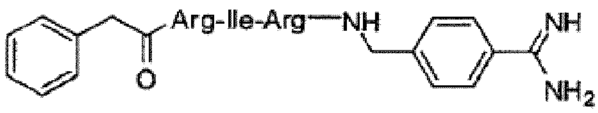

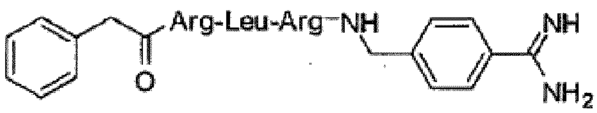

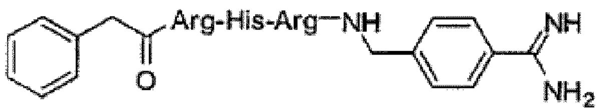

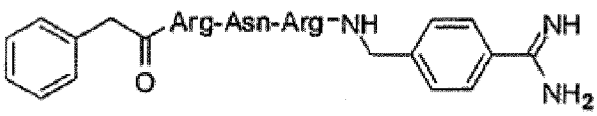

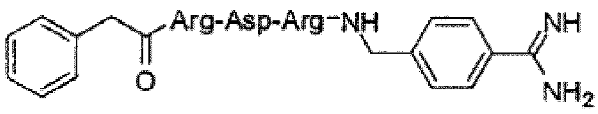

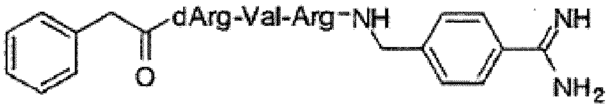

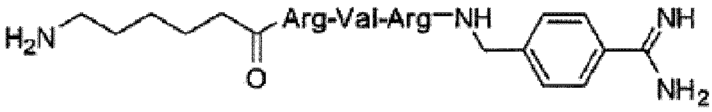

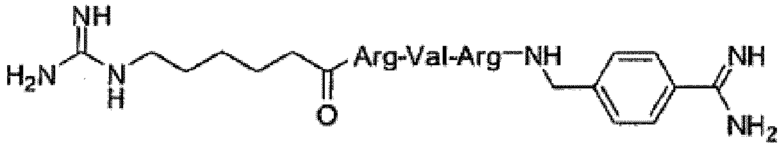

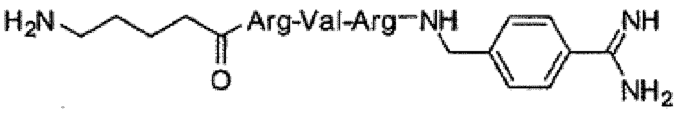

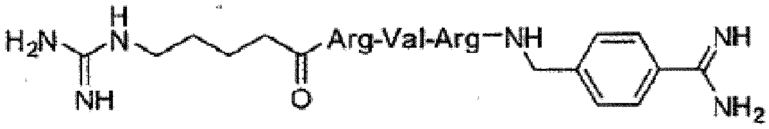

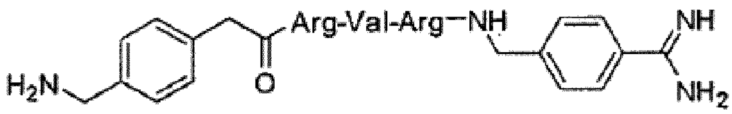

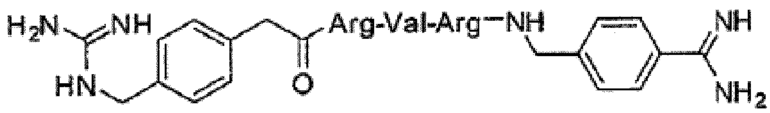

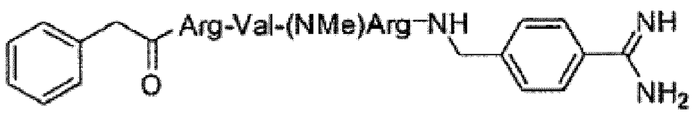

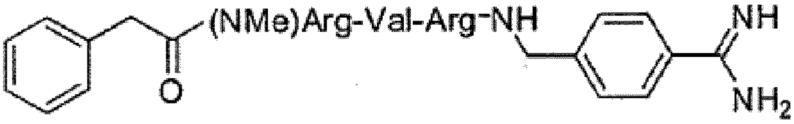

being most particularly preferred.

**7.** Method for preparing a compound according to one of claims 1 to 6 by a combination of solid phase and solution synthesis comprising the following steps:

- synthesis of the segment P5-P4-P3-P2 by solid phase peptide synthesis,
- cleaving the peptide segment from the resin under suitable acidic conditions, whereby side chain protective groups are either preserved or cleaved,
- coupling a suitable P1 component by standard methods free from racemisation, wherein the P1 component is present in either protected or unprotected form,
- purification by crystallisation, chromatography, preferably reverse phase or ion exchange chromatography or by countercurrent distribution.

**8.** Method for preparing a compound according to one of claims 1 to 6 by solution synthesis comprising the following steps:

a) synthesis of a P1 with a free amino group, suitably protected at the terminal amidino or guanidino group, the amino group being present either as a salt or as a free base, preferably synthesis of the following compound:

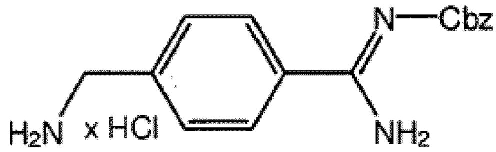

b) coupling of an Nα-Boc or otherwise suitably Nα-urethane-protected amino or imino acid with or without a suitable side chain protecting group to the free amino group of the P1 component,
c) working up the reaction mixture by conventional washing and crystallisation processes,
d) cleaving the Nα-protective group to obtain the P2-P1 segment with a free amino group at the P2 component,
e) coupling the Nα-Boc or otherwise suitably Nα-urethane-protected P3-amino or imino acid to the free amino group of the P2-P1 component,
f) working up the reaction mixture by conventional washing and crystallisation processes,
g) cleaving the Nα-protective group to obtain the P3-P2-P1 segment with a free N-terminal amino group at the P3 group,
h) coupling the Nα-Boc or otherwise suitably Nα-urethane-protected P4-amino or imino acid to the free amino group of the P3-P2-P1 component from step h),
i) working up the reaction mixture by conventional washing and crystallisation processes,
j) cleaving the Nα-protective group to obtain the P4-P3-P2-P1 segment with a free N-terminal amino group at the P4 group,
k) coupling the P5 group to the free amino group of the P4-P3-P2-P1 segment from step j),
l) cleaving all the remaining protective groups,
m) purification by crystallisation and/or chromatography.

**9.** Medicaments, containing at least one compound according to one of claims 1 to 6.

**10.** Compound according to one of claims 1 to 6 or a medicament according to claim 9 for the treatment or prevention of a disease of viral or bacterial origin, and for the treatment or prevention of diabetes, arteriosclerosis, cancer, Alzheimer's disease or obesity, particularly in oral, subcutaneous, intravenous or transdermal form or in the form of a spray for treating diseases in the respiratory tract, the diseases of bacterial origin being caused in particular by pathogens from the group comprising *Pseudomonas spec., Shigella spec., Corynebacterium diphtheriae, Bacillus cereus, Bacillus anthracis, Bacillus botulinum, Clostridium perfringens, Clostridium difficile, Clostridium septicum, Clostridium spiroforme, Bordetella pertussis, Salmonella enterica* or by selected strains of *Escherichia coli,* particularly strains of *E.* coliwhich cause haemolytic colitis, haemolytic uraemic syndrome and/or kidney failure, most particularly *E. coli* 0157-H7.

**11.** Compound according to one of claims 1 to 6 or a medicament according to claim 9, for the treatment or prevention of diseases caused by viruses, particularly caused by viruses of the group

- highly pathogenic avian influenza A viruses, especially of the H5 or H7 subtypes,
- minus-strand RNA viruses such as the parainfluenza viruses, measles virus, mumps virus, canine distemper virus, respiratory syncytial virus, filoviruses such as the Ebola and Marburg virus and Newcastle disease virus, and

• plus-strand RNA viruses such as HIV-1, HIV-2, HTLV and other retroviruses, particularly coronaviruses such as the SARS coronavirus, the flaviviruses, yellow fever viruses, West Nile virus, Dengue virus or early summer meningo-encephalitis virus, Venezuelan equine encephalitis virus or Rift Valley fever virus.

**12.** Compound according to one of claims 1 to 6 or a medicament according to claim 9 for inhibiting the proprotein convertases furin, PC1/3, PC2, PC4, PC5/6, PACE4 and/or PC7.

**13.** Compound according to one of claims 1 to 6 or a medicament according to claim 9 for the treatment of cancer and for inhibiting metastasis.

## Revendications

**1.** Composé selon la formule (I) :

P5-P4-P3-P2-P1 (1)

dans laquelle P5-P4-P3-P2-P1 est un dérivé tétrapeptidique et dans laquelle P1 est un mimétique d'arginine, P2, P3, P4 sont respectivement un résidu aminoacide et/ou iminoacide et P5 est une modification N-terminale du résidu aminoacide ou iminoacide P4, **caractérisé en ce que** :
P1 est un mimétique d'arginine décarboxylé choisi dans les structures suivantes :

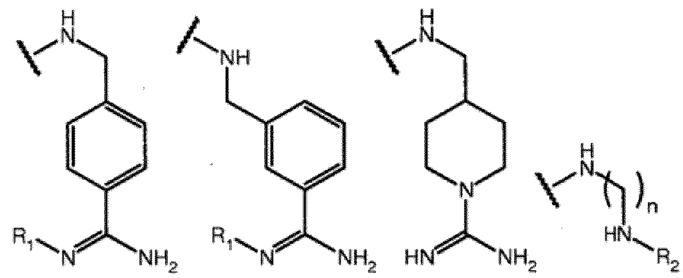

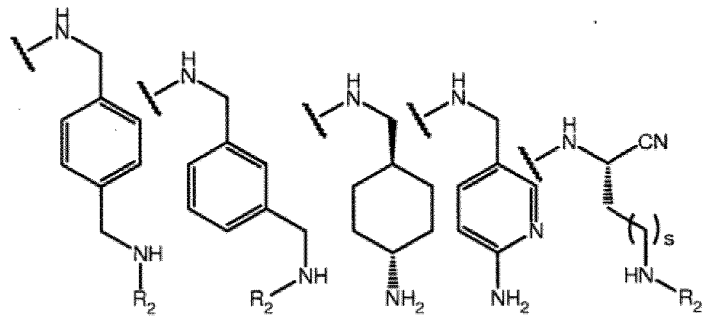

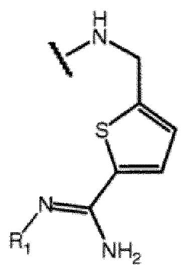

dans lesquelles :

• $R_1$ est choisi dans le groupe comprenant H, OH, O-$CH_3$, $NH_2$, O-CO-$CH_3$ ou -CO-O-$(CH_2)_m$-$CH_3$, où m

est un nombre entier de 1 à 5, et

- $R_2$ est H ou un groupement amidino et
- n est égal à 3, 4 ou 5 et s est égal à 2 ou 3,

P2 et P4 sont indépendamment l'un de l'autre respectivement un résidu aminoacide ou iminoacide choisi dans les structures suivantes :

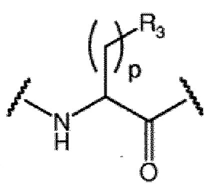

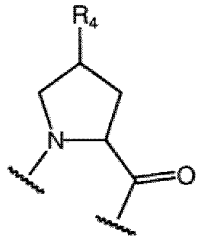

dans lesquelles :

- p est un nombre entier de 0 à 5 et
- $R_3$ peut être un groupement amino ou guanidino ou un groupement -O-guanidino ou encore un résidu aryle ou hétéroaryle substitué une ou plusieurs fois ou non substitué avec 5 à 10 atomes cycliques, où le cycle hétéroaryle peut contenir jusqu'à 3 hétéroatomes choisis parmi N, S ou 0, où le substituant sur le cycle aryle ou hétéroaryle est choisi parmi les suivants : $-NH_2$, $-CH_2-NH_2$, -amidino, -hydroxyamidino, -guanidino, $-CH_2$-guanidino, -halogène, -Cl, -Br, -I, -CN, $-CF_3$, -alkyle avec 1 à 3 atomes de carbone ou -alcoxy avec 1 à 3 atomes de carbone, et
- $R_4$ est -H, -OH ou $-O-(CH_2)_q-R_6$, où q est un nombre entier de 2 à 4 et $R_6$ un groupement amino ou guanidino ;

P3 est un résidu quelconque $\alpha$-aminoacide ou $\alpha$-iminoacide naturel ou artificiel avec une configuration en L ou en D, et

P5 est choisi parmi l'un des groupements :

- -H, $-CO-R_7$ ou $-CO-X-R_7$ ou
- un résidu pyroglutamyle ou un résidu aminoacide ou iminoacide ou
- $-SO_2-R_7$ ou
- un résidu alkyle non ramifié ou ramifié avec 1 à 10 atomes de carbone, où le résidu alkyle est éventuellement modifié par un halogène, un groupement $NH_2$, guanidino-COOH ou $COO-R_5$ et $R_5$ est défini comme ci-dessus, ou
- une sphyngosine acylée par un résidu bifonctionnel ou une sphyngosyl-phosphorylcholine acylée par un résidu bifonctionnel de structures suivantes :

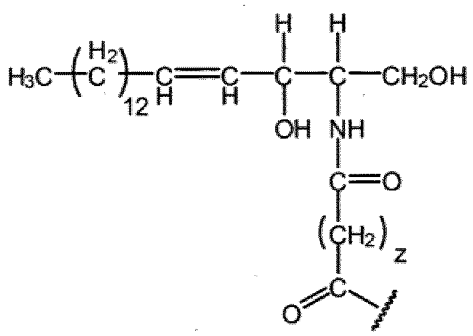

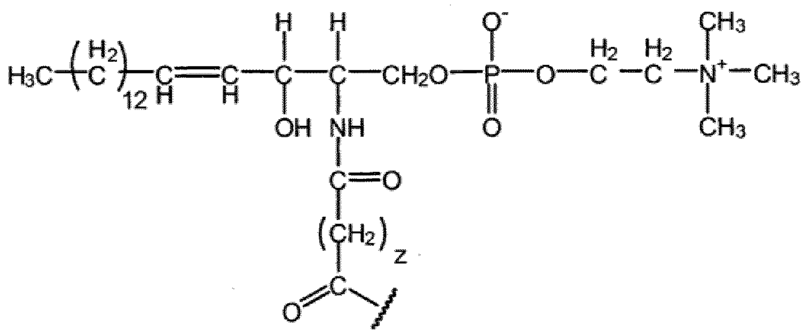

dans lesquelles :

- z est un nombre entier entre 2 et 20,
- X représente 0 ou NH,
- $R_5$ est l'hydrogène ou un groupement alkyle avec 1 à 3 atomes de carbone et
- $R_7$ est :

i) un radical alkyle saturé ou insaturé, non substitué ou éventuellement substitué une ou plusieurs fois, ramifié ou non ramifié, avec 1 à 24 atomes de carbone et 0 à 3 doubles liaisons, ou
ii) un radical aryle, hétéroaryle, aralkyle, hétéroaralkyle ou cycloalkyle non substitué ou éventuellement substitué une ou plusieurs fois avec 5 à 20 atomes de carbone, où le cycle hétéroaryle peut contenir jusqu'à 3 hétéroatomes choisis parmi N, S ou 0, où les substituants éventuellement présents sont choisis indépendamment l'un de l'autre parmi les groupements $-NH_2$, $-CH_2-NH_2$, -amidino, -hydroxyamidino, -guanidino, $-CH_2$-guanidino, -halogène, -Cl, -Br, -I, -CN, $-CF_3$, -alkyle avec 1 à 3 atomes de carbone ou alcoxy avec 1 à 3 atomes de carbone, $-COO-R_5$ ou un radical cholestérol, où P5 est de préférence choisi dans le groupe $-CO-R_7$, $-CO-X-R_7$ ou $SO_2-R_7$, où P5 est tout particulièrement choisi parmi les structures suivantes :

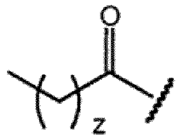

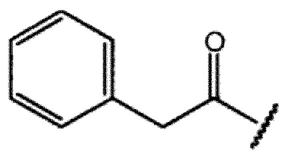

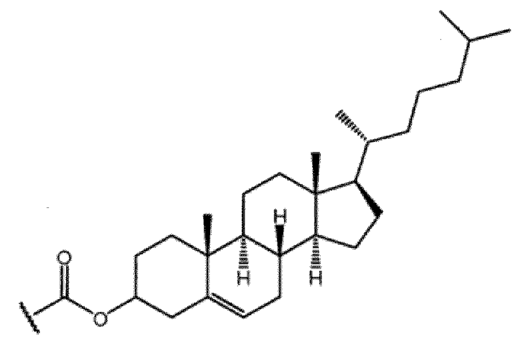

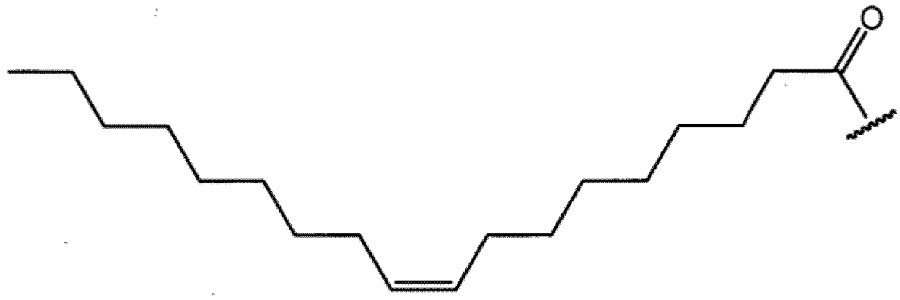

dans lesquelles z est un nombre entier de 1 à 18, tout particulièrement de 8, 14 ou 16.

2. Composé selon la revendication 1, **caractérisé en ce que** P1 est de préférence choisi parmi les structures suivantes :

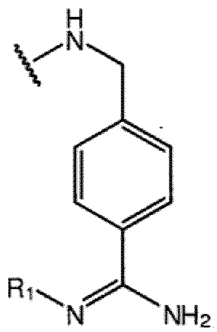

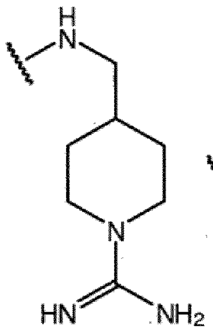

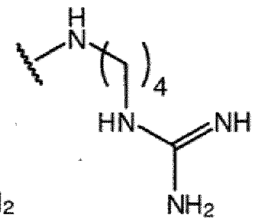

,

tout particulièrement :

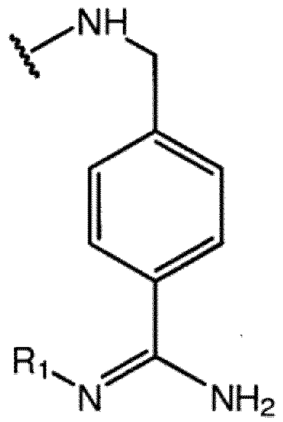

.

3. Composé selon la revendication 2, **caractérisé en ce que** P2 se présente en configuration L et est choisi de préférence dans les structures suivantes :

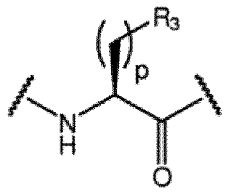

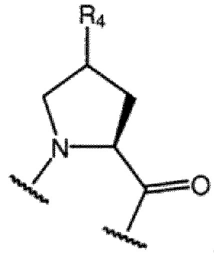

,

tout particulièrement :

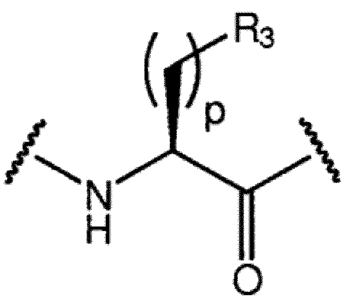

,

Où $R_3$ est de préférence :

- un groupement amino, guanidino ou -O-guanidino ou encore un radical aryle substitué avec 6 atomes cycliques ou un radical pyridyle non substitué, où l'azote peut se trouver en un point quelconque dans le cycle pyridyle et se présente éventuellement aussi sous la forme d'un oxyde de N et dans lequel le substituant sur le cycle aryle est choisi parmi les radicaux $-NH_2$, $-CH_2-NH_2$, -amidino, -hydroxyamidino, -guanidino ou $-CH_2$-guanidino.

**4.** Composé selon la revendication 3, **caractérisé en ce que** P4 est un radical aminoacide en configuration L.

**5.** Composé selon la revendication 4, **caractérisé en ce que** P3 est un radical α-aminoacide ou α-iminoacide non basique en configuration L, de préférence le résidu d'un α-aminoacide ou α-iminoacide protéinogène.

**6.** Composé selon l'une quelconque des revendications 1 à 5, **caractérisé en ce que** :

- P1 est un radical 4-amidinobenzylamide,
- P2 est un radical arginyle, lysyle ou canavanine en configuration L,
- P3 est un radical α-aminoacide ou α-iminoacide non basique en configuration L,
- P4 est un radical arginyle, lysyle ou canavanine en configuration L et
- P5 est un radical phénylacétyle ou un autre radical acyle,

dans lequel les composés, notamment leurs sels,

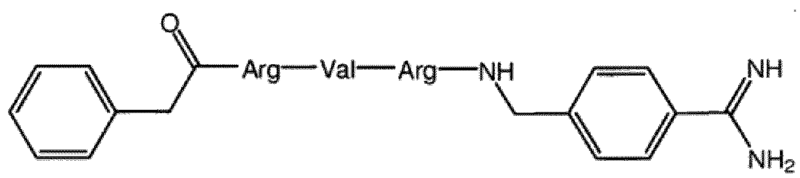

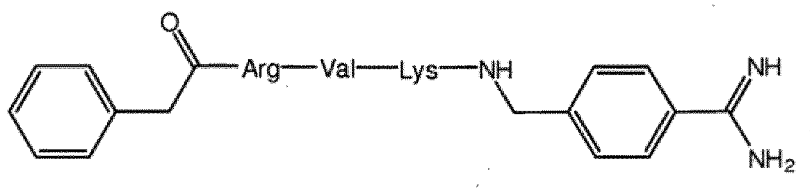

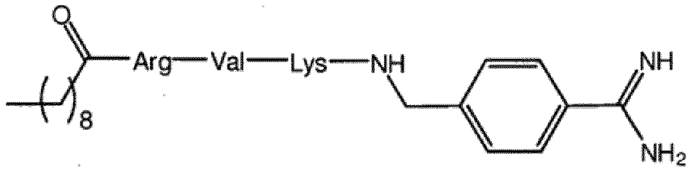

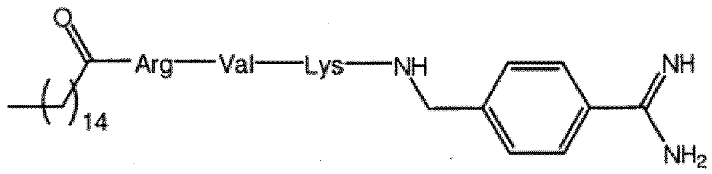

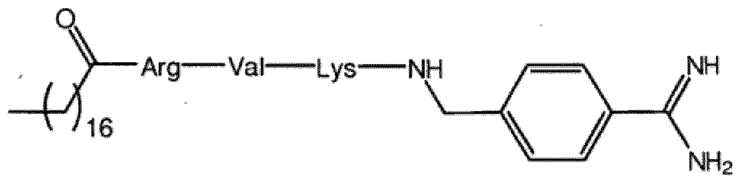

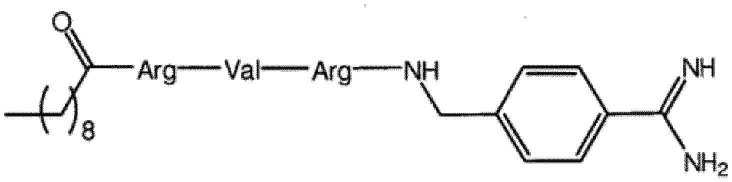

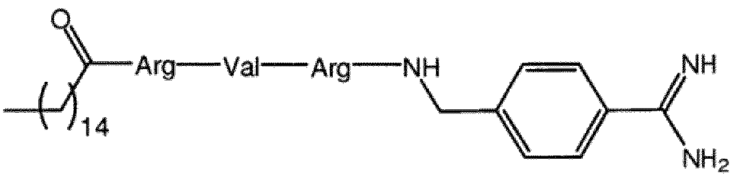

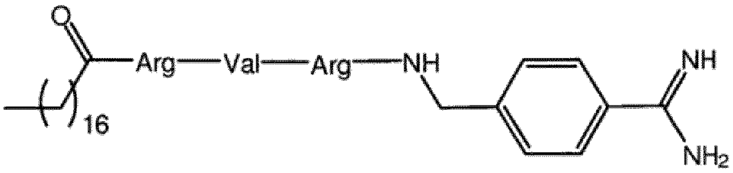

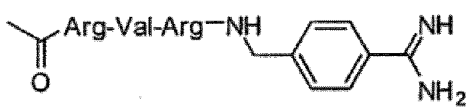

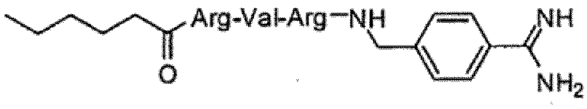

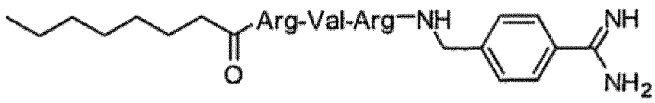

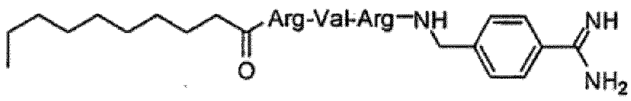

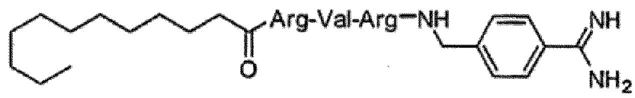

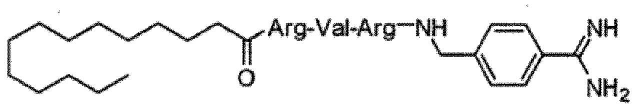

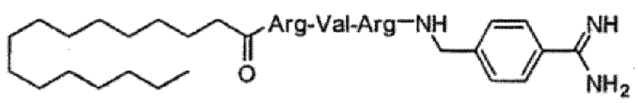

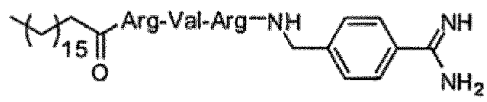

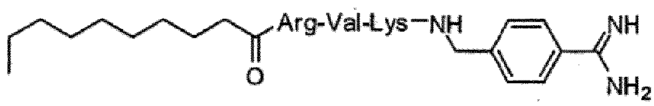

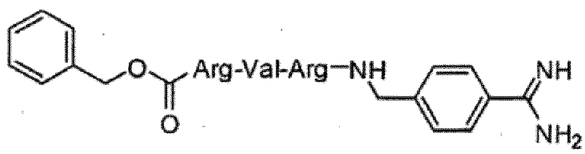

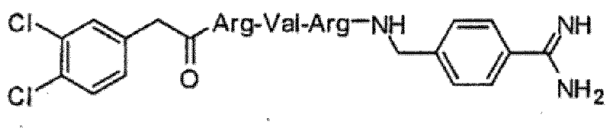

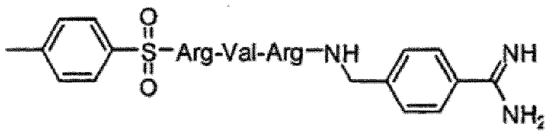

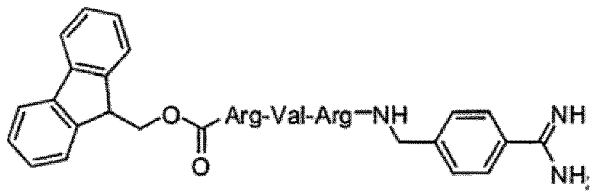

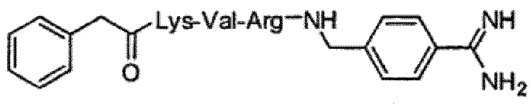

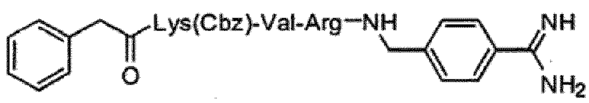

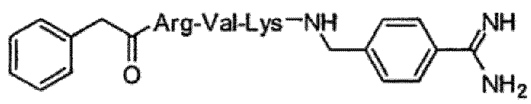

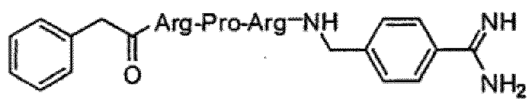

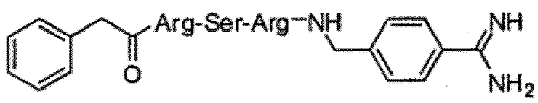

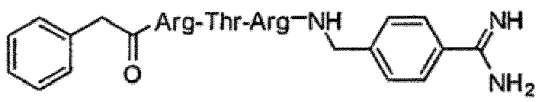

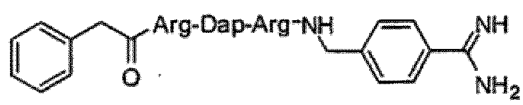

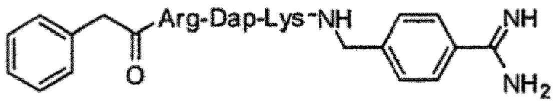

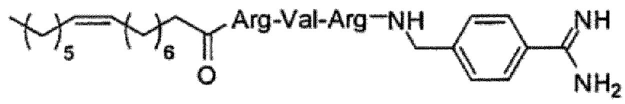

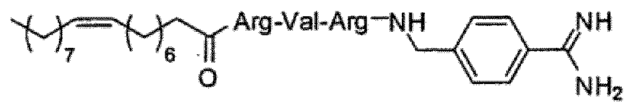

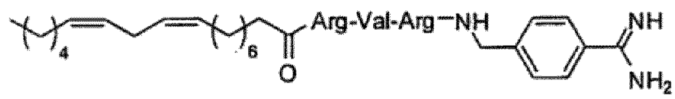

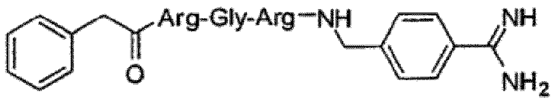

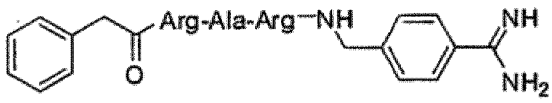

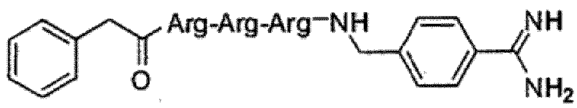

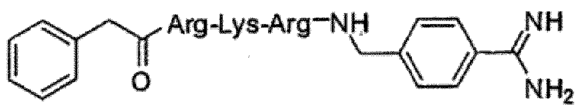

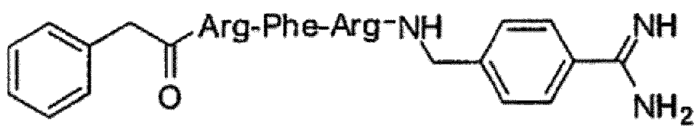

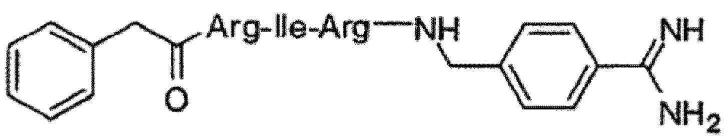

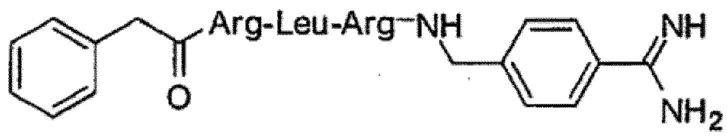

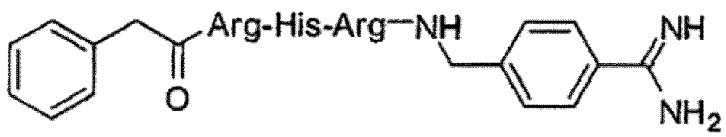

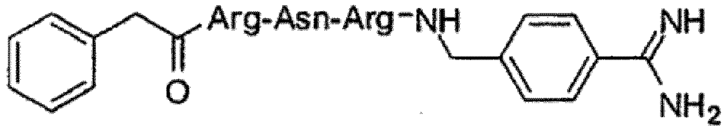

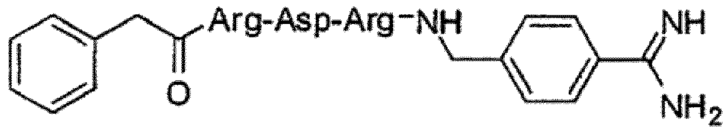

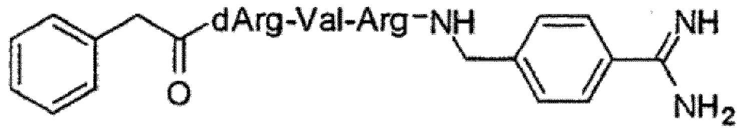

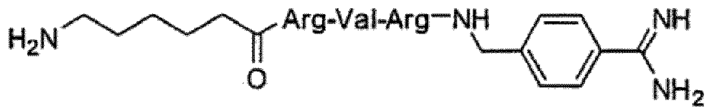

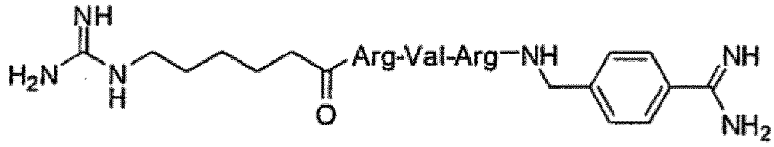

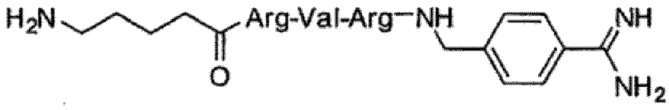

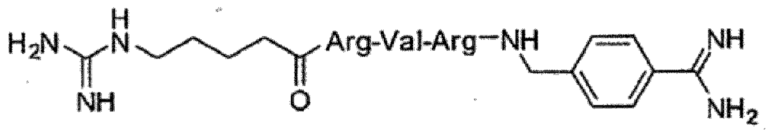

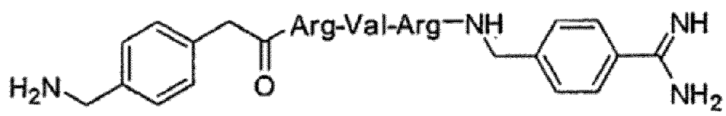

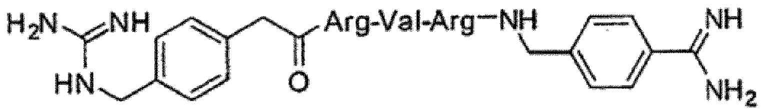

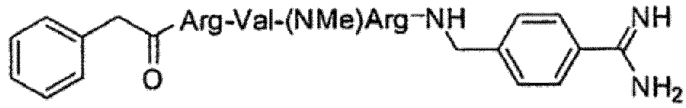

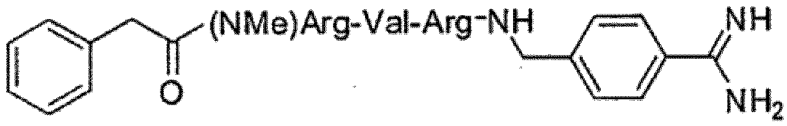

sont tout particulièrement préférés.

7. Procédé de fabrication d'un composé selon l'une quelconque des revendications 1 à 6 par une combinaison d'une synthèse de phase solide et de solution au moyen des étapes suivantes :

• synthèse du segment P5-P4-P3-P2 par synthèse de peptides en phase solide,
• séparation du segment peptidique de la résine dans des conditions acides appropriées, où des groupements de protection de chaînes latérales sont conservés ou séparés,
• couplage d'un composant P1 approprié par un procédé standard sans racémisation, dans lequel le composant P1 se présente sous une forme protégée ou non protégée, et
• purification par cristallisation, chromatographie, de préférence par chromatographie à phases inversées ou à échange ionique ou par distribution à contre-courant.

8. Procédé de fabrication d'un composé selon l'une quelconque des revendications 1 à 6 par synthèse en solution comprenant les étapes suivantes :

a) synthèse d'un P1 protégé de manière appropriée sur le groupement terminal amidino ou guanidino avec un groupement amino libre, où le groupement amino se présente sous la forme d'un sel ou d'une base, de préférence synthèse du composé suivant :

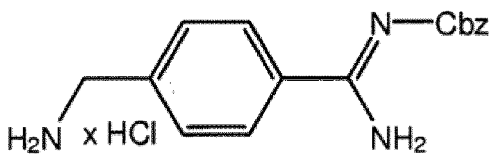

b) couplage d'un aminoacide ou iminoacide protégé par du Nα-Boc ou de manière autrement appropriée par

du Nα-uréthane avec ou sans groupement approprié de protection de chaînes latérales sur le groupement amino libre du composant P1,
c) traitement de la fraction réactionnelle par des procédés courants de lavage et de cristallisation,
d) séparation du groupement de protection Nα en maintenant le segment P2-P1 avec un groupement amino libre sur le composant P2,
e) couplage du P3-amino- ou iminoacide protégé par du Nα-Boc ou de manière autrement appropriée par du Nα-uréthane sur le groupement amino libre du segment P2-P1,
f) traitement de la fraction réactionnelle par des procédés courants de lavage et de cristallisation,
g) séparation du groupement de protection Nα en maintenant le segment P3-P2-P1 avec un groupement amino N-terminal libre sur le résidu P3,
h) couplage du P4-amino- ou iminoacide protégé par du Nα-Boc ou de manière autrement appropriée par du Nα-uréthane sur le groupement amino libre du segment P3-P2-P1 de l'étape h),
i) traitement de la fraction réactionnelle par des procédés courants de lavage et de cristallisation,
j) séparation du groupement de protection Nα en maintenant le segment P4-P3-P2-P1 avec un groupement amino N-terminal libre sur le résidu P4,
k) couplage du résidu P5 sur le groupement amino libre du segment P4-P3-P2-P1 de l'étape j),
l) séparation de tous les groupements de protection encore présents, et
m) purification par cristallisation et/ou chromatographie.

**9.** Médicament contenant au moins un composé selon l'une quelconque des revendications 1 à 6.

**10.** Composé selon l'une quelconque des revendications 1 à 6 ou médicament selon la revendication 9 destiné à la thérapie ou à la prophylaxie de maladies d'origine virale ou bactérienne, ainsi qu'à la thérapie et à la prophylaxie du diabète, de l'artériosclérose, du cancer, de la maladie d'Alzheimer ou de l'excédent de poids, en particulier sous forme buccale, sous-cutanée, intraveineuse ou transdermique ou sous la forme d'un jet pulvérisé pour le traitement de maladies des voies respiratoires, dans lequel les maladies d'origine bactérienne en particulier sont provoquées par des agents pathogènes tirés du groupe suivant : *Pseudomonas spec., Shigella spec., Corynebacterium diphtheriae, Bacillus cereus, Bacillus anthracis, Bacillum butulinum, Clostridium perfringens, Clostridium difficile, Clostridium speticum, Clostridium sprioforme, Bordetella pertussis, Salmonelle enterica,* ou par des souches *d'Escherichia coli* choisies, en particulier des souches de *E. coli* qui provoquent une colite hémolytique, le syndrome hémolytique et urémique et/ou des défaillances rénales, tout particulièrement la souche de *E. coli* 0157-H7.

**11.** Composé selon l'une quelconque des revendications 1 à 6 ou médicament selon la revendication 9 destiné à la thérapie ou à la prophylaxie de maladies provoquées par des virus, en particulier provoquées par des virus du groupe :

• des virus A de la grippe aviaire hautement pathogène, surtout les sous-type H5 ou H7,
• des virus à ARN négatif, tels que les virus parainfluenza, le virus de la rougeole, le virus des oreillons, le virus de la maladie du jeune âge des chiens, le virus respiratoire syncytial, les Filovirus, tels que le virus Ebola et le virus de Marbourg, ainsi que le virus de la maladie de Newcastle, ainsi que
• des virus à ARN positif, tels que le VIH-1, le VIH-2, le HTLV et d'autres rétrovirus, en particulier les Coronavirus, tels que le Coronavirus SARS, les Flavivirus, les virus de la fièvre jaune, le virus de West Nile, le virus de la dengue ou le virus de la méningo-encéphalite de l'été précoce, le virus de l'encéphalite équine du Venezuela ou le virus de la fièvre de la vallée du Rift.

**12.** Composé selon l'une quelconque des revendications 1 à 6 ou médicament selon la revendication 9 destiné à ralentir les proprotéine convertases de type furine, les PC1/3, PC2, PC4, PC5/6, PACE4 ou PC7.

**13.** Composé selon l'une quelconque des revendications 1 à 6 ou médicament selon la revendication 9 destiné à la thérapie du cancer et au ralentissement des métastases.

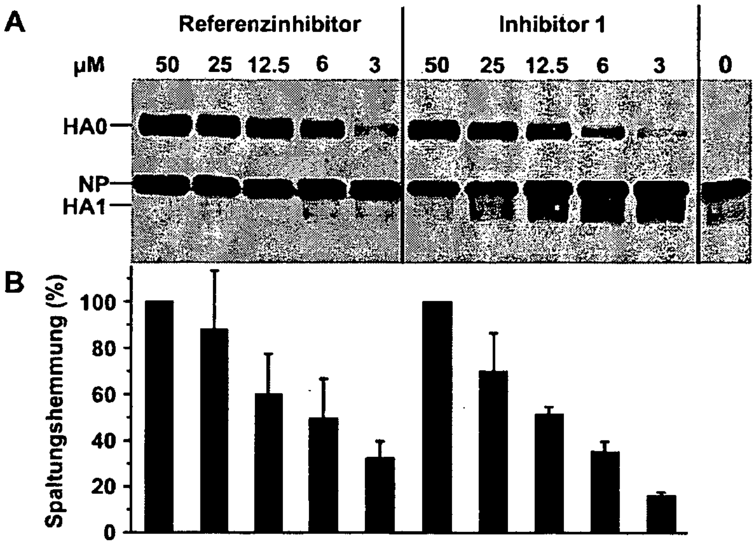


Figur 1

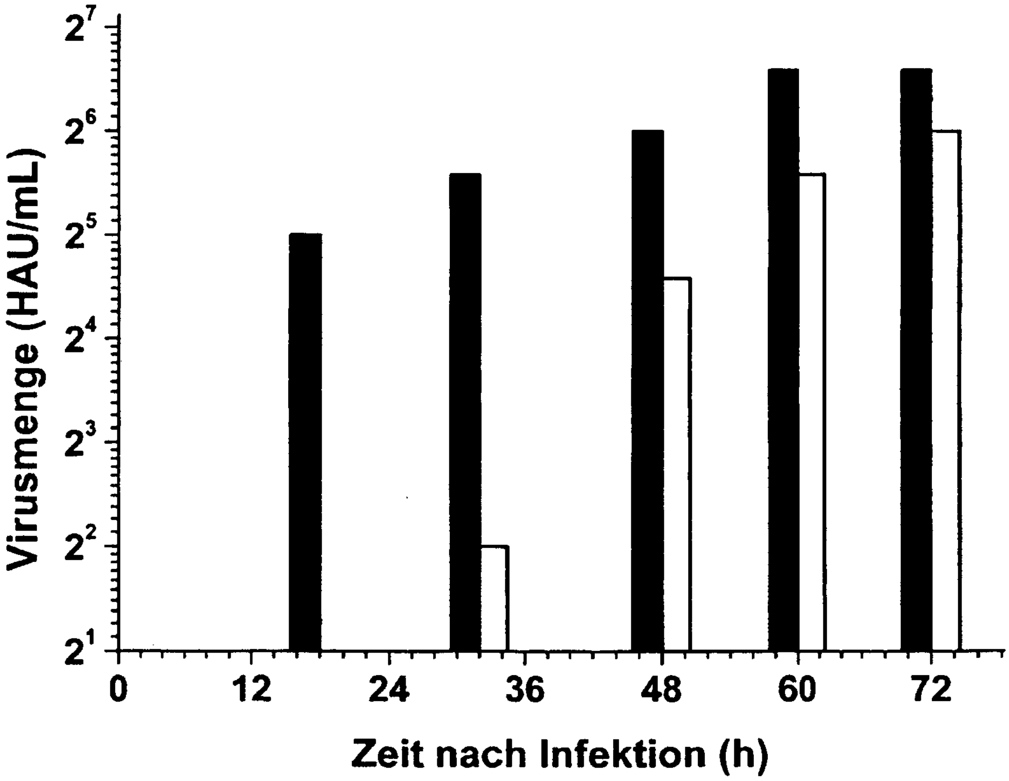


Figur 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008049595 A1
- WO 2007046781 A1


### In der Beschreibung aufgeführte Nicht-Patentliteratur


- **SEIDAH, N.G. ; PRAT, A.** *J. Mol. Med,* 2007, vol. 85, 685-696
- **SCHECHTER ; BERGER.** *Biochem. Biophys. Res. Comm.,* 1967, vol. 27, 157
- **THOMAS, G.** *Nat. Rev. Mol. Cell. Biol,* 2002, vol. 3, 753-766
- **REMACLE A.G. et al.** *J. Biol. Chem.,* 2008, vol. 283, 20897-20906
- **THOMAS, G.** *Nat. Rev. Mol. Cell. Biol.,* 2002, vol. 3, 753-766
- **BONTEMPS, Y. et al.** *Med. Res. Rev.,* 2007, vol. 27, 631-648
- **BENNETT, B.D. et al.** *J. Biol. Chem.,* 2000, vol. 275, 37712-37717
- **BASSI, D. E. ; MAHLOOGI, H. ; AL-SALEEM, L. ; LOPEZ DE CICCO, R. ; RIDGE, J. A. ; KLEIN-SZANTO, A. J.** Elevated furin expression in aggressive human head and neck tumors and tumor cell lines. *Mol Carcinog,* 2001, vol. 31, 224-32
- **KHATIB, A. M. ; BASSI, D. ; SIEGFRIED, G. ; KLEIN-SZANTO, A. J. ; OUAFIK, L.** Endo/exo-proteolysis in neoplastic progression and metastasis. *J Mol Med,* 2005, vol. 83, 856-64
- **LIN et al.** *J. Med. Chem.,* 2006, vol. 49, 7781-7791
- **MCNAUGHTON ; MILLER.** *Org. Lett.,* 2006, vol. 27, 1803-1806
- **KACPRZAK, M.M. et al.** *J. Biol. Chem.,* 2004, vol. 279, 36788-36794
- **BASAK, A.** *J. Mol. Med,* 2005, vol. 83, 844-855
- **HALLENBERGER, S. et al.** *Nature,* 1992, vol. 360, 358-361
- **JEAN, F. et al.** *Biochem. J.,* 1993, vol. 292, 891-899
- **ANGLIKER, H.** *J. Med. Chem.,* 1995, vol. 38, 4014-4018
- **BASAK, A. et al.** *Biochem. J.,* 1999, vol. 338, 107-113
- **SCHECHTER ; BERGER.** *Biochem. Biophys. Res. Comm.,* 1967, vol. 27, 157-162
- **ETTMAYER et al.** *J. Med. Chem.,* 2004, vol. 47, 2393-2404
- **STEINMETZER ; STÜRZEBECHER.** *Current Med. Chem.,* 2004, vol. 11, 2297-2321
- **BERNATOWICZ, M.S. et al.** *J. Org. Chem.,* 1992, vol. 57, 2497-2502
- **LILA et al.** *Synth. Commun.,* 1998, vol. 28, 4419-4429
- **SCHWEINITZ, A. et al.** *Med. Chem.,* 2006, vol. 2, 349-361
- **STEINMETZER et al.** *J. Enz. Inhib.,* 1999, vol. 14, 203-216
- **GARTEN et al.** *Virology,* 1989, vol. 172, 25-31
- **STEINMETZER et al.** *Biol. Chem.,* 2000, vol. 381, 603-610